# EUROPEAN PATENT SPECIFICATION

(11) **EP 3 706 773 B1**
(45) Date of publication and mention of the grant of the patent: **26.08.2026**
(21) Application number: 18800163.0
(22) Date of filing: 09.11.2018
(51) Int. Cl.: A61K 38/17, A61P 1/00, A61P 11/00, A61P 1/12

(54) **HBD2 FOR USE IN THE TREATMENT OF NECROTISING ENTEROCOLITIS IN A PRETERM INFANT**
HBD2 ZUR VERWENDUNG IN DER BEHANDLUNG VON NECROTISIERDENDER ENTEROCOLITIS BEI FRÜHGEBORENEN
HBD2 POUR L'UTILISATION DANS LE TRAITEMENT DE L'ENTEROCOLITE NÉCROTISANTE CHEZ LE NOURRISSON PRÉMATURÉ

(30) Priority: 10.11.2017 EP 17201002
(43) Date of publication of application: 16.09.2020
(73) Proprietor: Defensin Therapeutics ApS, 2200 København N (DK)
(72) Inventor: NORDKILD, Peter, 1316 København K (DK)
(74) Representative: Høiberg P/S
(86) International application number: PCT/EP2018/080814
(87) International publication number: WO 2019/092201

(56) References cited:
- WO-A1-2017/129195
- WO-A2-2014/165105
- XIAO-FANG WANG ET AL: "Identification of sociodemographic and clinical factors associated with the levels of human β-defensin-1 and human β-defensin-2 in the human milk of Han Chinese", BRITISH JOURNAL OF NUTRITION, vol. 111, no. 05, 15 October 2013 (2013-10-15), UK, pages 867 - 874, XP055548998, ISSN: 0007-1145, DOI: 10.1017/S0007114513003292
- MICHAEL L. JENSEN ET AL: "Similar efficacy of human banked milk and bovine colostrum to decrease incidence of necrotizing enterocolitis in preterm piglets", AMERICAN JOURNAL OF PHYSIOLOGY - REGULATORY , INTEGRATIVE AND COMPARATIVE PHYSIOLOGY, vol. 305, no. 1, 1 July 2013 (2013-07-01), US, pages R4 - R12, XP055626998, ISSN: 0363-6119, DOI: 10.1152/ajpregu.00094.2013
- QINGFENG SHENG ET AL: "Human [beta]-defensin-3 promotes intestinal epithelial cell migration and reduces the development of necrotizing enterocolitis in a neonatal rat model", PEDIATRIC RESEARCH, vol. 76, no. 3, 23 June 2014 (2014-06-23), US, pages 269 - 279, XP055549554, ISSN: 0031-3998, DOI: 10.1038/pr.2014.93
- QINGFENG SHENG ET AL: "Human [beta]-defensin-3 promotes intestinal epithelial cell migration and reduces the development of necrotizing enterocolitis in a neonatal rat model", PEDIATRIC RESEARCH, vol. 76, no. 3, 23 June 2014 (2014-06-23), US, pages 269 - 279, XP055551284, ISSN: 0031-3998, DOI: 10.1038/pr.2014.93
- ANN CATHRINE F. STØY ET AL: "Necrotizing Enterocolitis in Preterm Pigs Is Associated with Increased Density of Intestinal Mucosa-Associated Bacteria Including <b><i>Clostridium perfringens</i></b>", NEONATOLOGY, vol. 108, no. 3, 7 August 2015 (2015-08-07), CH, pages 188 - 195, XP055551134, ISSN: 1661-7800, DOI: 10.1159/000431280
- QINGFENG SHENG ET AL: "Human [beta]-defensin-3 promotes intestinal epithelial cell migration and reduces the development of necrotizing enterocolitis in a neonatal rat model", PEDIATRIC RESEARCH, vol. 76, no. 3, 23 June 2014 (2014-06-23), US, pages 269 - 279, XP055551346, ISSN: 0031-3998, DOI: 10.1038/pr.2014.93
- KE ZHANG: "Evaluation of anti-human respiratory syncytial virus effects of short interfering RNAs and [beta]-defensin-4", THESIS, 1 January 2014 (2014-01-01), Hong Kong, XP055551128, Retrieved from the Internet <URL:http://hub.hku.hk/handle/10722/209570> DOI: 10.5353/th_b5312330
- ANN CATHRINE FINDAL STØY: "The effect of bovine colostrum products on intestinal dysfunction and inflammation in a preterm pig model of necrotizing enterocolitis", 20 October 2015 (2015-10-20), Denmark, pages FP1-4, 1-151, XP055548710, Retrieved from the Internet <URL:http://orbit.dtu.dk/files/54900213/Ann_Cathrine_F_St_y_Final_B5_thesis_2013.pdf> [retrieved on 20190129], DOI: 10.1002/mbo3.280
- XIAO-FANG WANG ET AL: "Concentration characteristics of bovine [beta]-defensin 1 and 2 in fresh bovine milk and infant formula", INTERNATIONAL JOURNAL OF DAIRY TECHNOLOGY, vol. 68, no. 2, 1 May 2015 (2015-05-01), GB, pages 299 - 301, XP055548836, ISSN: 1364-727X, DOI: 10.1111/1471-0307.12214
- JOANNA BARICELLI ET AL: "[beta]-defensin-2 in breast milk displays a broad antimicrobial activity against pathogenic bacteria", JORNAL DE PEDIATRIA, vol. 91, no. 1, 1 January 2015 (2015-01-01), BR, pages 36 - 43, XP055551294, ISSN: 0021-7557, DOI: 10.1016/j.jped.2014.05.006
- J. LEE ET AL: "Oropharyngeal Colostrum Administration in Extremely Premature Infants: An RCT", PEDIATRICS, vol. 135, no. 2, 26 January 2015 (2015-01-26), pages e357 - e366, XP055549030, ISSN: 0031-4005, DOI: 10.1542/peds.2014-2004
- DAVID KYLE MEYERHOLZ: "Developmental influence on respiratory epithelia to paramyxoviridae infection, beta-defensin expression and adenoviral delivery of a beta-defensin gene", 1 January 2004 (2004-01-01), pages FP1-6,1-148, XP055551247, ISBN: 978-0-496-04051-3, Retrieved from the Internet <URL:https://lib.dr.iastate.edu/cgi/viewcontent.cgi?article=2109&context=rtd> [retrieved on 20190204]
- ANDREAS C.W. JENKE ET AL: "Human [beta]-defensin 2 expression in ELBW infants with severe necrotizing enterocolitis", PEDIATRIC RESEARCH, vol. 72, no. 5, 17 August 2012 (2012-08-17), US, pages 513 - 520, XP055551305, ISSN: 0031-3998, DOI: 10.1038/pr.2012.110
- QINGFENG SHENG ET AL: "Human [beta]-defensin-3 promotes intestinal epithelial cell migration and reduces the development of necrotizing enterocolitis in a neonatal rat model", PEDIATRIC RESEARCH, vol. 76, no. 3, 23 June 2014 (2014-06-23), US, pages 269 - 279, XP055551351, ISSN: 0031-3998, DOI: 10.1038/pr.2014.93
- MICHAEL S. CAPLAN ET AL: "Role of Asphyxia and Feeding in a Neonatal Rat Model of Necrotizing Enterocolitis", PEDIATRIC PATHOLOGY., vol. 14, no. 6, 9 January 1994 (1994-01-09), US, pages 1017 - 1028, XP055551283, ISSN: 0277-0938, DOI: 10.3109/15513819409037698
- YINGYING HE ET AL: "Human Milk Components Modulate Toll-Like Receptor-Mediated Inflammation", ADVANCES IN NUTRITION, vol. 7, no. 1, 1 January 2016 (2016-01-01), United States, pages 102 - 111, XP055551609, ISSN: 2161-8313, DOI: 10.3945/an.115.010090
- ELIZABETH A. CRISTOFALO ET AL: "Randomized Trial of Exclusive Human Milk versus Preterm Formula Diets in Extremely Premature Infants", JOURNAL OF PEDIATRICS., vol. 163, no. 6, 1 December 2013 (2013-12-01), US, pages 1592 - 1595.e1, XP055461209, ISSN: 0022-3476, DOI: 10.1016/j.jpeds.2013.07.011
- ARMOGIDA SHEILA A ET AL: "Identification and quantification of innate immune system mediators in human breast milk", ALLERGY AND ASTHMA PROCEEDI, PROVIDENCE, RI, US, vol. 25, no. 5, 1 September 2004 (2004-09-01), pages 297 - 304, XP009150956, ISSN: 1088-5412

## Description

### Field of the invention

The present invention relates to an antimicrobial peptide selected from the group consisting of: hBD-2 (SEQ ID NO: 2), truncated hBD-2 (SEQ ID NO: 16), and functionally equivalent variants having 1-2 amino acid modification(s), for use in the prevention or treatment of necrotizing enterocolitis (NEC) in a preterm infant.

### Background

### Extreme low birth weight (ELBW) infants (birthweight < 1.000 gram).

At birth, all newborn infants have to adapt to the dramatic transition from a sterile life in the mother to an environment where environmental microbes colonize all epithelial surfaces of the body (e.g. gut, lungs, skin) within the first days and weeks after birth. Adaptation requires a well-developed innate immune system at epithelial surfaces, that allows tolerance to harmless or beneficial microbes and exclusion of harmful pathogens. After a normal gestation length, delivery process, maternal care and hygiene, newborn infants adapt well, but a markedly higher prevalence of infection, sepsis and inflammatory disorders in early life nevertheless shows that infants and children are very sensitive to the dramatic microbiological and immunological transition at birth.

Of the 15 million infants born preterm each year worldwide, more than 1 million die (Strunk, 2014). Many of these deaths relates to their immature ability to fight bacterial colonization at the gut, lung and skin surfaces. Infections are related to immature lung, skin and gut barrier functions and especially tolerance to the great number of bacteria along the gut surface from a few days after birth (≈10 billion) is a problem. These infections induce impaired gut function and pathogens/toxins may translocate into the blood stream, create sepsis and inflammatory reactions throughout the body. Morbidity is inversely related to gestational age, however there is no gestational age, including term that is wholly exempt. Although most organs are immature, the brain and lung are especially susceptible to the consequences of preterm birth, which leads to high rates of long-term neurological and health problems. In a review, more than half of ELBW children were readmitted to hospital at least once in the first 1-2 years of life, mostly as a result of respiratory illnesses (Doyle, 2003). Most studies of very low birth weight (VLBW) infants show continued sequelae such as cognitive deficits, academic underachievement, grade failures, and the need for increased remedial assistance during mid-childhood and adolescence (Saigal, 2008).

### Lung function

Normal lung development follows a series of orchestrated events. Premature birth interrupts normal in utero lung development, which results in significant alterations in lung function and physiology with an early transition from the hypoxic intrauterine environment to a comparatively hyperoxic environment (Colin, 2010) leading to respiratory distress syndrome, bronchopulmonary dysplasia, patent ductus arteriosus and chronic lung disease. Total lung volume undergoes rapid changes during the last trimester of gestation. Calculations by Langston, 1984 revealed that at 30 weeks, the lung volume is only 34% of the ultimate lung volume at mature birth. Immaturity of innate immunity contributes to the increased susceptibility of human neonates to infection. The lung is a major portal of entry for potential pathogens in the neonate. Alfa defensins, beta defensins and cathelicidin participate in the pulmonary innate immunity. Very few studies exist describing the role of antimicrobial peptides in neonate development but Tirone, 2010 found that preterm infants even with a gestational age of 30 weeks or less were able to produce alfa-defensins and Starner, 2015 found that treatment with the anti-inflammatory corticosteroid dexamethasone resulted in a significant decrease in hBD-2 expression.

### Infection and sepsis

Infections can be congenital or acquired. The congenital infections consist of pneumonia and chorioamnionitis attributable to maternal enteric organisms. The acquired infections are mainly caused by candida and nosocomial bacteria. Infection of the amniotic fluid leading to pneumonia is the major cause of death in the ELBW infants (Barton, 1999).

Bacterial translocation and endotoxemia caused by the loss of gut mucosal barrier function have been linked with organ injury, systemic inflammation and multiple organ failure. The gut mucosal barrier defense of premature and immune-incompetent infants offers imperfect protection against bacterial translocation and endotoxemia. Gastrointestinal distension is a common event in premature infants that may impair gut barrier function and hence initiate bacterial translocation (Sharma, 2007).

The risk of sepsis is inversely related to birth weight and gestational age. Infants with sepsis are at increased risk for a number of neonatal morbidities, for prolonged hospitalization and for death (Stoll, 2003). In the ELBW infant, invasive candidiasis leads to poor neurodevelopmental outcomes. Invasive candidiasis is the second most common cause of infectious disease related death in the extremely premature infant. Invasive candidiasis was found in 1.5% of VLBW infants. CNS candidiasis is under-investigated and difficult to diagnose but portends a very poor outcome (Barton, 2014). Previous treatment with antibiotics, presence of a central catheter or endotracheal tube were found to be strongly associated with invasive candidiasis (Benjamin, 2010).

In the past decade, several immune interventions aimed to prevent or improve the outcome of neonatal sepsis, such as colony-stimulating growth factors and intravenous immunoglobulin have been unsuccessful (Strunk, 2014).

### Necrotizing enterocolitis (NEC)

is a devastating gut inflammatory disease that occurs in 1-7% of all hospitalized preterm infants and is the second highest cause of mortality in preterms (Anand, 2007). The disease is characterized by bacterial overgrowth, dysregulated immunity and intestinal necrosis and is particularly prevalent in infants lacking breast-feeding due to the common maternal illness and lack of lactation. NEC appears to develop in the stressed newborn suffering from an immature innate and adaptive immune system after a disruption in the intestinal barrier and translocation of bacterial endotoxins (Sharma, 2007). The infants that survive NEC are at high risk of developing long-term complications, particularly neurodevelopmental impairment e.g. cerebral palsy (Strunk, 2014), as well as short bowel syndrome and impaired growth (Patel, 2012). Humoral mediators, including pro-inflammatory cytokines are likely to be key mediators in the pathogenesis of cerebral injury (Strunk, 2014).

NEC and sepsis often appear together but it remains unclear how gut bacterial dyscolonization and impaired immunity directly relate to bacterial translocation and the common complications of preterm birth, such as respiratory failure, cardiovascular abnormalities, impaired immunity, NEC and brain dysfunction (Sangild, 2013). The role of defensins in NEC is unclear. Salzman, 1998 originally found the expression of defensins and the number of Paneth cells to be higher in the neonatal gut of NEC patients. Puiman, 2011 on the other hand found that Paneth cell abundance in the small intestine was not significantly different in acute NEC from preterm controls. Richter, 2010 speculated that low fecal hBD-2 may be a risk factor in preterm infants to develop neonatal enteric disease, such as necrotizing enterocolitis. Jenke, 2012 found that low hBD-2 expression was associated with TLR4 expression, suggesting an inadequate response to luminal bacteria, possibly predisposing those infants to the development of NEC. Risk factors for NEC are gut immaturity, dysmolality, abnormal microbiota and increased permeability.

Støy, A.C.F (2012), discloses that in a preterm piglet model of necrotizing enterocolitis (NEC) induced by standard milk formula, the oral administration of bovine colostrum (BC) was found to restore intestinal dysfunction and reduce a proinflammatory response.

Antibiotics have been shown to modulate intestinal immunity and prevent NEC in a preterm neonatal piglet model (Jensen, 2014) and hBD-3 has been shown to decrease the incidence of NEC and survival in a neonatal rat model (Sheng, 2014). Manzoni, 2014 found the incidence of NEC to be significantly lower in VLBW infants treated with bovine lactoferrin plus *lactobacillus rhamnosus* versus placebo.

### Impaired neurodevelopment

Several studies in preterm infants show an association between late-onset sepsis and adverse neurodevelopmental outcomes in childhood, with repeated infections and Gram-negative pathogens conferring the highest risk. The association between sepsis and cerebral injury seems however to be largely independent of the bacterial species involved suggesting that a detrimental final common pathway can be activated by diverse initial host-microbe interactions (Strunk et. 2014). Examples of neurodevelopmental impairments in the early years are cerebral palsy, mental retardation, and sensory impairments such as visual and auditory deficits (Saigal, 2008).

The commonest lesion associated with inflammation in preterm infants is white-matter injury, which is characterized by focal cystic periventricular leukomalacia, diffuse necrosis or both (Strunk, 2014). Humoral mediators, including proinflammatory cytokines such as IL-1 and IL-6 and chemokines such as CXCL-8, TNF-α, type I and II interferons, and reactive oxygen species are likely to be key mediators in the pathogenesis of cerebral injury. Detrimental neurotoxic effects are not only induced by direct host-microbe interaction, but might also be generated by exposure to perinatal inflammation, activation of fetal or neonatal immune cells triggered by bacterial products that activate pattern recognition receptors, or maternal proinflammatory mediators that cross the placenta (Girard, 2009).

### Extrauterine growth restriction

Extrauterine growth restriction (infants with a weight below the 10^{th} percentile between birth and discharge) in VLBW infants affects their growth and development prognoses as well as their incidence of adult diseases (Sakurai, 2008). Sakurai found the incidence of extrauterine growth restriction to be 28%, 34% and 16% for weight, length, and head circumference, respectively. For each growth parameter, the incidence of extra uterine growth restriction increased with decreasing gestation and birth weight. In a cohort of infants weighing 600 g or less, it was reported that, at hospital discharge and 2 years of age, 94% were below the 10^{th} percentile for weight, length and head circumference. Abnormal neurodevelopmental outcome was found in 90% of survivors (Sweet, 2003).

### Neonatal microbiome

The microbiome evolves within a healthy host from birth to death, constantly fine-tuning it to maintain homeostatic balance with the host's immune system. The first and most important contribution to the genesis of the microbiome is vertical transmission of maternal microbiota. Colonization of mucosa in the digestive, respiratory, urogenital tracts as well as the skin begins at or perhaps even before, the time of birth when a newborn is exposed to a mother's microbiota. Recent studies suggest the presence of a microbiome within the placenta as well as fetal meconium, suggesting that the colonization process begins well before delivery.

The gastrointestinal tract has the greatest diversity and abundance of microbes. More than 99% of the gut microbes are anaerobes. Infants born via vaginal delivery have intestinal colonization reflective of maternal vaginal flora such as Lactobacillus and Prevotella species. Infants born via Caesarian delivery are colonized by epidermal rather than vaginal species such as Clostridium, Staphylococcus, Propionebacterium and Corynebacterium. Further development of the neonatal gut microbiome after birth, regardless of mode of delivery is governed by interaction between the microbiota and the host's immune system. The progression of how this evolves remains incompletely characterized. Nutrition, be it in breast milk or formula, has been demonstrated to play a major role in early colonization patterns of the neonatal gut microbiota (Gritz and Bandari, 2015). Interestingly, even relatively small amounts of formula supplementation of breast-fed infants will result in shifts from a breast-fed to a formula-fed pattern (Guaraldi and Salvatori, 2012).

Preterm infants, especially ELBW infants, are at a disadvantage when it comes to development of a healthy microbiome. Factors contributing to this are not limited to their gut immaturity, but also include preterm ruptured membranes, maternal infection, increased incidence of Caesarian delivery, perinatal and postnatal broad spectrum antibiotic exposure as well as exposure to other gut-modifying medications (Gritz and Bandari, 2015). A 2007 study by Butel found that healthy full-term breastfed infants are colonized by Bifidobacterium by day 7 of life, whereas preterm infants are not. Interestingly, they also suggest that there may be gestational age thresholds for colonization with certain microbes - 33 weeks appears to be the milestone for appearance of Bifidobacterium, the organism most commonly implicated in development and maintenance of a healthy microbiome.

### Colostrum

Human milk protects against infections in the breastfed offspring mainly via the secretory IgA antibodies, but also most likely several other factors like the bactericidal lactoferrin. Protection against infections has been well evidenced during lactation against, e.g. acute and prolonged diarrhea, respiratory tract infections, otitis media, urinary tract infection, neonatal sepsis and NEC (Hanson, 1998).

Consumption of human milk provides passive immunity to the newborn GI tract through a large number of soluble and cellular components. Milk contains antimicrobial proteins and peptides including lactoferrin, lysozyme and defensins, which modulate the gut microbiome. In addition to antimicrobial activity, breast milk may contribute to maintenance of the barrier function of the GI tract, through growth factors and cytokines, or to the defense of the GI epithelium through soluble pattern recognition receptors such as secretory IgA and soluble CD-14 receptor, which may prevent bacterial attachment to enteric tissues through steric hindrance (Trend, 2016). The concentration of protein and immune factors such as slgA and leucocytes are reported to be altered by maternal age, mode of delivery, the volume of milk produced, smoking, BMI, parity and maternal infection. Many of these maternal parameters are also risk factors associated with preterm birth (Trend, 2016).

Armogida, 2004 found the highest concentrations of HNP-1 and HBD-2; moderate amounts of HD6 and low amounts and HD5 and HBD-1 in colostrum. HNP-1, HD5 and HD6 were present in significantly higher amounts in colostrum compared with mature milk. There was a trend toward higher levels of HBD-2 in breast milk from women who delivered premature infants when compared with those who delivered at term. Trend recently found the most abundant proteins measured to be lactoferrin, slgA, lysozyme and sCD-14. Defense peptides HBD-1 and HBD-2 were present in lower quantities.

Median values of IL-10, IL-13, TNF-α, IFN-γ and HD5 were at the limit of detection, as these molecules were measurable in fewer than half of the breast milk samples. Introduction of suboptimal enteral formula diets coupled with parenteral nutrition, predispose to disease, while advancing amounts of mothers milk from birth and in particular colostrum protects against disease (Siggers, 2010).

### Current treatment of preterm infants

With increasing and earlier use of antenatal corticosteroids, assisted ventilation and surfactant, and changing attitudes towards intensive care, survival rates for very preterm births, especially those born before 28 week's gestation, has improved strikingly since the mid 1990's (Saigal, 2008).

A Cochrane analysis from 2014 concluded that supplementation of the milk diet with enteral probiotics reduced the risk of severe NEC as well as mortality (AlFaleh, 2014). Probiotics are known to have several important effects on a cellular level - NFκB activation, upregulation of cytoprotective genes, prevention of apoptosis, generation of reactive oxygen species and expression of tight junctions (Patel, 2012). Several reports of probiotic associated sepsis have however raised concerns regarding routine clinical use of live bacteria in hosts, such as premature infants, who have immature epithelial-barrier defenses (Patel, 2012).

### Antimicrobial peptides

At birth, the immune system, especially the adaptive immunity, is immature, and characterized by low levels of immunoglobulins, naïve T cells and antigen presenting cells. The current hypothesis is that certain innate immune cells, which harbour effector molecules such as antimicrobial peptides and proteins, compensate to some degree for this impairment and play a major role in protecting against microbes in early life (Kai-Larsen, 2014). Antimicrobial peptides are already expressed when the foetus is in the womb, but the levels increase with gestational age. HD5 and HD6 transcripts have already appeared at 13 weeks of gestation (Mallow, 1996) and low levels of HD5 have been detected in the small intestine at 24 weeks of gestation (Salzman, 1998). Campeotto, 2010 found that hBD-2 can be detected at high level in the feces of full-term and preterm infants, independently of gestational age or mode of feeding.

In conclusion, there is a big need for new treatments of infants born prematurely. There is a particular need for treatments that can facilitate the maturation of the mucosal defense in both the lungs and intestines simultaneously. There is a need for a treatment that can be administered through both oral, pulmonary and subcutaneous administration.

### Summary

The invention is defined by the claims. Any subject matter falling outside the scope of the claims is provided for information purposes only.

The present invention concerns an antimicrobial peptide selected from the group consisting of: hBD-2 (SEQ ID NO: 2), truncated hBD-2 (SEQ ID NO: 16), and functionally equivalent variants having 1-2 amino acid modification(s), for use in the prevention or treatment of necrotizing enterocolitis (NEC) in a preterm infant.

The references to methods of treatment in this description are to be interpreted as references to the hBD-2 (SEQ ID NO: 2), truncated hBD-2 (SEQ ID NO: 16), and functionally equivalent variants having 1-2 amino acid modification(s) for use in the method for the treatment of the human (or animal) body as described in the claims.

The inventor has surprisingly demonstrated that mammalian defensins have the ability to change the gut microbiota through an increase of microbial presence and abundance; to increase mucosal integrity through an increase in IL-22 concentration; to reduce the incidence of necrotizing enterocolitis; to reduce airway hyperresponsiveness (AHR) and increase airway compliance (Cdyn); particularly to improve lung function through a reduction of AHR and increase of airway compliance (Cdyn); to reduce lung inflammation; to reduce neutrophil -, eosinophil - and macrophage count in bronchio-alveolar-lavage-fluid (BALF) as well as rebalance the immune system through normalization of IFN-γ, TNF-α, IL-4, IL-5, IL-6, IL-9, IL-10 and IL-13 concentrations. The inventor has also demonstrated efficacy in reduction of histological inflammation in an asthma model. Particularly, the inventor has also demonstrated efficacy in reduction of histological inflammation in the lungs in an asthma model as well as in the intestines and liver in a model of graft versus host disease.

The data indicate that administration of the peptides for the use according to the claims results in reduction of the major complications associated with necrotizing enterocolitis (IDC10; P77) in preterm infants.

Surprisingly it has been demonstrated in both a preventive and a therapeutic high fat diet mouse model that both α- and β- defensins have a strong influence on the composition of the microbiota but also that these effects are different from one defensin to another and that the combination of an α- and a β-defensin is yet again different from the individual effects.

It has further surprisingly been demonstrated in a mouse model of Dithizone/Klebsiella induced necrotizing enterocolitis that β-defensins can prevent or dramatically reduce the incidence of necrotizing enterocolitis possibly facilitated through increased mucosal integrity by increase of IL-22 production.

It has further surprisingly been demonstrated in a mouse model of house dust mite allergy that a dosage of human beta-defensin 2 (hBD-2) whether administered orally or intranasally is capable of preventing the development of asthma and impaired lung function as defined by increased AHR, decreased Cdyn, inflammatory cell count in BALF and inflammatory cytokine production in lung tissue in a steroid-sensitive murine model, where mice are immunized by house dust mite (HDM) + Freund's adjuvant and challenged with HDM. Contrary to conventional asthma prophylaxis, where immune suppression is the target, prophylaxis with hBD-2 seems to rebalance the immune system and thus maintain a well-functioning immune response.

It has further surprisingly been demonstrated in a mouse model of house dust mite allergy that a dosage of human beta-defensin 2 (hBD-2) whether administered orally or intranasally is also capable of treating asthma and improve lung function by decreasing AHR, increasing Cdyn, reducing histological lung inflammation, reducing inflammatory cell count in BALF and inflammatory cytokine production in lung tissue in a steroid-sensitive murine model, where mice are immunized by house dust mite (HDM) + Freund's adjuvant and challenged with HDM.

Without hBD-2 treatment, animals developed asthma characterized by dramatically increased AHR, decreased Cdyn, inflammatory histological changes of the lung tissue, increased white blood cell count, in particular neutrophils, eosinophils and macrophages and increased concentration of inflammatory cytokines.

It has further surprisingly been demonstrated in human PBMC's, a marker of systemic toxicity, that the systemic toxicity of defensins varies. hBD-1, hBD-2 and HD5 were found not to be toxic in PBMC's. hBD-4 was found to be moderately toxic, whereas hBD-3 was found to be toxic. This is an important and surprising finding as Sheng, 2014 found hBD-3 not to be toxic, when tested in gut epithelial cell lines. Shengs finding was not surprising as the beta defensins are expressed on epithelial surfaces. The epithelial surfaces in the preterm infant e.g. the gut are however immature and leaky and orally administered defensins are thus likely to be absorbed and exert their effect systemically.

It has further surprisingly been demonstrated in a murine model of graft versus host disease, a model that shares a number of similarities to the preterm infant that prophylactic treatment with hBD-2 dramatically decreased mortality and weight loss; decreased the histology score of the small and large intestine as well as the liver all suggesting improved gut health and gut integrity. Treatment with hBD2 reduced the infiltration with CD45+ leucocytes in gut epithelium/lamina propria and reduced intestinal T cell and myeloid cell infiltration. Prophylactic treatment with hBD2 also showed reduction of TNF-α and IL-6 and induction of IL-10. The hBD-2 treatment additionally showed a reduction of IL-1β from myeloid cells (FACS analysis of the spleen and reduced Th1 cytokine especially TNF-α and IFN-γ production.

### Description of Drawings

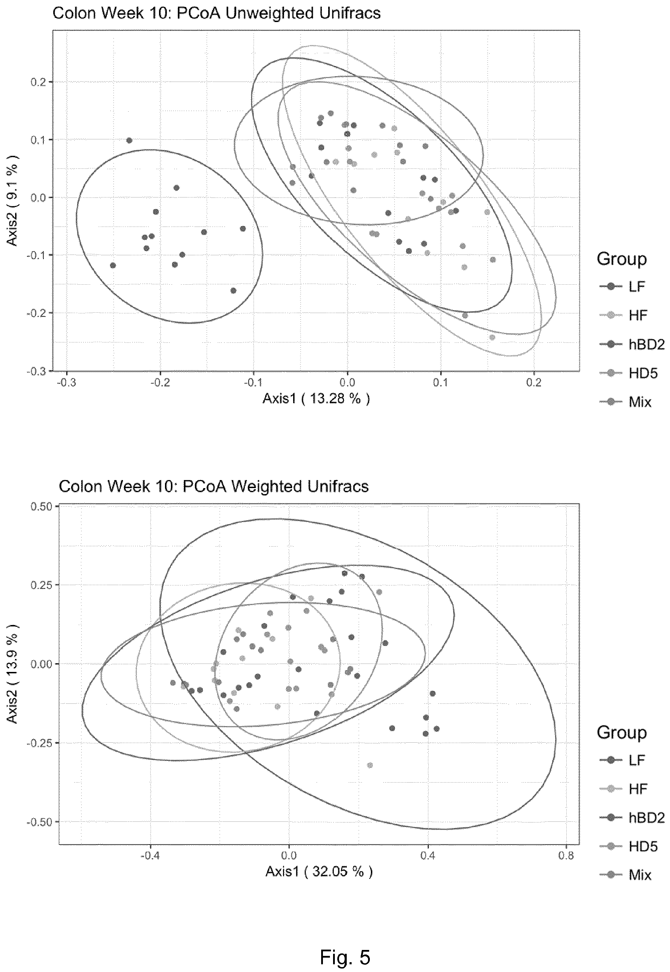

Figure 1.
   Schematic outline of the experimental setup for investigating the effects of mammalian defensins (HD5, hBD-2 and HD5+hBD-2) on the composition of the microbiota in a high fat diet murine model.
Figure 2.
   Schematic outline of the experimental set up for investigating the effects of prophylactic treatment with mammalian defensins in a murine steroid-sensitive model for prevention of asthma, where the mice are immunized by house dust mite (HDM) + Freund's adjuvant and challenged with HDM.
Figure 3.
   Schematic outline of the experimental set up for investigating the effects of treatment with mammalian defensins in a murine steroid-sensitive model for treatment of asthma, where the mice are immunized by house dust mite (HDM) + Freund's adjuvant and challenged with HDM.
Figure 4.
   Clustal W (2.1) multiple sequence alignment of A) human beta defensin 1-4 and B) : HD5 and HD6
   In the Clustal W alignments:
   - *: indicates positions which have a single, fully conserved residue.
   - :: indicates that one of the following 'strong' groups is fully conserved: -S,T,A; N,E,Q,K; N,H,Q,K; N,D,E,Q; Q,H,R,K; M,I,L,V; M,I,L,F; H,Y; F,Y,W.
   - .: indicates that one of the following 'weaker' groups is fully conserved: -C,S,A; A,T,V; S,A,G; S,T,N,K; S,T,P,A; S,G,N,D; S,N,D,E,Q,K; N,D,E,Q,H,K; N,E,Q,H,R,K; V,L,I,M; H,F,Y.
Figure 5.
   Unweighted and weighted unifrac analysis of microbial presence (describing the variety of bacteria present) and abundance (describing the amount of a given bacteria present as a percentage of all bacteria present) following prophylactic treatment with oral HD5, hBD-2 and HD5+hBD-2 (mix) in a murine high fat diet model. Untreated high fat diet (HF) and low fat diet (LF) are used as controls.
Figure 6.
   Genus analysis of microbial abundance following prophylactic treatment with oral HD5, hBD-2 and HD5+hBD-2 (mix) in a murine high fat diet model.
Figure 7.
   Abundance of Allobaculum in the small intestine following prophylactic treatment with oral HD5 and hBD-2 in a murine high fat diet model.
Figure 8.
   Abundance of Lactobacillaceae in colon following prophylactic treatment with oral hBD-2 in a murine high fat model.
Figure 9.
   Relative abundance of Barnesiella in colon following 4 and 10 weeks of prophylactic treatment with oral hBD-2 in a murine high fat diet model.
Figure 10.
   Unweighted unifrac analysis of microbial presence and abundance (see text to figure 5) following therapeutic treatment with HD5 or hBD-2 in a murine high fat diet model.
Figure 11.
   Relative abundance of Alloprevotella in colon following therapeutic intervention with oral HD5 and hBD-2 in a murine high fat diet model.
Figure 12.
   Relative abundance of Bifidobacteriaceae in the small intestine and colon following therapeutic intervention with HD5 or hBD-2 in a murine high fat diet model.
Figure 13.
   Clinical score (body weight loss, stool consistency and presence of blood per rectum) in a murine 14-week therapeutic SCID CD4+CD25+ Tcell transfer colitis model showing effect of hBD-2, 1 mg/kg s.c. OD on par with 100 µg/mouse anti TNF-α s.c. twice weekly (Enbrel) and 0.3 mg/kg dexamethasone intraperitoneally OD.
Figure 14.
   Colon weight in a murine 14-week therapeutic SCID CD4+CD25+ Tcell transfer colitis model showing effect of hBD-2, 1 mg/kg s.c. OD on par with 100 µg/mouse anti TNF-α s.c. twice weekly (Enbrel) and 0.3 mg/kg dexamethasone intraperitoneally OD.
Figure 15
   Myeloperoxidase activity in intestinal tissue as an expression of gut health in a murine 14-week therapeutic SCID CD4+CD25+ Tcell transfer model of colitis showing effect of 1 mg/kg s.c. hBD-2 on par with 100 µg/mouse anti TNF-α s.c. twice weekly (Enbrel) and 0.3 mg/kg dexamethasone intraperitoneally OD.
Figure 16:
   Airway hyper responsiveness in the murine House Dust Mite steroid-sensitive asthma model following prophylactic intranasal and oral administration of hBD-2 respectively.
Figure 17:
   Pulmonary compliance in the murine House Dust Mite steroid-sensitive asthma model following prophylactic intranasal and oral administration of hBD-2 respectively.
Figure 18.
   Cytokine concentrations of TNF-αin lung homogenate in the House Dust Mite murine steroid-sensitive asthma model following prophylactic intranasal (A) and oral (B) administration of hBD-2 respectively.
Figure 19
   Cytokine concentrations of IL-4 in lung homogenate in the House Dust Mite murine steroid-sensitive asthma model following prophylactic intranasal (A) and oral (B) administration of hBD-2 respectively.
Figure 20
   Cytokine concentrations of IL-5 in lung homogenate in the House Dust Mite murine steroid-sensitive asthma model following prophylactic intranasal (A) and oral (B) administration of hBD-2 respectively.
Figure 21
   Cytokine concentrations of IL-6 in lung homogenate in the House Dust Mite murine steroid-sensitive asthma model following prophylactic intranasal (A) and oral (B) administration of hBD-2 respectively.
Figure 22
   Cytokine concentrations of IL-9 in lung homogenate in the House Dust Mite murine steroid-sensitive asthma model following prophylactic intranasal (A) and oral (B) administration of hBD-2 respectively.
Figure 23
   Cytokine concentrations of IL-13 in lung homogenate in the House Dust Mite murine steroid-sensitive asthma model following prophylactic intranasal (A) and oral (B) administration of hBD-2 respectively.
Figure 24
   Cytokine concentrations of IL-33 in lung homogenate in the House Dust Mite murine steroid-sensitive asthma model following prophylactic intranasal (A) and oral (B) administration of hBD-2 respectively.
Figure 25:
   Airway hyper responsiveness in the House Dust Mite murine steroid-sensitive asthma model following therapeutic intranasal (A) and oral (B) administration of hBD-2 respectively. Saline is the non-challenged control. HDM/Vehicle is the House Dust Mite challenged control treated with vehicle.
   "hBD-2 IN 1.2 mpk" is hBD-2 administered intranasally at 1.2 mg/kg. 5mpk is 5 mg/kg.
Figure 26:
   Pulmonary compliance in the House Dust Mite murine steroid-sensitive asthma model following therapeutic intranasal (A) and oral (B) administration of hBD-2 respectively.
Figure27:
   Total and differential cell count in BALF in the House Dust Mite murine steroid-sensitive asthma model following therapeutic intranasal administration of hBD-2
Figure 28.
   Cytokine concentrations of IFN-γ in lung homogenate in the House Dust Mite murine steroid-sensitive asthma model following therapeutic intranasal and oral administration of hBD-2 respectively. Data from the intranasal arm is shown on the left and data from the peroral arm is shown on the right.
Figure 29
   Cytokine concentrations of TNF-α in lung homogenate in the House Dust Mite murine steroid-sensitive asthma model following therapeutic intranasal and oral administration of hBD-2 respectively. Data from the intranasal arm is shown on the left and data from the peroral arm is shown on the right.
Figure 30
   Cytokine concentrations of IL-6 in lung homogenate in the House Dust Mite murine steroid-sensitive asthma model following therapeutic intranasal and oral administration of hBD-2 respectively.
Figure 31
   Cytokine concentrations of IL-9 in lung homogenate in the House Dust Mite murine steroid-sensitive asthma model following therapeutic intranasal and oral administration of hBD-2 respectively.
Figure 32
   Cytokine concentrations of IL-4 in lung homogenate in the House Dust Mite murine steroid-sensitive asthma model following therapeutic intranasal and oral administration of hBD-2 respectively. Data from the intranasal arm is shown on the left and data from the peroral arm is shown on the right.
Figure 33
   Cytokine concentrations IL-5 in lung homogenate in the House Dust Mite murine steroid-sensitive asthma model following therapeutic intranasal and oral administration of hBD-2 respectively.
Figure 34
   Cytokine concentrations of KC (IL-8) in lung homogenate in the House Dust Mite murine steroid-sensitive asthma model following therapeutic intranasal and oral administration of hBD-2 respectively.
Figure 35
   Cytokine concentrations of IL-10 in lung homogenate in the House Dust Mite murine steroid-sensitive asthma model following therapeutic intranasal and oral administration of hBD-2 respectively. Data from the intranasal arm is shown on the left and data from the peroral arm is shown on the right.
Figure 36
   Cytokine concentrations of IL-13 in lung homogenate in the House Dust Mite murine steroid-sensitive asthma model following therapeutic intranasal and oral administration of hBD-2 respectively.
Figure 37.
   Lung histology with H&E/PAS preparation in the House Dust Mite murine steroid-sensitive asthma model following therapeutic intranasal and oral administration of hBD-2 respectively. Upper left panel: untreated and unchallenged control. Upper right panel: untreated and HDM challenged control. Lower left panel: HDM challenged treated with hBD-2 PO. Lower right panel: HDM challenged treated with hBD-2 IN. 50X enlargement.
Figure 38.
   Lung inflammation severity in the House Dust Mite murine steroid-sensitive asthma model following therapeutic intranasal and oral administration of hBD-2 respectively.
Figure 39.
   Cell viability of PBMC cells following exposure for 24 hours to Alamar Blue and HD5, hBD-1, hBD-2, hBD-3 and hBD-4 at concentrations of 1, 10 and 40 µg/ml.
Figure 40.
   Histological scoring system for evaluation of severity of necrotizing enterocolitis in a murine model of Klebsiella/Dithizone induced necrotizing enterocolitis. > 2 indicates significant gut inflammation.
Figure 41.
   A statistically significant reduction of the incidence of necrotizing enterocolitis is noted 16 hours post challenge in the mice that received one dose of 1.2 mg/kg hBD-2 one hour post challenge with Klebsiella/Dithizone.
Figure 42.
   A statistically significant gut mucosa protective increase of IL-22 is noted 9 hours post challenge in the group of mice that received one dose of 1.2 mg/kg hBD-2 one hour post challenge with Klebsiella/Dithizone.
Figure 43.
   A highly statistically significant reduction of mortality (p < 0.0001) in 15 mice treated with oral hBD-2 for 10 days from the day of stem cell transplantation compared with 15 mice treated with PBS.
Figure 44.
   A statistically significant reduction of histology score of the small intestine, colon and liver in 10 mice treated with oral hBD-2 for 10 days from the day of stem cell transplantation.
Figure 45.
   Weight loss in % from baseline (a) and in gram (b) in mice treated with oral hBD-2 for 10 days from the day of stem cell transplantation.
Figure 46.
   Reduced CD45+ leucocyte cell migration into lamina propria of colon and small intestine in 10 mice treated with oral hBD-2 for 10 days from the day of stem cell transplantation.
Figure 47 a-c.
   Reduction of intestinal T cell and myeloid cell infiltration of the lamina propria of the colon and small intestine in 10 mice treated with oral hBD-2 for 10 days from the day of stem cell transplantation.
Figure 48 a-c.
   Cytokine concentrations of TNF-α (a), IL-6 (b) and IL-10 (c) in serum of 10 mice treated with oral hBD-2 for 10 days from the day of stem cell transplantation.
Figure 49 a-c.
   Reduced IL-1β production in myeloid cells in 10 mice treated with oral hBD-2 for 10 days from the day of stem cell transplantation.
Figure 50.
   Reduced proportion of neutrophils (a) and reduced Th 1 cytokine production - IFN-γ in CD4 T cells (b) and CD8 T cells (c) and CD69+CD4 T cells (d); TNF-α in CD4 T cells (e) and CD8 T cells (f) in 10 mice treated with oral hBD-2 for 10 days from the day of stem cell transplantation.
Figure 51.
   Increased inflammation, myeloid cell and leucocyte infiltration as well as increased tissue repair in colon in 10 mice treated with oral PBS for 10 days from the day of stem cell transplantation.
Figure 52.
   A statistically significant reduction of mortality (p = 0.03) in 7 mice treated with oral hBD-2 for 10 days from the day of stem cell transplantation compared with 13 mice treated with cyclosporine and PBS respectively.
Figure 53.
   Weight loss in % from baseline in mice treated with oral hBD-2, cyclosporine or PBS for 10 days from the day of stem cell transplantation.
Figure 54.
   A significant reduction of mortality in 22 mice treated with oral hBD-2, oral HD5 or PBS respectively for 10 days from the day of stem cell transplantation.
Figure 55.
   Bacterial population control exerted by orally administered HD5 and hBD-2 measured as the distance from the intestinal lining to bacterial population (lysis zone) in µm in mice compared with mice on a low fat or western diet.

### Detailed description

### Definitions:

The term "defensin" as used herein refers to polypeptides belonging to the defensin class of antimicrobial peptides. Defensins represent one of the dominant innate host defences that serve to maintain a healthy microbiome and ward off potential pathogens (Wehkamp et al.et al., 2002 and Salzman et al., 2007). Defensins are peptides possessing antimicrobial activity against Gram positive and negative bacteria, fungi and archaea as well as exerting anti-inflammatory activity.

Human defensins are small cationic peptides divided into α- and β-defensins based on the topology of their three intramolecular cysteine disulphide bonds. α-defensins can be further subdivided into those expressed in intracellularly in neutrophil granules (HNP1-4) and those expressed by Paneth cells in the crypts of the small intestine (HD5 and HD6 or DEFA5 and DEFA6). β-defensins (DEFBn) are mainly produced by epithelial cells in various tissues and organs including the skin, eye, middle ear, mouth, trachea, lungs, gastrointestinal tract, urogenital system, kidneys, vagina, liver, pancreas and mammary glands. Examples of defensins include human intestinal alpha defensin 5 (HD5; SEQ ID NO: 5); human intestinal alpha defensin 6 (HD6; SEQ ID NO: 6); human neutrophil peptide 1 (HNP-1; SEQ ID NO: 8); human neutrophil peptide 2 (HNP-2; SEQ ID NO :9); human neutrophil peptide 3 (HNP-3; SEQ ID NO: 10); human neutrophil peptide 4 (HNP-4; SEQ ID NO: 11) all belonging to the alfa defensin class; and also human beta defensin 1 (hBD-1; SEQ ID NO: 1); human beta defensin 2 (hBD-2; SEQ ID NO: 2); human beta defensin 3 (hBD-3; SEQ ID NO: 3); human beta defensin 4 (hBD-4; SEQ ID NO: 4); mouse beta defensin 3 (SEQ ID NO: 7); and truncated hBD-2 (SEQ ID NO: 16). Defensins are expressed as precursors and are processed by cleavage of the signal peptide and in some cases pro-peptides as well before secretion into the extracellular space. The best characterized members of the human β-defensin family are hBD-1-4. Some of the human defensins e.g. hBD-1 are produced constitutively, whereas others e.g. hBD-2, hBD-3 and hBD-4 are induced by pro-inflammatory cytokines or exogenous microbial products. The above-identified sequences represent the predicted mature bioactive defensins. It will be understood by one of skill in the art that processing may differ from cell to cell and that the resulting secreted mature peptide may differ by one or two C- or N-terminal amino acids from the predicted sequences and still retain bioactivity.

The term "Cathelicidin", also known as LL-37, as used herein relates to a family of antimicrobial peptides found in lysosomes of macrophages and Granulocyte/polymorphonuclear leukocytes. Cathelicidins serve a critical role in mammalian innate immune defense against invasive bacterial infection. The cathelicidin family of peptides are classified as antimicrobial peptides (AMPs). The AMP family also includes the defensins. Whilst the defensins share common structural features, cathelicidin-related peptides are highly heterogeneous. Members of the cathelicidin family of antimicrobial polypeptides are characterized by a highly conserved region (cathelin domain) and a highly variable cathelicidin peptide domain. One example is human cathelicidin (LL-37, SEQ ID NO: 12).

The term "Lactoferrin", also known as lactotransferrin, as used herein is related to a multifunctional protein of the transferrin family. Lactoferrin is a globular protein with a molecular mass of about 80 kDa that is widely represented in various secretory fluids, such as milk, saliva, tears, and nasal secretions. Lactoferrin is also present in secondary granules of neutrophil granulocytes and is secreted by acinar cells. Lactoferrin can be purified from milk or produced recombinantly. Human colostrum has the highest concentration, followed by human milk, then cow milk (150 mg/L). Lactoferrin is one of the components of the immune system of the body; it has antimicrobial activity (bacteriocidal, fungicidal) and is part of the innate defense. One example is human lactoferrin (SEQ ID NO: 13)

The term "Lactoferricin" as used herein relates to an amphipathic, cationic peptide with anti-microbial properties. It can be generated by pepsin-mediated digestion of lactoferrin. One example is lactoferricin-H (SEQ ID NO: 14)

The term "Lysozyme", also known as muramidase or N-acetylmuramide glycanhydrolase, as used herein refers to an antimicrobial enzyme produced by animals and forms part of the innate immune system. Lysozyme is a glycoside hydrolase that catalyzes the hydrolysis of 1,4-beta-linkage between N-acetylmuramic acid and N-acetyl-D-glucosamine residue in peptidoglycan, which is the major component of the gram-positive bacterial cell wall. This hydrolysis in turn compromises the integrity of bacterial cell walls causing lysis of the bacteria. Lysozyme is abundant in secretions including tears, saliva, human milk, and mucus. It is also present in cytoplasmic granules of the macrophages and the polymorphonuclear neutrophils. One example is human lysozyme (SEQ ID NO: 15)

The term "identity" as used herein refers to the relatedness between two amino acid sequences or between two nucleotide sequences. The degree of identity between two amino acid sequences is determined using the Needleman-Wunsch algorithm (Needleman and Wunsch, 1970, J. Mol. Biol. 48: 443-453) as implemented in the Needle program of the EMBOSS package (Rice *et* al.,2000, http://emboss.org), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the nobrief option) is used as the percent identity and is calculated as follows: (Identical Residues x 100)/(Length of Alignment - Total Number of Gaps in Alignment).

The term "normal microbiota" is used herein to indicate a microbiota that is not dysbiotic. Normal microbiota is characterized by having large gene richness.

Normal intestinal microbiota is characterized by comprising bacteria belonging to the genera *Bacteriodetes, Faecalibacterium, Roseburia, Blautia, Ruminococcus, Coprococcus, Bifidobacterium, Methanobrevibacter, Lactobacillus, Coprococcus, Clostridium, Akkermansia, Eubacterium.*

Normal lung microbiota is characterized by comprising bacteria belonging to the genera *Bacteroidetes, Firmicutes,* and *Proteobacteria* with the core microbiota consisting of *Pseudomonas, Streptococcus, Prevotella, Fusobacteria, Veillonella, Haemophilus, Neisseria* and *Porphyromonas*

The term "improving intestinal health", as used herein, refers to an increase in the mucosal proportion of the gut wall, increase in the height of the intestinal villi, increase in the density of intestinal goblet cells, increase in the digestive brush border enzyme activity and lower intestinal myeloperoxidase activity

The term "white-matter injury", as used herein, is characterized by focal cystic periventricular leukomalacia, diffuse necrosis or both.

The term "sensory impairments", as used herein, refers to conditions such as visual and auditory deficits.

The terms "treatment" and "treating" as used herein refer to the management and care of a patient for the purpose of combating a condition, disease or disorder. The term is intended to include the full spectrum of treatments for a given condition from which the patient is suffering, such as administration of the active compound for the purpose of: alleviating or relieving symptoms or complications; delaying the progression of the condition, disease or disorder; curing or eliminating the condition, disease or disorder; and/or preventing the condition, disease or disorder, wherein "preventing" or "prevention" is to be understood to refer to the management and care of a patient for the purpose of hindering, reducing the active compounds to prevent or reduce the risk of the onset of symptoms or complications. The patient to be treated is preferably a mammalian, in particular a human being. The term "patient" as used herein refers to a prematurely born infant or to a woman who is about to give birth prematurely (preterm labor).

The term "preterm infant", as used herein, refers to an infant who has been born before gestational age of 37 weeks, such before gestational age of 35 week, for example 30 weeks, such as 25 weeks. The term includes VLBW (very low birth weight) infants with a birth weight below 1,500 g and ELBW (extremely low birth weight) infants with a birth weight below 1,000 g.

The term "woman about to give birth to a premature infant", as used herein, refers to a pregnant woman who is in labor and about to give birth to an infant who has a gestational age of 37 or less, such a gestational age of 35 week or less, for example 30 weeks or less, such as 25 weeks or less. The woman about to give birth to a premature infant may also be a pregnant woman who has been diagnosed with a particular disorder such as an inflammatory disorder of the lungs or the intestines, suffers from particular symptoms indicative of a disorder, such as an inflammatory disorder of the lungs or the intestines. Such a pregnant woman is considered to be at risk of giving premature birth.

### hBD-2 (SEQ ID NO: 2), truncated hBD-2 (SEQ ID NO: 16), and functionally equivalent variants having 1-2 amino acid modification(s).

This invention relates to an antimicrobial peptide selected from the group consisting of: hBD-2 (SEQ ID NO: 2), truncated hBD-2 (SEQ ID NO: 16), and functionally equivalent variants having 1-2 amino acid modification(s), for use in the prevention or treatment of necrotizing enterocolitis (NEC) in a preterm infant.

In a preferred embodiment, the antimicrobial peptide is hBD-2.

In another embodiment, the antimicrobial peptide differs from SEQ ID NO: 2, by less than 3 amino acids, such as less than 2 amino acids..

In a preferred embodiment, the peptide for the use according to the claims consists of human beta defensin 2 (SEQ ID NO: 2).

More preferably, the peptide for the use according to the claims consists of human beta defensin 2, and functionally equivalent variants thereof having 1-2 amino acid modification(s).

A "functionally equivalent variant" of a mammalian (e.g. human) alfa or beta defensin is a modified mammalian alfa or beta defensin exhibiting approximatively the same effect on microbiota in the lung or the intestine or the skin as the parent mammalian alfa and/or beta defensins. A functionally equivalent variant comprises 1-2 amino acid modification(s), and in particular one amino acid modification, as compared to the mammalian defensin amino acid sequence of SEQ ID NO: 2 and SEQ ID NO: 16. Preferablycompared to human beta defensin 2, having SEQ ID NO: 2.

The term "modification" means herein any chemical modification of the peptides for the use according to the claims. The modification(s) can be substitution(s), deletion(s) and/or insertions(s) of the amino acid(s) as well as replacement(s) of amino acid side chain(s); or use of unnatural amino acids with similar characteristics in the amino acid sequence. In particular the modification(s) can be amidations, such as amidation of the C-terminus. Preferably, amino acid modifications are of a minor nature, that is conservative amino acid substitutions or insertions that do not significantly affect the folding and/or activity of the polypeptide; single deletions; small amino- or carboxyl-terminal extensions; or a small extension that facilitates purification by changing net charge or another function, such as a poly-histidine tag, an antigenic epitope or a binding domain. In one embodiment the small extension, such as a poly-histidine tag, an antigenic epitope or a binding domain is attached to the mammalian alfa or beta defensin through a small linker peptide of up to about 20-25 residues and said linker may contain a restriction enzyme cleavage site.

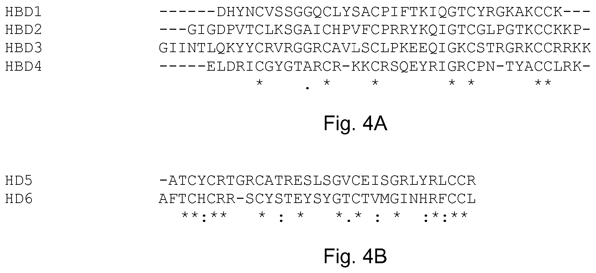

The Clustal W alignments in Figure 4 can be used to predict which amino acid residues can be substituted without substantially affecting the biological activity of the protein. The sequences were aligned using Clustal W 2.1
(http://www.qeno,me.jp/tools/clustalw/ ) and the following settings: Gap Open Penalty:10, Gap Extension Penalty: 0,05, Weight Transition: NO, Hydrophilic Residues for Proteins: GPSNDQE, Hydrophilic Gaps: YES, Weight Matrix: BLOSUM (for PROTEIN). Substitutions within the following group (Clustal W, 'strong' conservation group) are to be regarded as conservative substitutions:
- S,T,A; N,E,Q,K; N,H,Q,K; N,D,E,Q; Q,H,R,K; M,I,L,V; M,I,L,F; H,Y; F,Y,W.

Substitutions within the following group (Clustal W, 'weak' conservation group) are to be regarded as semi-conservative substitutions: -C,S,A; A,T,V; S,A,G; S,T,N,K;
S,T,P,A; S,G,N,D; S,N,D,E,Q,K; N,D,E,Q,H,K; N,E,Q,H,R,K; V,L,I,M; H,F,Y.

Examples of conservative substitutions are substitutions made within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter specific activity are known in the art and are described, for example, by Neurath and Hill (1979). The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/lle, Leu/Val, Ala/Glu, and Asp/Gly.

In addition to the 20 standard amino acids, non-standard amino acids (such as 4-hydroxyproline, 6-N-methyl lysine, 2-aminoisobutyric acid, isovaline, and alpha-methyl serine) may be substituted for amino acid residues of a wild-type polypeptide. A limited number of non-conservative amino acids, amino acids that are not encoded by the genetic code, and unnatural amino acids may be substituted for amino acid residues. "Unnatural amino acids" have been modified after protein synthesis, and/or have a chemical structure in their side chain(s) different from that of the standard amino acids. Unnatural amino acids can be chemically synthesized, and preferably, are commercially available, and include pipecolic acid, thiazolidine carboxylic acid, dehydroproline, 3- and 4-methylproline, and 3,3-dimethylproline.

Essential amino acids in a mammalian alfa and/or beta defensin can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for biological activity *(i.e.,* activity against an airway hyper responsiveness or suppression of cytokines e.g. TNF-alpha activity) to identify amino acid residues that are critical to the activity of the molecule. See also, Hilton et al., 1996, J. Biol. Chem. 271: 4699-4708. The identities of essential amino acids can also be inferred from analysis of identities with polypeptides which are related to mammalian alfa and/or beta defensins (see Clustal W alignment in figure 4).

Single or multiple amino acid substitutions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochem. 30:10832-10837; U.S. Patent No. 5,223,409; WO 92/06204), and region-directed mutagenesis (Derbyshire et al., 1986, Gene 46:145; Ner et al., 1988, DNA 7:127). When the result of a given substitution cannot be predicted with certainty, the derivatives may be readily assayed according to the methods described herein to determine the presence or absence of biological activity.

In one embodiment, the treatment of NEC comprises administration of an effective amount of the hBD-2 (SEQ ID NO: 2), truncated hBD-2 (SEQ ID NO: 16), and functionally equivalent variants having 1-2 amino acid modification(s) to a preterm infant or to a woman about to give birth to a preterm infant (preterm labor). A preferred embodiment provides administration of hBD-2 for use in treatment of NEC in a preterm infant.

The half-life of the peptide for the use according to the claims may be extended by fusing or conjugating the peptide with another moiety i.e. constructing a long acting biologically active peptide linked to a pharmaceutically acceptable molecule providing an *in vivo* plasma half-life of the peptide, which is increased substantially compared to the *in vivo* plasma half-life of the non-conjugated peptide administered in the same manner.

In one embodiment, the hBD-2 (SEQ ID NO: 2), truncated hBD-2 (SEQ ID NO: 16), and functionally equivalent variants having 1-2 amino acid modification(s) for use according to the claims, further comprises at least one additional moiety selected from the group consisting of a cell penetrating peptide (CPP), an Albumin Binding Moiety (ABM), a detectable moiety (Z), and a half-life extending peptide.

A long acting biologically active hBD-2 (SEQ ID NO: 2), truncated hBD-2 (SEQ ID NO: 16), and functionally equivalent variants having 1-2 amino acid modification(s) for use according to the claims linked to a pharmaceutically acceptable molecule selected from the group consisting of a molecule having binding to a neonatal Fc receptor (FcRn), transferrin, albumin (HAS), XTEN^{®} or PEG, a homo-amino acid polymer (HAP), a proline-alanine-serine polymer (PAS), or an elastin-like peptide (ELP), hyaluronic acid, a negatively charged highly siasylated peptide such as the carboxy-terminal peptide (CTP) of chorionic gonadotropin (CG) β-chain, human IgG, and CH₃(CH2)ₙCO- wherein n is 8 to 22.

The peptide for the use according to the claims may be linked to the pharmaceutically acceptable molecule in various ways as described in the prior art literature, such as without limitation chemical coupling through a bifunctional linker, gene technologically by coupling the N-terminal or C-terminal of the peptide to the pharmaceutically acceptable molecule, such as albumin or an albumin analog. In particular, the N-terminal of albumin or an albumin analogue, e.g. human albumin, can be coupled to the C-terminal of the peptide, or the N-terminal of the peptide; or the C-terminal of albumin, e.g. human albumin, can be coupled to the C-terminal of the peptide, or the N-terminal of the peptide. A linker sequence can be inserted between the albumin and the peptide chain.

The peptide for the use according to the claims may be linked to the pharmaceutically acceptable molecule through a stable linker or a more labile linker. Several linkers are known in the art, including bifunctional PEG molecules (e.g. see Paige et.al Pharmaceutical Research, vol. 12, no. 12, 1995), hydrolysable linkers (Shechter et al. Bioconjugate Chem. 2005,16: 913- 920 and International Journal of Peptide Research and Therapeutics, Vol. 13, Nos. 1-2, June 2007 and WO2009095479), PDPH and EMCH see e.g. in WO2010092135. In the special case where chemical conjugation (linking of two or more molecules) of the peptide, to the pharmaceutically acceptable molecule, strongly reduce the functional peptide activity, it may be preferable to use a more labile linker that can release the functional peptide.

Half-life extension may also be accomplished through acylation of the backbone of the peptide for the use according to the claims with a spacer e.g. γ-L-glutamyl spacer and a C-18 fatty di-acid chain to Lysine. The fatty di-acid site chain and the spacer mediate a strong but reversible binding to albumin, slowing release from the injection site and reducing renal clearance.

### Methods and Uses

Human beta defensin 2, and human defensin 5 plus human beta defensin 2, are found to be able to increase the microbial presence and abundance in the intestines; thus showing potent activity as a medicament for preventing or treating necrotizing enterocolitis.

Human beta defensin 2 is found to be able to decrease the incidence of necrotizing enterocolitis and prevent weight loss through a normalized gut function - increase mucosal proportion of the intestinal wall, increase the height of the intestinal villi and increase the density of goblet cells, increase the digestive brush border enzyme activity, lower the intestinal myeloperoxidase activity and increased IL-22 production; thus showing potent activity as a medicament for prevention or treatment of necrotizing enterocolitis, short bowel syndrome, sepsis, neurodevelopmental impairment and extrauterine growth restriction.

Human beta defensin 2 has been shown in a therapeutic animal model of severe intestinal inflammation and dysbiosis (CD4+CD25+ T-cell transfer), that β-defensins whether administered orally or subcutaneously mitigate weight loss, improve intestinal health and importantly decreases the disease activity index on par with strong immunosuppressants such as prednisolone/dexamethasone and cyclosporine as well as anti TNF-α, all routinely used in treatment of colitis such as Crohn's Disease and Ulcerative Coloitis in adults thus showing potent activity as a prophylactic or therapeutic treatment of preterm infants with necrotizing enterocolitis.

It has been demonstrated that β-defensins can normalize gut health and function through a decrease of myeloperoxidase activity, thus improving gut health in preterm infants.

Human beta defensin 2 is found to be able to prevent an increase of airway hyper responsiveness; to prevent a decrease of pulmonary compliance; to prevent lung inflammation; to prevent neutrophil -, eosinophil - and macrophage migration into BALF as well as normalize TNF-α, IL-4, IL-5, IL-6, IL-9 and IL-13 concentrations in lung tissue homogenate.

Particularly, human beta defensin 2 and human defensin 5 have been demonstrated in a murine model of graft versus host disease to dramatically decreased mortality and weight loss on par or better than the standard treatment of graft versus host disease with cyclosporine. It has further been demonstrated that the defensins decreased the histology score of the small and large intestine as well as the liver all suggesting improved gut health and gut integrity. Treatment with defensins reduced the infiltration with CD45+ leucocytes as well as intestinal T cell and myeloid cell infiltration in gut epithelium/lamina propria. Prophylactic treatment with defensins also showed reduction of TNF-α and IL-6 and induction of IL-10. The defensins prophylactic treatment additionally showed a reduction of IL-1β from myeloid cells (FACS analysis of the spleen) and reduced Th1 cytokine especially TNF-α and IFN-γ production.

Human beta defensin 2 was found to be able to improve lung function through prevention of an increase of airway hyper responsiveness; to prevent a decrease of pulmonary compliance; to prevent lung inflammation; to prevent neutrophil -, eosinophil - and macrophage migration into BALF as well as normalize TNF-α, IL-4, IL-5, IL-6, IL-9 and IL-13 concentrations in lung tissue homogenate.

Human beta defensin 2 is found to be able to reduce airway hyper responsiveness; increase pulmonary compliance; reduce lung inflammation; reduce BALF neutrophil -, eosinophil - and macrophage count as well as normalize IFN-γ, TNF-α, IL-4, IL-5, IL-6, IL-9, IL-10 and IL-13 concentrations in lung homogenate.

Human beta defensin 2 is found to be able to rebalance the immune system normalizing tissue cytokine production of IFN-γ, TNF-α, IL-4, IL-5, IL-6, IL-9, IL-10 and IL-13.

Surprisingly, it has been found that both parenteral but also oral administration of defensins is effective to prevent and treat inflammatory conditions of the lung. This is unexpected, as it is known that hBD-2 is not absorbed from the gut. An advantage of this observation is that severely ill preterm infants on assisted ventilation can be treated by oral administration of the peptide for the use as described in the claims.

Human beta defensin 2 has been found to mature and normalize the intestinal microbiota, prevent or treat inflammation of the gut and/or lung, sepsis, respiratory illness, neurodevelopmental impairment and extra uterine growth restriction in the preterm infant.

In one embodiment, the administration is oral, buccal, sublingual, rectal, vaginal, intratracheal, intrapulmonary, intranasal, intracranial, subcutaneous, intravenous, dermal or transdermal. Preferably the administration is oral. Oral and parenteral administration is advantageous for preterm infants with compromised breathing or preterm infants undergoing medical ventilation.

The provided use according to the claims can mature or normalize gut microbiota in a preterm infant by increasing the presence and abundance of key commensal bacteria.

The provided use according to the claims can improve intestinal health, normalize gut function and increase food uptake in a preterm infant through increasing the mucosal proportion of the gut wall, increasing the height of the intestinal villi and increasing the density of intestinal goblet cells, increasing the digestive brush border enzyme activity and lower intestinal myeloperoxidase activity.

The use according to the claims may also normalize cytokine production of e.g. IFN-γ, TNF-α, IL-1β, IL-4, IL-5, IL-6, IL-8, IL-9, IL-10, IL-13 and IL-33 in lung tissue homogenate from a preterm infant affected by NEC and thus prevent a cytokine storm despite inflammatory cell migration into BALF.

The provided use according to the claims can treat or prevent lung and/or gut inflammation by changing bacterial phenotypes through a change at the transcriptional level as well as structure and composition of the lung and/or gut bacterial flora or the lung and/or gut metabolome of the preterm infant affected by NEC as described herein.

Without being bound by theory the effects observed using oral administration may be ascribed to a change in the gut microflora and gut metabolome that may have an effect on the lungs through the so-called gut-lung axis. Chronic lung disorders such as asthma, COPD and cystic fibrosis all exhibit a component of intestinal disease manifestation indicating that there is a vital cross talk between these two mucosal sites of the human body and a variety of respiratory diseases have been associated with a dysbiosis not only of the airway microbiota but also the intestinal microbiota (Marsland et al, 2015). Caesarian birth reduces the diversity and alters the composition of the intestinal microbiota early in life and is at the same time linked to a predisposition towards asthma during childhood (Jakobsson et al, 2014).

Commensal microbes calibrate innate and adaptive immune responses and impact activation thresholds for pathogenic stimulations, in large part by producing small molecules that mediate host-microbial interactions (Donia and Fishback, 2015). While the epithelial barrier ensures that microorganisms are largely confined to the gut, microbial metabolites can penetrate the epithelial barrier, allowing them to enter and accumulate in the host circulatory system where they are sensed by immune cells (Dorrestein, 2014). Trompette, 2013 demonstrated in mice that fermentable fibers in the diet changed the composition not only of the gut but also the lung microbiota in particular the ratio of *Firmicutes* to *Bacteriodetes,* the latter leading to increased local and systemic levels of Short Chain Fatty Acids, which in turn influenced Dendritic Cell hematopoiesis and functionality thus shaping the immunological environment in the lung and influencing the severity of allergic inflammation. Schirmer et al (2016) further demonstrated in the Human Functional Genomics Project that inter-individual variation in cytokine response is linked to specific microbial organisms as well as microbial functions. The majority of detected associations were both cytokine and stimulus specific, suggesting that the immune system recognizes and interacts with microbial organisms and products with high specificity and that these microbial factors are associated with a particular immunological phenotype. TNF-α and IFN-γ production capacity appeared to be more strongly influenced by the microbiome, whereas other cytokines such as IL-1β, IL-6 and Th17 derived IL-17 and IL-22 exhibited fewer, but more specific, associations with the gut microbiota.

The use according to the claims can be performed by administration the hBD-2 (SEQ ID NO: 2), truncated hBD-2 (SEQ ID NO: 16), and functionally equivalent variants having 1-2 amino acid modification(s) in combination with either a mammalian lactoferrin/lactoferricin, a mammalian lysozyme, a mammalian cathelicidin, surfactant, prebiotics, probiotics, glucocorticoids, antibiotics, immunosuppressants, GLP-2 or GLP-2 analogs or a combination of these. The hBD-2 (SEQ ID NO: 2), truncated hBD-2 (SEQ ID NO: 16), and functionally equivalent variants having 1-2 amino acid modification(s) for use according to the claims can be administered separately or together with one or more of these therapies. The hBD-2 (SEQ ID NO: 2), truncated hBD-2 (SEQ ID NO: 16), and functionally equivalent variants having 1-2 amino acid modification(s) for use according to the claims can also be administered together with other medicaments which can be used in the preterm infant or administered to a mother in preterm labor.

Normalizing the gut microbiota may also involve changing the metabolome to one that produces relatively more butyrate and relatively less acetate.

### In vitro synthesis

The peptides for the use according to the claims may be prepared by *in vitro* synthesis, using conventional methods as known in the art. Various commercial synthetic apparatuses are available, for example automated synthesizers by Applied Biosystems Inc., Beckman, etc. By using synthesizers, naturally occurring amino acids may be substituted with unnatural amino acids, particularly D-isomers (or D-forms) e.g. D-alanine and D-isoleucine, diastereoisomers, side chains having different lengths or functionalities, and the like. The particular sequence and the manner of preparation will be determined by convenience, economics, purity required, and the like. Chemical linking may be provided to various peptides or proteins comprising convenient functionalities for bonding, such as amino groups for amide or substituted amine formation, e.g. reductive amination, thiol groups for thioether or disulphide formation, carboxyl groups for amide formation, and the like. If desired, various groups may be introduced into the peptide during synthesis or during expression, which allow for linking to other molecules or to a surface. Thus cysteines can be used to make thioethers, histidines for linking to a metal ion complex, carboxyl groups for forming amides or esters, amino groups for forming amides, and the like.

The peptides for the use according to the claims, may also be isolated and purified in accordance with conventional methods of recombinant synthesis. Recombinant synthesis may be performed using appropriate expression vectors and a eukaryotic or prokaryotic expression system. A solution may be prepared of the expression host and the media and the defensins present purified using HPLC, exclusion chromatography, gel electrophoresis, affinity chromatography, or other purification technique. Methods for recombinant expression of human beta defensin-2 in E. coli are disclosed in WO 2010/007166 (Novozymes).

The peptides for the use according to the claims may also be induced by administration of the corresponding mRNA to a preterm infant or to a woman about to give birth to a preterm infant.

### Dosages

The hBD-2 (SEQ ID NO: 2), truncated hBD-2 (SEQ ID NO: 16), and functionally equivalent variants having 1-2 amino acid modification(s) for use according to the claims may be employed in pharmaceutical compositions in an amount which is effective to prevent or treat necrotizing enterocolitis in the preterm infant, preferably with acceptable toxicity to the patient. The hBD-2 (SEQ ID NO: 2), truncated hBD-2 (SEQ ID NO: 16), and functionally equivalent variants having 1-2 amino acid modification(s) for use according to the claims may be employed in pharmaceutical compositions in an amount which is effective to maintain a normal microbiota composition in the lung and/or the intestine or to normalize a dysbiotic microbiota in the lung and/or the intestine, preferably with acceptable toxicity to the patient in need of the treatment.

For such treatments, the appropriate dosage will, of course, vary depending upon, for example, the chemical nature and the pharmacokinetic data of a compound used, the individual host, the mode of administration and the nature and severity of the conditions being treated.

However, in general, for satisfactory results in mammals, for example humans, an indicated daily dosage of a human alfa defensin is preferably from about 0.1 mg HD5 /kg body weight to about 10 mg HD5 /kg body weight, more preferably from about 0.5 mg HD5 /kg body weight to about 10 mg HD5 /kg body weight; such as 1 mg HD5 /kg body weight to 10 mg HD5 /kg body weight, more preferably from about 1.2 mg HD5 /kg body weight to about 10 mg HD5 /kg body weight, preferably from about 1.2 mg HD5 /kg body weight to about 5 mg HD5 /kg body weight, even more preferably 1.2 mg HD5 /kg body weight, for example, administered in divided doses up to one, two or three times a day.

In one embodiment an indicated daily dosage of a human beta defensin is preferably from about 0.1 mg hBD-2 /kg body weight to about 10 mg hBD-2 /kg body weight, more preferably from about 0.5 mg hBD-2 /kg body weight to about 10 mg hBD-2 /kg body weight; such as 1 mg hBD-2 /kg body weight to 10 mg hBD-2 /kg body weight, more preferably from about 1.2 mg hBD-2 /kg body weight to about 10 mg hBD-2 /kg body weight, preferably from about 1.2 mg hBD-2 /kg body weight to about 5 mg hBD-2 /kg body weight, even more preferably 1.2 mg hBD-2 /kg body weight, for example, administered in divided doses up to one, two or three times a day.

When two different defensins are administered in one dosage, the dosage may comprise equal or approximately equal amounts of the two defensins determined on a weight basis or on a molar basis. The ratio may also differ so that the ratio of alpha defensin to beta-defensin varies from 10:1 to 1:10, such as 5:1 to 1:5, for example 2:1 to 1:2 determined on a weight or molar basis.

The hBD-2 (SEQ ID NO: 2), truncated hBD-2 (SEQ ID NO: 16), and functionally equivalent variants having 1-2 amino acid modification(s) for use according to the claims can be administered to mammals, for example humans, by similar modes of administration at similar dosages than conventionally used.

In one embodiment, the peptide for the use according to the claims is provided wherein the daily dosage is between 0.1 and 10 mg defensin/kg, such as between 0.5 and 5 mg defensin/kg, such as between 1 and 2 mg defensin/kg, such as 1.2 mg defensin/kg per day.

In certain embodiments, the hBD-2 (SEQ ID NO: 2), truncated hBD-2 (SEQ ID NO: 16), and functionally equivalent variants having 1-2 amino acid modification(s) for use according to the claims may be in a pharmaceutical composition of preferred embodiments in an amount of about 0.01 mg or less to about 1500 mg or more per unit dosage form, preferably from about 0.01, 0.02, 0.03, 0.04, or 0.05 mg to about 150, 200, 250, 300, 350, 400, 450, 500, 600, 700, 800, 900, or 1000 mg, and more preferably from about 1, 2, 3, 4, 5, 6, 7, 8, 9, 10, 15, 20, or 25 mg to about 30, 35, 40, 45, 50, 55, 60, 65, 70, 75, 80, 85, 90, 95, or 100 mg. In certain embodiments, however, lower or higher dosages than those mentioned above may be preferred. Appropriate concentrations and dosages can be readily determined by one skilled in the art.

In further embodiments, the peptide for the use according to the claims may be in pharmaceutical compositions of preferred embodiments including a mammalian alfa defensin, such as a human alfa defensin, wherein the alfa defensin and the peptide for the use according to the claims are present in equal amounts on a molarity basis or on a mg/mL basis.

In one embodiment, the hBD-2 (SEQ ID NO: 2), truncated hBD-2 (SEQ ID NO: 16), and functionally equivalent variants having 1-2 amino acid modification(s) is administered at least once daily, such as at least twice daily, for example at least 3 times daily.

### Formulations for oral or parenteral administration

The peptides for the use according to the claims can be employed therapeutically in compositions formulated for administration by any conventional route.

In one embodiment, the administration of the peptide for the use according to the claims is oral, buccal, sublingual, rectal, vaginal, intratracheal, intrapulmonary, intranasal, intracranial, subcutaneous, intravenous, dermal or transdermal. Preferably the administration is oral.

In one embodiment, the administration is generally intranasal or intrapulmonary. Intranasal and intrapulmonary administration is normal for pulmonary drug delivery. In one embodiment, the administration is oral.

In one embodiment, the administration is subcutaneous or intravenous.

Within some embodiments, the hBD-2 (SEQ ID NO: 2), truncated hBD-2 (SEQ ID NO: 16), and functionally equivalent variants having 1-2 amino acid modification(s) for use according to the claims may be formulated as a lyophilizate, utilizing appropriate excipients that provide stability as a lyophilizate, and subsequently after rehydration. The peptides for the use according to the claims can be in a pharmaceutical composition manufactured according to conventional methods, e.g., by mixing, granulating, coating, dissolving or lyophilizing processes. In a preferred embodiment, hBD-2 (SEQ ID NO: 2), truncated hBD-2 (SEQ ID NO: 16), and functionally equivalent variants having 1-2 amino acid modification(s) for use according to the claims may be formulated as a sterile and isotonic solution.

Pharmaceutically acceptable carriers and/or diluents are familiar to those skilled in the art. For compositions formulated as liquid solutions, acceptable carriers and/or diluents include saline and sterile water should be included, and the composition may optionally include antioxidants, buffers, bacteriostats, and other common additives.

The peptides for the use according to the claims may be formulated in a wide variety of formulations for oral administration. Solid form preparations may include powders, tablets, drops, capsules, cachets, lozenges, and dispersible granules. Other forms suitable for oral administration may include liquid form preparations including emulsions, syrups, elixirs, aqueous solutions, aqueous suspensions, toothpaste, gel dentifrice, chewing gum, or solid form preparations which are intended to be converted shortly before use to liquid form preparations, such as solutions, suspensions, and emulsions.

The peptides for the use according to the claims may be formulated in a wide variety of formulations for buccal, sublingual, oral, rectal, vaginal, dermal, transdermal, intracranial, subcutaneous or intravenous administration. The formulation can contain (in addition to a mammalian alfa defensin and/or a mammalian beta defensin and/or a mammalian cathelicidin and/or a mammalian lactoferrin/lactoferricin and/or a mammalian lysozyme, and other optional active ingredients) carriers, fillers, disintegrators, flow conditioners, sugars and sweeteners, fragrances, preservatives, stabilizers, wetting agents, emulsifiers, solubilizers, salts for regulating osmotic pressure, buffers, diluents, dispersing and surface-active agents, binders, lubricants, and/or other pharmaceutical excipients as are known in the art. One skilled in this art may further formulate mammalian alfa defensins, mammalian beta defensins, mammalian cathelicidins, mammalian lactoferrins/lactoferricins and mammalian lysozymes in an appropriate manner, and in accordance with accepted practices, such as those described in Remington's Pharmaceutical Sciences, Gennaro (1990).

The hBD-2 (SEQ ID NO: 2), truncated hBD-2 (SEQ ID NO: 16), and functionally equivalent variants having 1-2 amino acid modification(s) for use according to the claims can be used alone, or in combination therapies with one, two, or more other pharmaceutical compounds or drug substances, for example with surfactant, prebiotics, probiotics, glucocorticoids, antibiotics, immunosuppressants, GLP-2 or GLP-2 analogs or a combination of these and/or with one or more pharmaceutically acceptable excipient(s).

### Airway Administration

Airway administration may be used for administering the hBD-2 (SEQ ID NO: 2), truncated hBD-2 (SEQ ID NO: 16), and functionally equivalent variants having 1-2 amino acid modification(s) for use according to the claims. The term "intrapulmonary administration" as used herein refers to a topical administration to the lungs. When used herein the terms "intratracheal, intrabronchial or intra alveolar administration" include all forms of such administration whereby the peptides for the use according to the claims is applied into the trachea, the bronchi or the alveoli, respectively, whether by instillation of a solution, by applying in a powder form, or by allowing them to reach the relevant part of the airway by inhalation as an aerosolized or nebulized solution or suspension or inhaled powder or gel, with or without added stabilizers or other excipients.

Methods of intrabronchial/alveolar administration include, but are not limited to, bronchoalveolar lavage (BAL) according to methods well known to those skilled in the art, using as a lavage fluid a physiologically acceptable composition in which the peptides for the use according to the claims has been dissolved or indeed by any other effective form of intrabronchial administration including the use of inhaled powders containing the peptides for the use according to the claims , with or without excipients, or the direct application of the peptides for the use according to the claims in solution or suspension or powder form during bronchoscopy. Methods for intratracheal administration include, but are not limited to, blind tracheal washing with a similar solution of dissolved hBD-2 (SEQ ID NO: 2), truncated hBD-2 (SEQ ID NO: 16), or functionally equivalent variants having 1-2 amino acid modification(s), or hBD-2 (SEQ ID NO: 2), truncated hBD-2 (SEQ ID NO: 16), or functionally equivalent variants having 1-2 amino acid modification(s) suspension, or the inhalation of nebulized fluid droplets containing dissolved hBD-2 (SEQ ID NO: 2), truncated hBD-2 (SEQ ID NO: 16), and functionally equivalent variants having 1-2 amino acid modification(s), or hBD-2 (SEQ ID NO: 2), truncated hBD-2 (SEQ ID NO: 16), or functionally equivalent variants having 1-2 amino acid modification(s) suspension obtained by use of any nebulizing apparatus adequate for this purpose.

In another embodiment, intratracheal, intrabronchial or intra alveolar administration does not include inhalation of the product but the instillation or application of a solution of the peptide for the use according to the claims or a powder or a gel containing the peptide for the use according to the claims into the trachea or lower airways.

Other preferred methods of administration may include using the following devices:
1. Pressurized nebulizers using compressed air/oxygen mixture
2. Ultrasonic nebulizers
3. Electronic micropump nebulizers
4. Metered dose inhaler (MDI)
5. Dry powder inhaler systems (DPI),

The aerosol may be delivered via a) facemasks or b) via endotracheal tubes in intubated patients during mechanical ventilation (device 1, 2 and 3). The devices 4 and 5 can also be used by the patient without assistance provided that the patient is able to self-activate the aerosol device.

Preferred concentrations for a solution comprising the hBD-2 (SEQ ID NO: 2), truncated hBD-2 (SEQ ID NO: 16), and functionally equivalent variants having 1-2 amino acid modification(s) for use according to the claims are in the range of from about 0.1 µg to 1000 µg per ml solution, such as in the range of from about 0.1 µg to 250 µg per ml solution.

### Intrapulmonary administration

The hBD-2 (SEQ ID NO: 2), truncated hBD-2 (SEQ ID NO: 16), and functionally equivalent variants having 1-2 amino acid modification(s) for use according to the claims in combination with, preferably dissolved in, a pharmaceutically acceptable carrier, preferably an aqueous carrier or diluent, or carried to the lower airways as a pegylated preparation or as a liposomal or nanoparticle preparation administered as an aerosol via inhalation, or as a lavage fluid administered via a bronchoscope as a bronchoalveloar lavage or as a blind intratracheal wash or lavage. A variety of aqueous carriers may be used, including, but not limited to 0.9% saline, buffered saline, physiologically compatible buffers and the like. The peptides for the use according to the claims may be sterilized by conventional techniques well known to those skilled in the art. The resulting aqueous solutions may be packaged for use or filtered under aseptic conditions and freeze-dried, the freeze-dried preparation being dissolved in a sterile aqueous solution prior to administration

In one embodiment a freeze-dried hBD-2 (SEQ ID NO: 2), truncated hBD-2 (SEQ ID NO: 16), and functionally equivalent variants having 1-2 amino acid modification(s) preparation for use according to the claims may be pre-packaged for example in single dose units. In an even more preferred embodiment the single dose unit is adjusted to the patient.

The compositions may contain pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

The formulations may contain pharmaceutically acceptable carriers and excipients including microspheres, liposomes, microcapsules, nanoparticles or the like. Conventional liposomes are typically composed of phospholipids (neutral or negatively charged) and/or cholesterol. The liposomes are vesicular structures based on lipid bilayers surrounding aqueous compartments. They can vary in their physiochemical properties such as size, lipid composition, surface charge and number and fluidity of the phospholipids bilayers. The most frequently used lipid for liposome formation are: 1,2-Dilauroyl-sn-Glycero-3-Phosphocholine (DLPC), 1,2-Dimyristoyl-sn-Glycero-3-Phosphocholine (DMPC), 1,2-Dipalmitoyl-sn-Glycero-3-Phosphocholine (DPPC), 1,2-Distearoyl-sn-Glycero-3-Phosphocholine (DSPC), 1,2-Dioleoyl-sn-Glycero-3-Phosphocholine (DOPC), 1,2-Dimyristoyl-sn-Glycero-3-Phosphoethanolamine (DMPE), 1,2-Dipalmitoyl-sn-Glycero-3-Phosphoethanolamine (DPPE), 1,2-Dioleoyl-sn-Glycero-3-Phosphoethanolamine (DOPE), 1,2-Dimyristoyl-sn-Glycero-3-Phosphate (Monosodium Salt) (DMPA), 1,2-Dipalmitoyl-sn-Glycero-3-Phosphate (Monosodium Salt) (DPPA), 1,2-Dioleoyl-sn-Glycero-3-Phosphate (Monosodium Salt) (DOPA), 1,2-Dimyristoyl-sn-Glycero-3-[Phospho-rac-(1-glycerol)] (Sodium Salt) (DMPG), 1,2-Dipalmitoyl-sn-Glycero-3-[Phospho-rac-(1-glycerol)] (Sodium Salt) (DPPG), 1,2-Dioleoyl-sn-Glycero-3-[Phospho-rac-(1-glycerol)] (Sodium Salt) (DOPG), 1,2-Dimyristoyl-sn-Glycero-3-[Phospho-L-Serine] (Sodium Salt) (DMPS), 1,2-Dipalmitoyl-sn-Glycero-3-[Phospho-L-Serine) (Sodium Salt) (DPPS), 1,2-Dioleoyl-sn-Glycero-3-[Phospho-L-Serine] (Sodium Salt) (DOPS), 1,2-Dioleoyl-sn-Glycero-3-Phosphoethanolamine-N-(glutaryl) (Sodium Salt) and 1,1',2,2'-Tetramyristoyl Cardiolipin (Ammonium Salt). Formulations composed of DPPC in combination with other lipids or modifiers of liposomes are preferred e.g. in combination with cholesterol and/or phosphatidylcholine.

Long-circulating liposomes are characterized by their ability to extravasate at body sites where the permeability of the vascular wall is increased. The most popular way of producing long-circulating liposomes is to attach hydrophilic polymer polyethylene glycol (PEG) covalently to the outer surface of the liposome. Some of the preferred lipids are: 1,2-Dipalmitoyl-sn-Glycero-3-Phosphoethanolamine-N-[Methoxy(Polyethylene glycol)-2000] (Ammonium Salt), 1,2-Dipalmitoyl-sn-Glycero-3-Phosphoethanolamine-N-[Methoxy(Polyethylene glycol)-5000] (Ammonium Salt), 1,2-Dioleoyl-3-Trimethylammonium-Propane (Chloride Salt) (DOTAP).

Possible lipids applicable for liposomes are supplied by Avanti, Polar Lipids, Inc, Alabaster, AL. Additionally, the liposome suspension may include lipid-protective agents which protect lipids against free-radical and lipid-peroxidative damage on storage. Lipophilic free-radical quenchers, such as alpha-tocopherol and water-soluble iron-specific chelators, such as ferrioxianine, are preferred.

A variety of methods are available for preparing liposomes, as described in, e.g., Szoka et al., Ann. Rev. Biophys. Bioeng. 9:467 (1980), U.S. Pat. Nos. 4, 235,871, 4,501,728 and 4,837,028. Another method produces multilamellar vesicles of heterogeneous sizes. In this method, the vesicle-forming lipids are dissolved in a suitable organic solvent or solvent system and dried under vacuum or an inert gas to form a thin lipid film. If desired, the film may be redissolved in a suitable solvent, such as tertiary butanol, and then lyophilized to form a more homogeneous lipid mixture which is in a more easily hydrated powder-like form. This film is covered with an aqueous solution of the targeted drug and the targeting component and allowed to hydrate, typically over a 15-60 minute period with agitation. The size distribution of the resulting multilamellar vesicles can be shifted toward smaller sizes by hydrating the lipids under more vigorous agitation conditions or by adding solubilizing detergents such as deoxycholate.

Micelles are formed by surfactants (molecules that contain a hydrophobic portion and one or more ionic or otherwise strongly hydrophilic groups) in aqueous solution.

Common surfactants well known to one of skill in the art can be used in the micelles of the present invention. Suitable surfactants include sodium laureate, sodium oleate, sodium lauryl sulfate, octaoxyethylene glycol monododecyl ether, octoxynol 9 and PLURONIC F-127 (Wyandotte Chemicals Corp.). Preferred surfactants are nonionic polyoxyethylene and polyoxypropylene detergents compatible with IV injection such as, TWEEN-80, PLURONIC F-68, n-octyl-beta-D-glucopyranoside, and the like. In addition, phospholipids, such as those described for use in the production of liposomes, may also be used for micelle formation.

It is an aspect of the present disclosure to provide hBD-2 (SEQ ID NO: 2), truncated hBD-2 (SEQ ID NO: 16), and functionally equivalent variants having 1-2 amino acid modification(s) for use in a method of preventing or treating necrotizing enterocolitis in a preterm infant, the method comprising administering the hBD-2 (SEQ ID NO: 2), truncated hBD-2 (SEQ ID NO: 16), and functionally equivalent variants having 1-2 amino acid modification(s) for use according to the claims to a preterm infant or to a woman about to give birth to a preterm infant.

In one aspect, the present disclosure provides a method for preventing or treating necrotizing enterocolitis in a preterm infant, the method comprising improving intestinal health by administering a the hBD-2 (SEQ ID NO: 2), truncated hBD-2 (SEQ ID NO: 16), and functionally equivalent variants having 1-2 amino acid modification(s) to a preterm infant.

### Examples

### Example 1. Modulation of gut microbiota by prophylactic treatment with defensins (Reference example).

### Materials and methods:

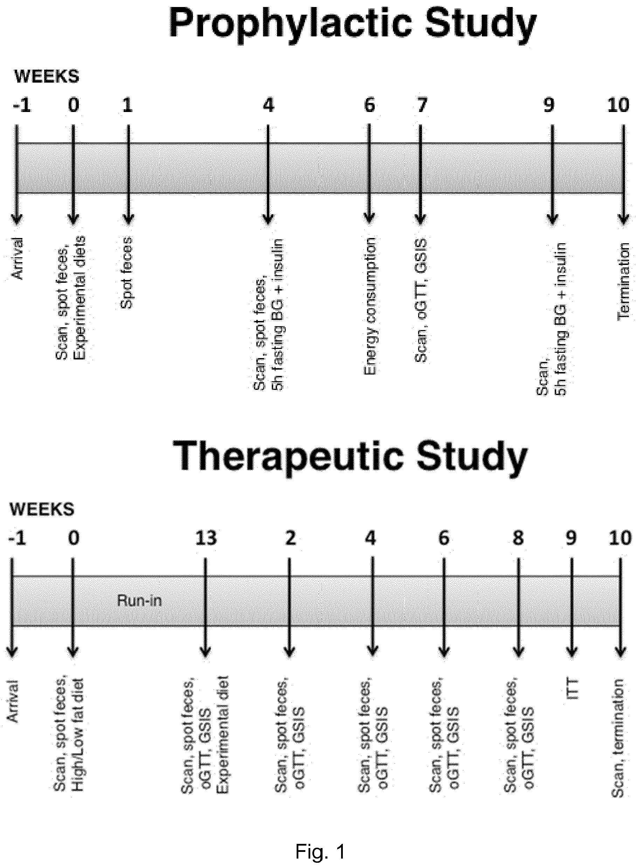

The overall experimental design is shown in Figure 1, top panel "Prophylactic study".

Mice: Mice were housed in trios, 4 cages per group. Feed intake was registered daily just before lights were turned off at 6 pm. Individual mice were subjected to experimental procedures in altered order both group and cage wise. Mice were kept at room temperature under a 12-hour light/dark cycle at SPF standard conditions.

Diets: For dosing, the average weight was estimated to be 25 grams per mouse. Mice eat approximately 3 grams of feed per mouse per day.

Treatment regime: Mice were fed either a high fat diet (HFD) or a low fat (LF) control diet. The HFD contained 4 subgroups; a) hBD-2, b) HD5, c) hBD-2/HD5 and d) standard HFD without supplementation of defensins. Defensin concentration was 1,2 mg hBD-2 per kg mouse per day. HD5 was given in equimolar concentration to hBD-2. The combinatory group was given 50% hBD-2 + 50% HD5, hence a total amount of defensins equivalent to the remaining test groups.

### Tests:

Microbial analyses were carried out to study the microbiota of the intestine.

Longitudinal 16S characterization was conducted on 4 paired samples from 60 mice, 240 samples in total. Each mouse was sampled prior to diet change, 1 week post diet change, 4 weeks post diet change and at termination, thus ensuring a thorough characterization of the faecal microbiota as a result of defensin treatment.

### Results:

Weight change. While the food intake was similar in all three experimental diet groups, both High Fat Diet (HFD) groups ( a) treated with hBD-2 and d) standard HFD without treatment) gained significantly more weight than the Low Fat Diet (LFD) reference group over the 10 week study period (*p<0.0001, 2-way ANOVA, Tukey Post Test).

The HFD plus hBD-2 group, however, gained significantly less weight than the HFD reference group (*p= 0.0028).

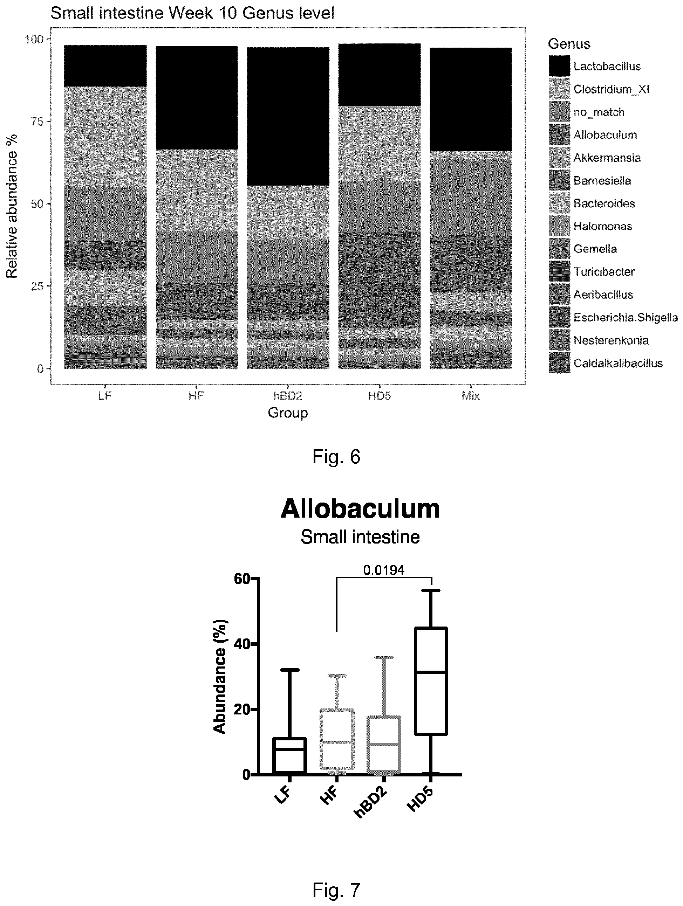

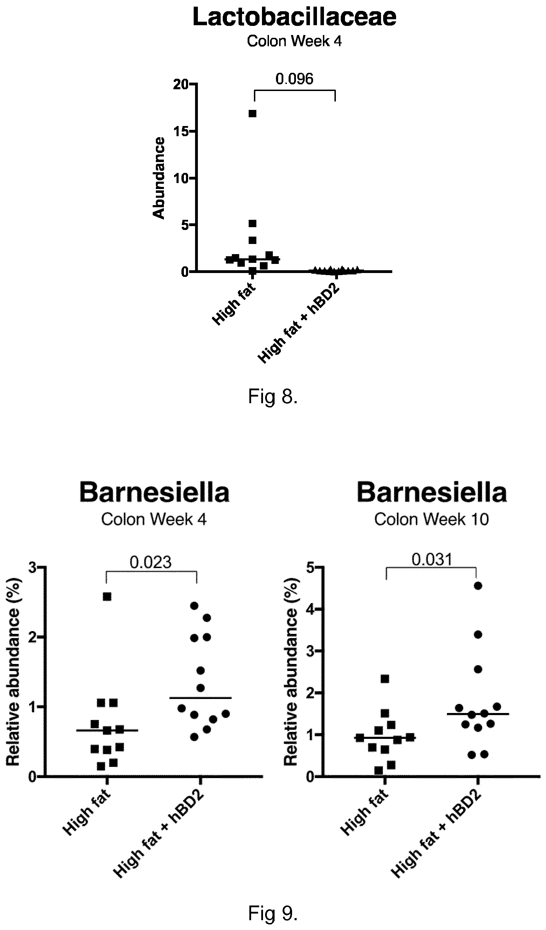

Microbiota. hBD-2 affected primarily the microbial presence, whereas HD5 and hBD-2+HD5 affected primarily the microbial abundance (Figure 5 and 6). Presence describes the number of different bacteria present. Increased presence signifies a more diverse microbiota and has in many publications over the past decade been associated with a healthier microbiota and in some cases with specific disease improvement in animal models. Abundance describes the number of bacteria of a given strain present. Ideally, one would like to increase the abundance of "good" bacteria such as Barnesiella, prevotella species and Bifidobacteriaceae, which have been identified as important species, while decreasing the abundance of e.g. pathogens. A statistically significant increase of abundance of Allobaculum was seen in the small intestine following prophylaxis with HD5 (p<0.02; Figure 7). Allobaculum is a short chain fatty acid producing species. A trend towards lower abundance of Lactobacillaceae was observed in colon following prophylactic treatment with hBD-2 (p=0.1; Figure 8). A statistically significant increase in abundance of Barnesiella in the colon was observed following prophylactic treatment with hBD-2 (p<0.03; Figure 9). Barnesiella is a bacteria that has been found to be able to eliminate and protect against the intestinal dominance of antibiotic-resistant pathogenic bacteria that can be observed in hospitalized patients. The abundance of Barnesiella corresponds with the amount of several immunoregulatory cells. The higher the level of Barnesiella in the colon, the more marginal zone B cells and invariant natural killer T cells enumerated in the spleen and liver. Furthermore, a direct association between a change in microbial composition in favor of Barnesiella and the resistance to arthritis has been demonstrated in mice. In the development of colitis in IL-10-/- mice, higher levels of a Barnesiella phylotype correlated with lower activity levels of the disease.

### Conclusions:

Premature infants are almost exclusively born via Caesarian sectio. The procedure is performed under sterile conditions and the infants are thus born without a natural microbial population especially of the large mucosal surfaces in the intestines, lungs and skin. The only approved treatment of premature infants today as per the recent Cochrane analysis of therapies (Alfaleh and Annebrees, 2014) are probiotics based on the theory that the mucosal surfaces of these infants will have to be populated by microbes via external sources to establish a microbiota. A foetus starts to produce defensins from the beginning of the third trimester to prepare the mucosal surfaces for the massive bacterial colonization following birth via the natural birth canal and the ELBW premature infants are thus born without any or only a very small natural production of defensins. The above example serves to demonstrate that defensins in the intestines have a profound influence in promoting important commensal bacteria and maintaining a normal microbiota and that the microbiota modulating effect of two different defensins is different.

### Example 2. Modulation of gut microbiota by interventional treatment with defensins (Reference example).

### Materials and methods:

The overall design of the study is shown in Figure 1, lower panel, "Therapeutic study".

Mice and diets. The experiment elucidates the effect of hBD-2 and HD5 on the microbiota in diet-induced obese mice. A run-in period of 13 weeks where mice were fed a very HFD (60% energy from fat) preceded the intervention. Only mice meeting the criteria of a minimum of 12 gram weight gain (approximately 50% of initial bodyweight) during the run-in period were included in the final analyses. Mice that did not meet these criteria stayed in their respective cages as hierarchy 'keepers'. They were exposed to all experimental tests, but excluded from the analyses.

Treatment regimen. Before the intervention all mice were MR scanned. Cages of mice were allocated to experimental groups based on their fat mass. All subsequent measures were paired with data from the same mouse before the intervention. A LFD (low fat diet) reference group was running in parallel. As controls for the intervention 2 additional groups were included: a) very HFD and b) LFD. Experimental mice stayed on the very HFD during the intervention. The mice were on the experimental diet for 10 weeks. They were co-housed throughout the experiment, 4 mice per cage, 3 cages per group. All tests ran over 3 days, 1 cage per group per day.

### Tests:

Microbial analyses were carried out to study the microbiota of the intestine.

Longitudinal 16S characterization was conducted on 4 paired samples from 60 mice, 240 samples in total. Each mouse was sampled prior to diet change, 1 week post diet change, 4 weeks post diet change and at termination, thus ensuring a thorough characterization of the faecal microbiota as a result of defensin treatment.

### Results:

Weight change - hBD-2. The standard high fat diet (HFD) fed groups had an equal food intake throughout the entire study period and had the same weight development with equal fat and lean mass the first 13 weeks, thus having the same starting point prior to the dietary intervention. The weight gain was significantly larger than in the low fat diet fed (LFD) group (*p<0.05 2-way ANOVA). After the dietary intervention the HFD groups continued to increase in weight, however the HFD plus hBD-2 group tended to gain less weight the first 4 weeks post dietary intervention, although not significant (*p=0.07, 2-way ANOVA). From week 4 to the end of the study period the HFD plus hBD-2 group gained similar weight as the standard HFD group (*p=0.82, 2-way ANOVA).

Weight change - HD5. All HFD fed groups had the same food intake during the study period and equal weight gain during the run-in period of 13 weeks. After dietary intervention the group fed HFD plus HD5 gained significantly less weight than the HFD control (*p<0.05, 2-way ANOVA). In addition, a tendency of decreasing fat percentage in the HFD plus HD5 group was observed, and a significantly lower fat percentage in the HFD plus HD5 was measured 4 weeks after dietary change in comparison to the HFD control (*p=0.009 2-way ANOVA).

### Microbiota.

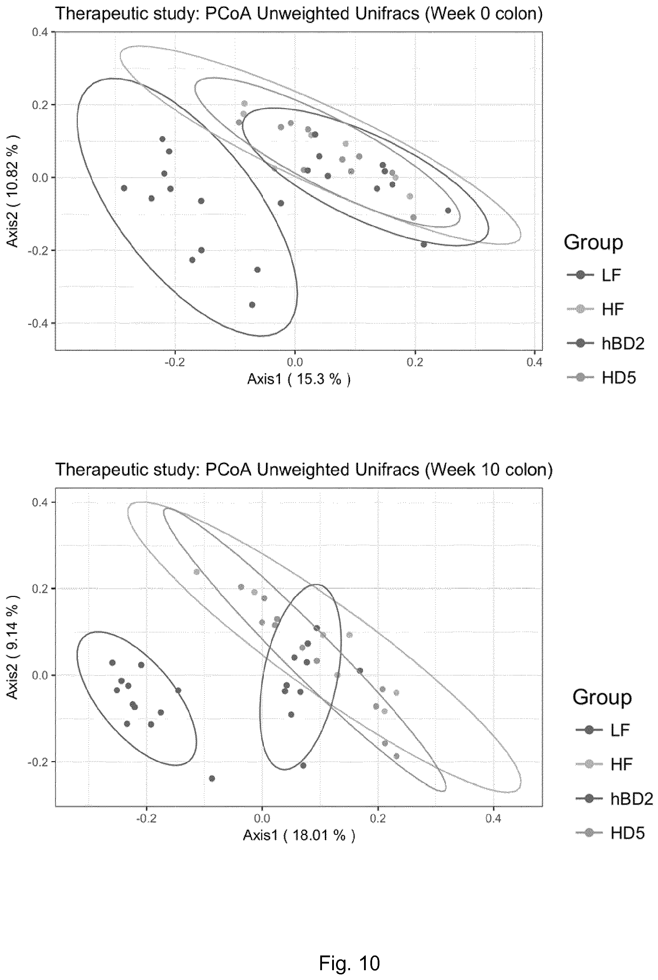

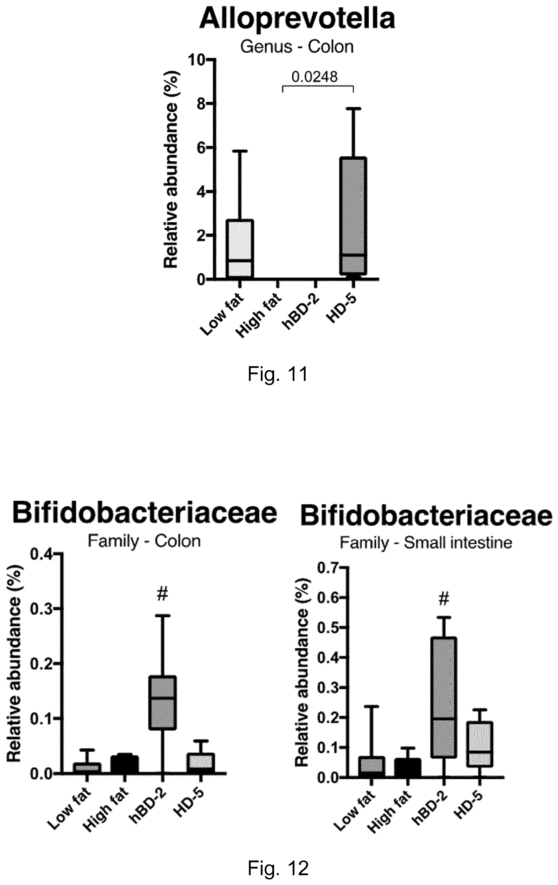

Both defensins were shown to have a profound influence on the bacterial presence as well as bacterial absence i.e. the capability to change the microbiota fascilitating the colonization of "good" bacteria and decreasing the colonization of "bad" bacteria or pathogens (Figure 10). HD5 increased the abundance of Alloprevotella statistically significantly in the colon (p<0.02) whereas hBD-2 had no influence on Alloprevotella abundance (Figure 11). hBD-2 dramatically and statistically significant increased the relative abundance of Bifidobacteriaceae both in the small intestine and in the colon (p<0.0001 and p<0.04 respectively; Figure 12). There was a trend towards HD5 increasing the abundance of Bifidobacteriaceae in the small intestine (Figure 12).

### Conclusions:

Premature infants are almost exclusively born via Caesarian sectio. The procedure is performed under sterile conditions and the infants are thus born without a natural microbial population especially of the large mucosal surfaces in the intestines, lungs and skin. The only approved treatment of premature infants today as per the recent Cochrane analysis of therapies (Alfaleh and Annebrees, 2014) are probiotics based on the theory that the mucosal surfaces of these infants will have to be populated by microbes via external sources to establish a natural microbiota. A foetus starts to produce defensins from the beginning of the third trimester to prepare the mucosal surfaces for the massive bacterial colonization following birth via the natural birth canal. The above example thus serves to demonstrate that defensins in the intestines have a profound influence in establishing and maintaining a normal microbiota and that the microbiota modulating effect of two different defensins is different.

### Example 3. Method to determine the efficacy of prophylactic treatment with oral mammalian α- and β-defensins in a premature piglet model of necrotizing enterocolitis.

### Materials and methods:

Treatment regime: 24 preterm piglets are delivered by caesarean section on day 105 of gestation. The newborn piglets are immediately transferred to incubators with regulated temperature and oxygen supply. When respiration has stabilized, the piglets are fitted with umbilical and orogastric catheters. All piglets are initially provided parenteral nutrition via an esophageal tube. The enteral formula diet is made from three commercially available products used for feeding infants 0-2 years of age.

Piglets are stratified according to birth weight and sex and allocated into a control and an intervention group receiving hBD-2.

### Tests:

Signs of discomfort or weakness (unwillingness to stand, cold extremities, distended abdomen, dehydration, pale skin color, diarrhea and bloody diarrhea) are recorded.

The piglets are weighed every day and their food intake recorded.

An in vivo intestinal permeability test with lactulose and mannitol is performed.

On day 5, all piglets are euthanized, tissue is collected, and a macroscopic NEC scoring system applied. Briefly each of the 5 regions (stomach, proximal, mid and distal small intestine and the colon) of the gastrointestinal tract are macroscopically evaluated for pathological changes, indicative of inflammation and necrosis. The lesions are graded as follows: 1=absence of lesions; 2=local hyperemia; 3=hyperemia, extensive edema and local haemorrhage; 4=extensive haemorrhage; 5=local necrosis and pneumatosis intestinalis and 6=extensive transmural necrosis and pneumatosis intestinalis. An animal is designated as NEC positive when a minimum disease score of 3 in at least one region is observed.

For determination of mucosal proportion, a 10 cm segment from each small intestinal region is removed and slit along its length. The mucosa is gently scraped off with a plastic slide, and the proportion of mucosa determined on a wet weight basis. The weight of the heart, lungs, liver, kidneys, spleen and stomach are determined on a wet weight basis.

Samples of PFA-fixed intestine from proximal and distal regions of the small intestine and colon are embedded in paraffin and stained with HE for histomorphology and histopathology. For quantification of goblet cells in the distal small intestine and colon, tissue slides are stained with Alcian Blue and Periodic Acid Schiff.

As a measure of inflammation, tissue myeloperoxidase activity is assayed. IL-1β, IL-6, IL-8 and TNF-α are determined on tissue samples from the distal small intestine. Brush border enzyme activity is measured in snap-frozen samples from the proximal, middle and distal regions of the small intestine from each piglet.

### Example 4. Efficacy of s.c. hBD-2 versus s.c. anti TNF-α (Enbrel) and intraperitoneal dexamethasone in a therapeutic, murine, 14 weeks, CD4+CD25+ T cell transfer induced colitis model.

### Materials and methods:

Treatment regime: 70 female BALB/c mice housed in groups of five per cage were allocated to 7 different treatment groups. Colitis was induced in SCID mice by transplantation of CD4+CD25 Tcells from BALB/c mice. Lymphocytes isolated from spleen and lymph nodes from BALB/c mice were subjected to negative selection of CD4+ Tcells. Afterwards, CD4+CD25+ cells were positively isolated by binding to the beads from the CD4+ Tcell suspensions and the CD4+CD25- were collected from the supernatant. The animals were treated with hBD-2, 0.1 mg/kg s.c. once daily 86 consecutive days from day 7 (OD); 1 mg/kg s.c. OD or 3 mg/kg s.c. OD;with mouse anti TNFα 100 µg/mouse s.c. twice weekly (enbrel); with dexamethasone 0.3 mg/kg intraperitoneally OD (dex); with PBS s.c. as vehicle from day 7 and 86 consecutive days (vehicle) or sham treated (sham).

### Treatment groups:

1. 0.1 mg/kg hBD-2, once daily subcutaneously (n= 11)
2. 1 mg/kg hBD-2, once daily subcutaneously (n=11)
3. 3 mg/kg hBD-2, once daily subcutaneously (n=11)
4. PBS (hBD-2 vehicle), once daily subcutaneously (n= 11)
5. 0.3 mg/kg Dexamethasone, once daily intraperitoneally (n= 11)
6. 100 µg/mouse soluble human 75-kilodalton TNF receptor linked to Fc portion of IgG1 (Enbrel), twice a week subcutaneously (n= 11)
7. Sham treated group (n= 6)

### Tests:

Clinical assessment was based on body weight loss, stool consistency and presence of blood per rectum. The animals were sacrificed on day 95 and colon removed for assessment of weight and myeloperoxidase activity.

### Results:

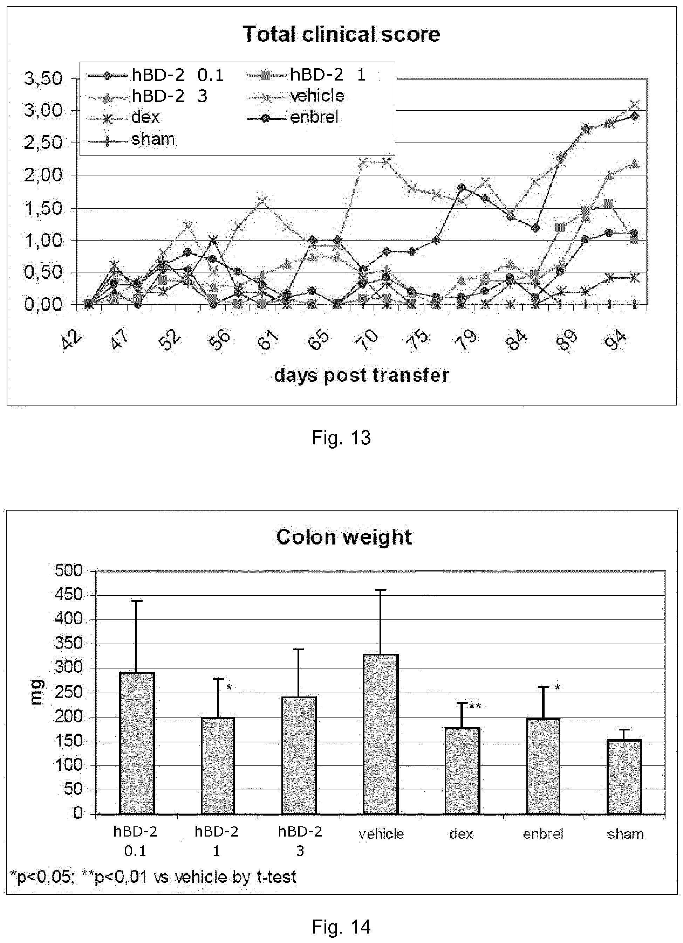

A statistically significant and similar effect on clinical score was observed for hBD-2 1 mg/kg s.c. OD, for Dexamethasone 0.3 mg/kg i.p. OD and for Enbrel (Figure 13).

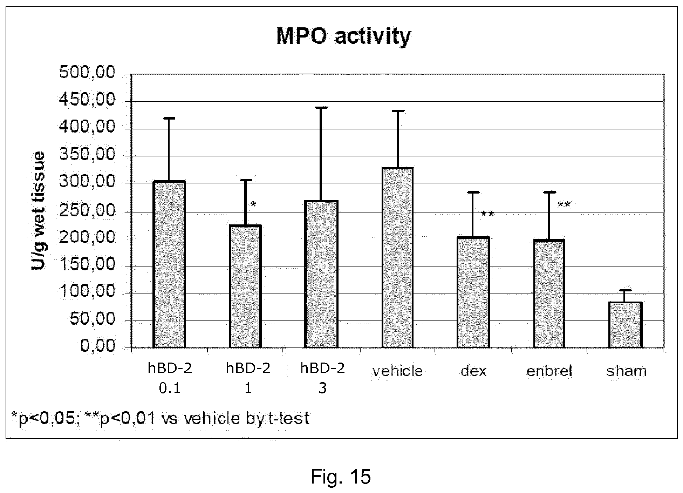

Similarly these three dosing regimens showed a statistically significant and similar effect on colon weight (Figure 14) and myeloperoxidase activity (Figure 15).

### Conclusions:

DSS induces a type of colitis in mice that shares a number of characteristics with necrotizing enterocolitis seen in human preterm infants. This example serves to demonstrate that hBD-2 had a statistically significant effect in relieving especially clinical symptoms on par with both dexamethasone and anti TNF-α, compounds that are commonly used to treat necrotizing enterocolitis.

### Example 5. Determining and assessing the efficacy of prophylactic treatment with Intranasal versus Oral mammalian β-defensins in a murine house dust mite driven model of allergic asthma (Reference example).

### Materials and methods:

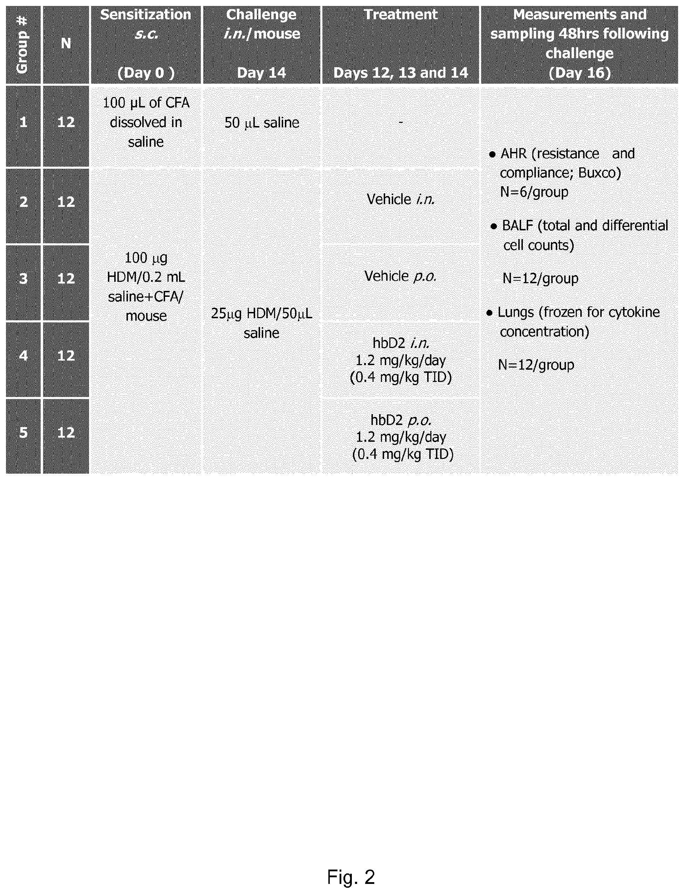

The study design is shown in Figure 2.

Treatment regime: Female 7-10 weeks old BALB/c mice were randomly allocated into 5 study groups one day prior to study start and subcutaneously (SC) sensitized to house dust mite (100 µg HDM in 200 µL saline plus Freund's complete adjuvant in 0.9% saline). The mice were treated with hBD-2 orally and intranasally respectively at a dose of 1.2 mg/kg/day (0.4 mg/kg TID) starting on day 12 in the morning and continued TID at approximately 6 hours intervals. The last dose was administered on day 14 one hour prior to challenge. The total number of doses was 8 doses or a total of 2 mg/kg hBD-2. Mice were then intranasally (IN) challenged with HDM on day 14 (HDM 25 µg in 50µL of saline) (Figure 2).

### Tests:

Airway inflammation: At 48 hours post challenge, bronchoalveolar lavage was performed washing the lungs with 3 volumes of cold PBS (0.4; 0.3 and 0.3 mL, total 1 mL). Total and differential leucocyte cell counts were determined on an automated haematological analyser Sysmex XT-2000iV.

Lung function: Starting 48 hours after HDM challenge, measurements of lung resistance and lung compliance were carried out after methacholine challenge (3.125 MCH1; 6.25 MCH2; 12.5 MCH3 and 25 mg/mL MCH4) by anaesthetized, cannulated mice using DSI's Buxco Finepoint RC system. Data are represented as airway resistance at 10 mg/kg methacholine and as dose responsive curves.

Lung sampling for cytokine analysis: After completion of every BAL, lungs were removed from the thorax, snap frozen in liquid nitrogen and stored frozen at -80 degrees Celcius until analysis of cytokine concentration of TNF-α, IL-4, IL-5, IL-6, IL-9, IL-13 and IL-33 in lung homogenate by ELISA.

### Results:

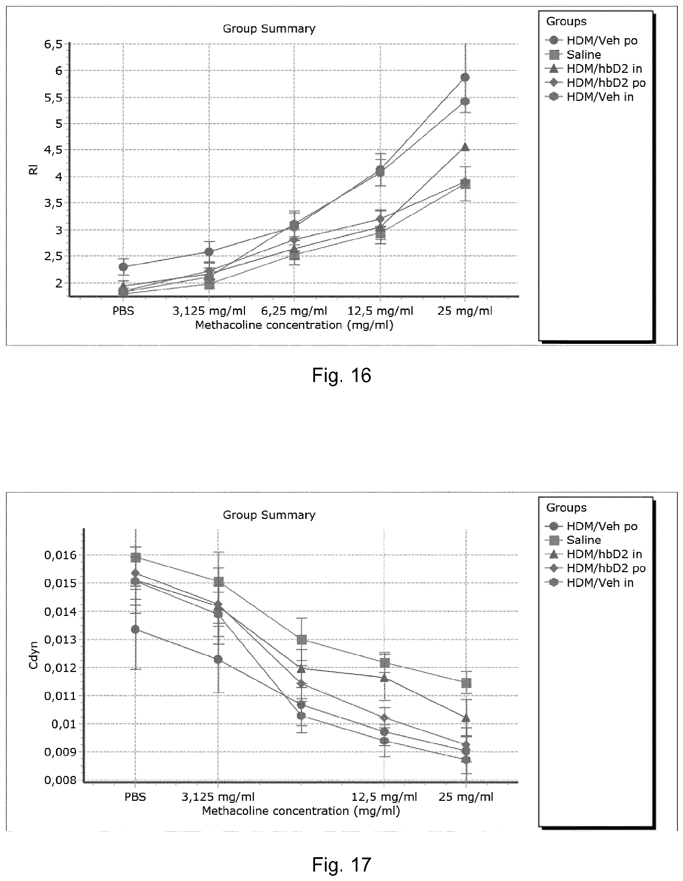

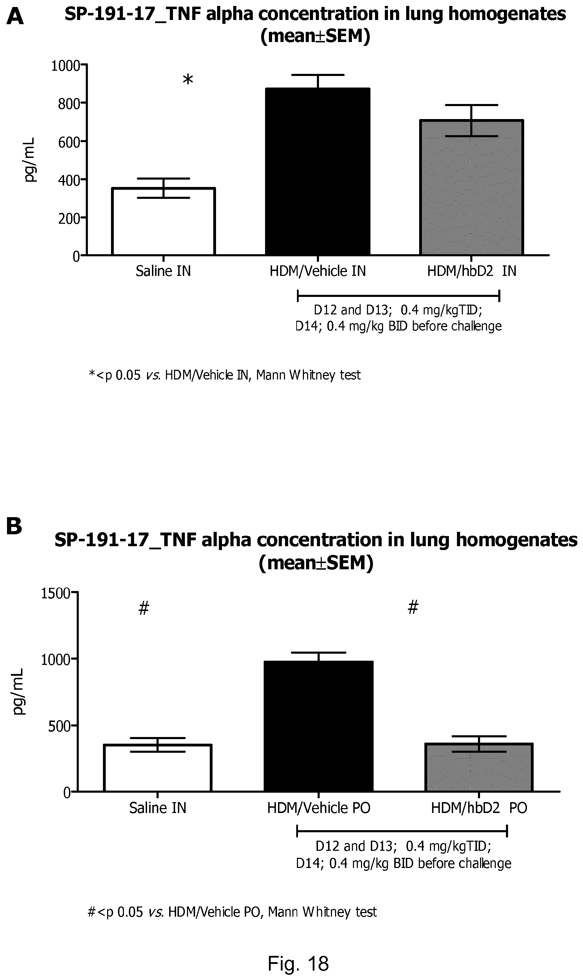

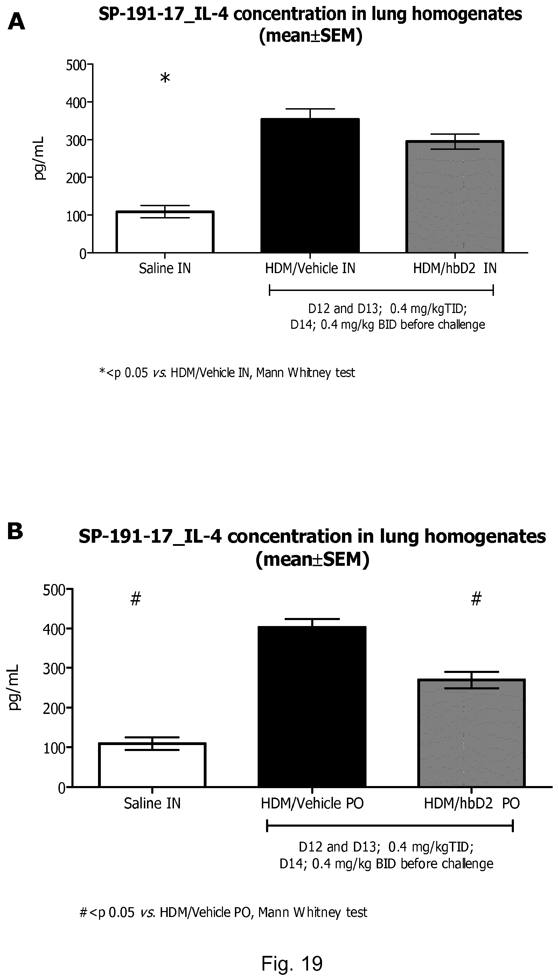

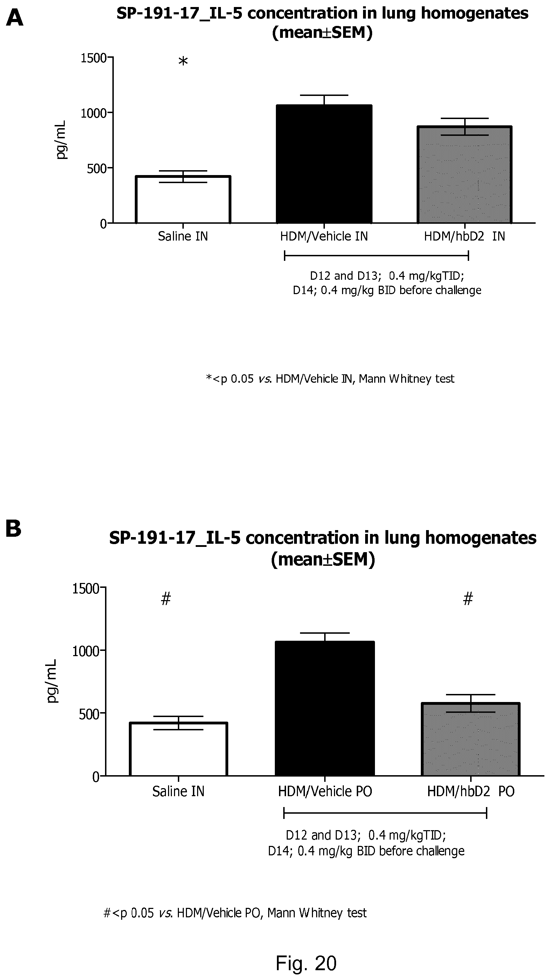

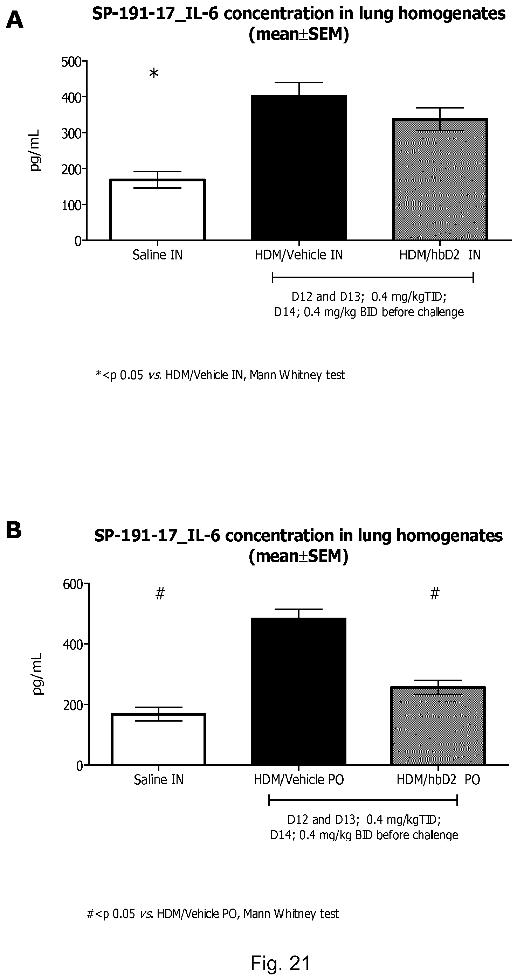

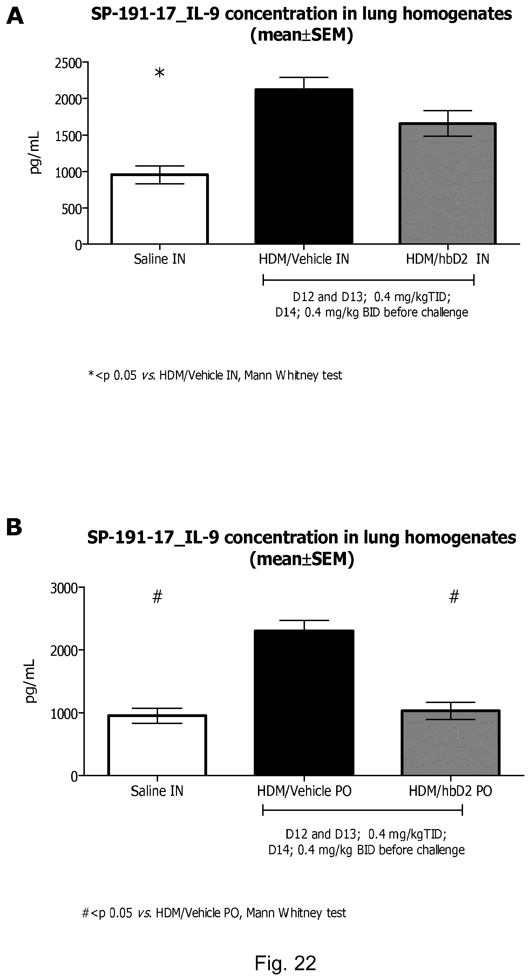

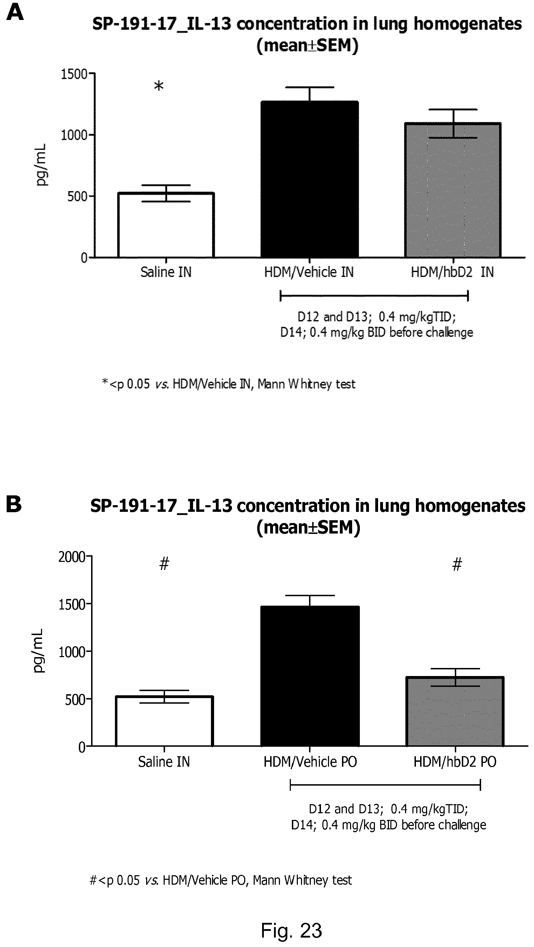

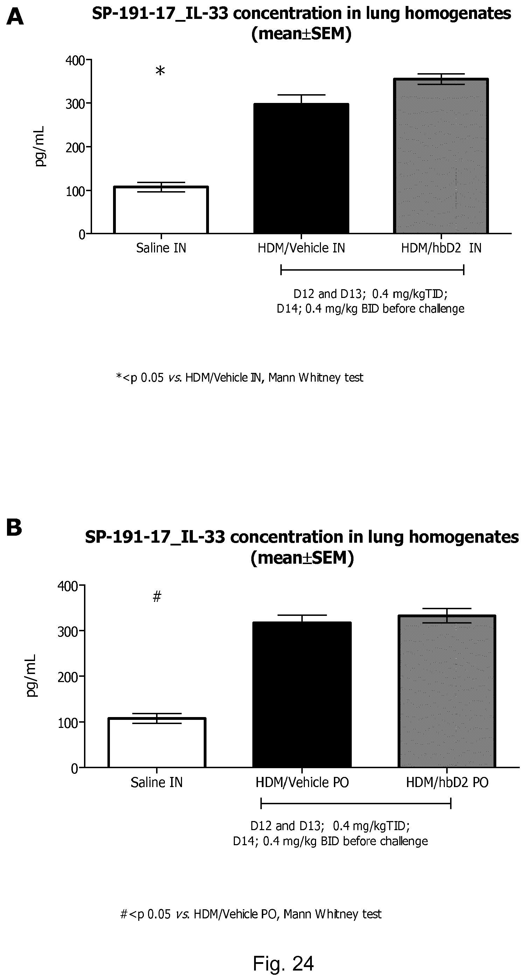

An increase of lung resistance values and decrease of pulmonary compliance values in HDM-challenged vehicle treated animals in comparison to saline-challenged (non-asthmatic) mice was observed (Figure 16 and 17). An inflammatory response in both vehicle-treated groups of mice (oral and intranasal) was induced by a single HDM challenge 14 days post sensitization with HDM. It was characterized by a statistically significant increase in total cell, eosinophil, neutrophil, macrophage and lymphocyte counts in BALF (p<0.05) when compared to saline-challenged controls (data not shown). Also, analysis of concentration of seven cytokines TNF-α, IL-4, IL-5, IL-6, IL-9, IL-13 and IL-33 in lung tissue homogenates revealed significantly higher levels in HDM-challenged animals compared to saline-challenged controls (Figures 18-24).

hBD-2, both after oral and intranasal application TID, administered from day 12 to day 14 (a total of 2.0 mg/kg in 8 administrations), effectively inhibited increase of airway resistance (Figure 16) and decrease of pulmonary compliance (Figure 17) as compared to HDM challenged vehicle treated animals. An effect on cellular influx in BALF was observed after oral application, which significantly inhibited neutrophil counts (data not shown). A rebalance of the immune system with a complete normalization of cytokine concentrations in lung tissue homogenates was observed on TNF-α, IL-4, L-5, IL-6, IL-9 and IL-13 cytokine levels following oral administration (Figure 18b-24b). There was a trend towards lowering of TNF-α, IL-4, IL-5, IL-6, IL-9 and IL-13 following intranasally administered hBD-2, but this was not statistically significantly different from the control (Figure 18a-24a). All obtained results indicate clear preventive and anti-inflammatory effects of hBD-2 in the house dust mite driven mouse model of allergic asthma.

### Conclusions:

The high mortality in especially ELBW infants is primarily caused by sepsis either due to bacterial translocation from necrotizing enterocolitis in the immature intestines or bacterial translocation from pneumonia and inflammation of the immature lung. The lung in especially ELBW infants is not fully developed and production of hBD-2 at best limited. This example serves to demonstrate that oral treatment with hBD-2 has a profound influence on inflammation and cytokine production in the lung and perhaps even more importantly on lung function.

### Example 6. Determining and assessing the efficacy of Intranasal versus Oral therapeutic intervention with mammalian β-defensins in a murine house dust mite/Freunds complete adjuvant driven model of allergic asthma (Reference example).

### Materials and Methods:

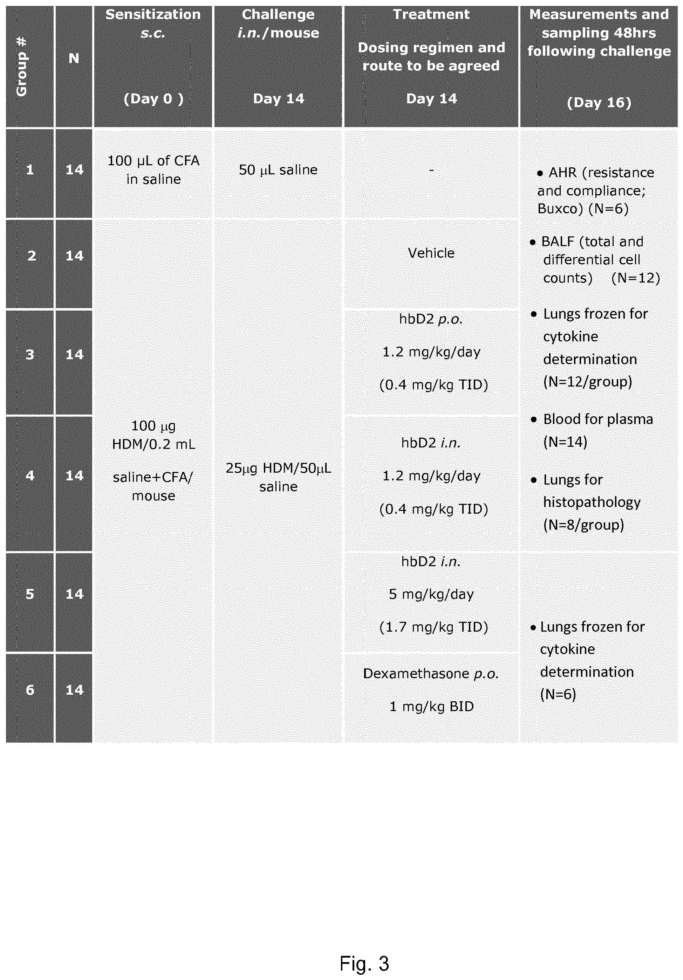

The study design is shown in Figure 3.

Treatment regime: Female 7-10 weeks old BALB/c mice were randomly allocated into 6 study groups one day prior to study start and subcutaneously (SC) sensitized to house dust mite (100 µg HDM in 200 µL saline plus Freund's complete adjuvant in 0.9% saline). Mice were then intranasally (IN) challenged with HDM on day 14 (HDM 25 µg in 50µL of saline). Dexamethasone was administered orally (1 mg/kg BID; 50µL phosphate buffered saline (PBS)) on day 14. hBD-2 was administered IN or orally (1.7 mg/kg TID IN; 0.4 mg/kg TID IN; 0.4 mg/kg TID orally, 50 µL phosphate buffered saline) on day 14. The initial dose was administered 60 minutes prior to challenge, and the subsequent two doses approximately 6 hours apart (Figure 3).

### Tests:

Airway inflammation: At 48 hours post challenge, bronchoalveolar lavage was performed washing the lungs with 3 volumes of cold PBS (0.4; 0.3 and 0.3 mL, total 1 mL). Total and differential leucocyte cell counts were determined on an automated haematological analyser Sysmex XT-2000iV.

Lung function: Starting 48 hours after HDM challenge, measurements of lung resistance and lung compliance were carried out after methacholine challenge (3.125 MCH1; 6.25 MCH2; 12.5 MCH3 and 25 mg/mL MCH4) by anaesthetized, cannulated mice using DSI's Buxco Finepoint RC system. Data are represented as airway resistance at 10 mg/kg methacholine and as dose responsive curves.

Lung sampling for cytokine analysis: After completion of every BAL, lungs were removed from the thorax, snap frozen in liquid nitrogen and stored frozen at -80 degrees Celcius until analysis of cytokine concentration of IL-1β, TNF-α, IL-6, IL-10 and IFNγ by ELISA.

### Results:

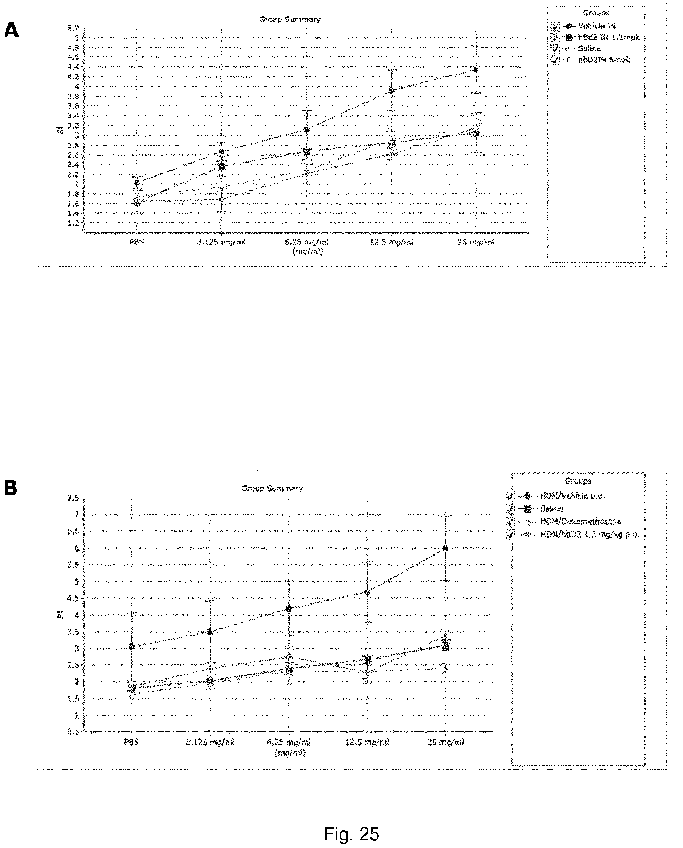

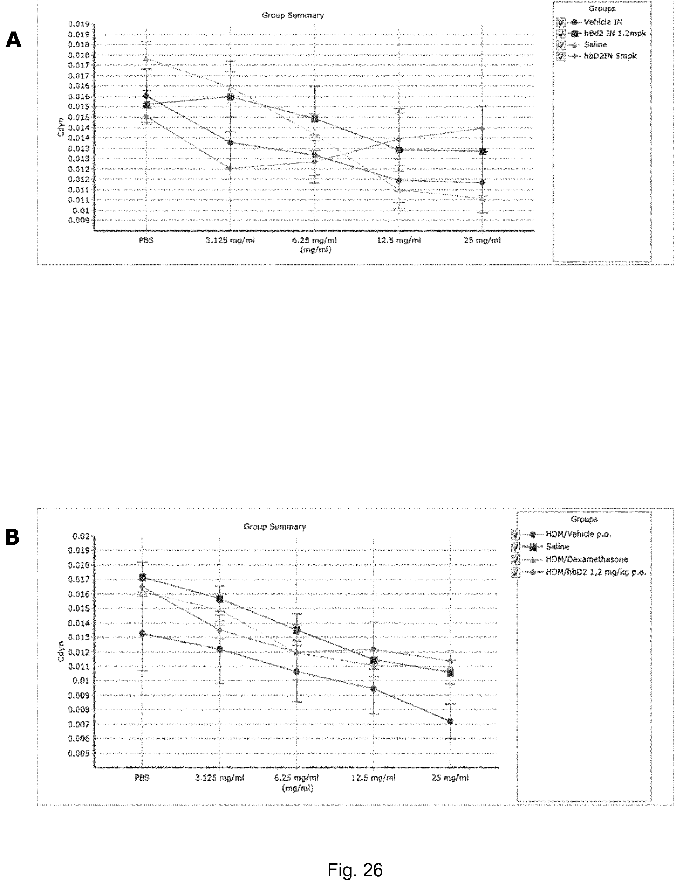

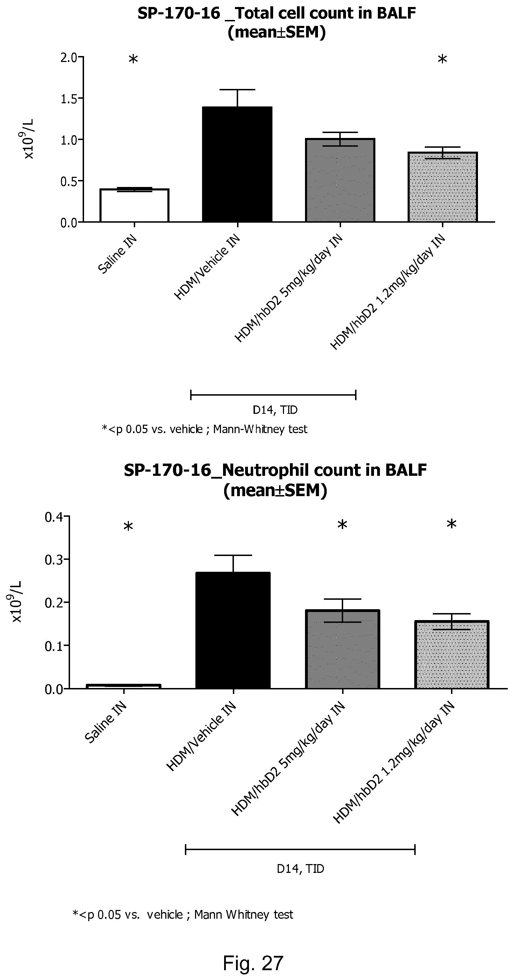

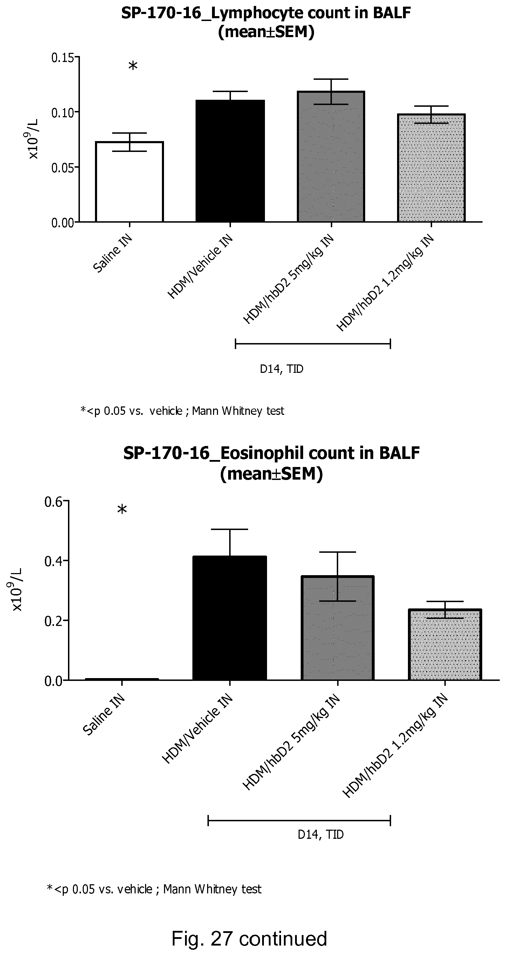

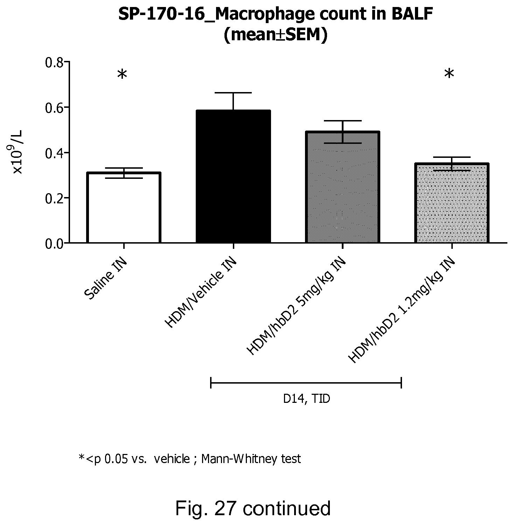

An increase of lung resistance values and decrease of pulmonary compliance values in HDM-challenged vehicle treated animals in comparison to saline-challenged (non-asthmatic) mice was observed (Figure 25 and 26). An inflammatory response in both vehicle-treated groups of mice (oral and intranasal) was induced by a single HDM challenge 14 days post sensitization with HDM and adjuvant. It was characterized by a statistically significant increase in total cell, eosinophil, neutrophil, macrophage and lymphocyte counts in BALF (p<0.05) when compared to saline-challenged controls (Figure 27). Also, analysis of concentration of five cytokines TNF-α, IL-6, IL-10 and IFN-γ in lung tissue homogenates revealed significantly higher levels in HDM-challenged animals compared to saline-challenged controls.

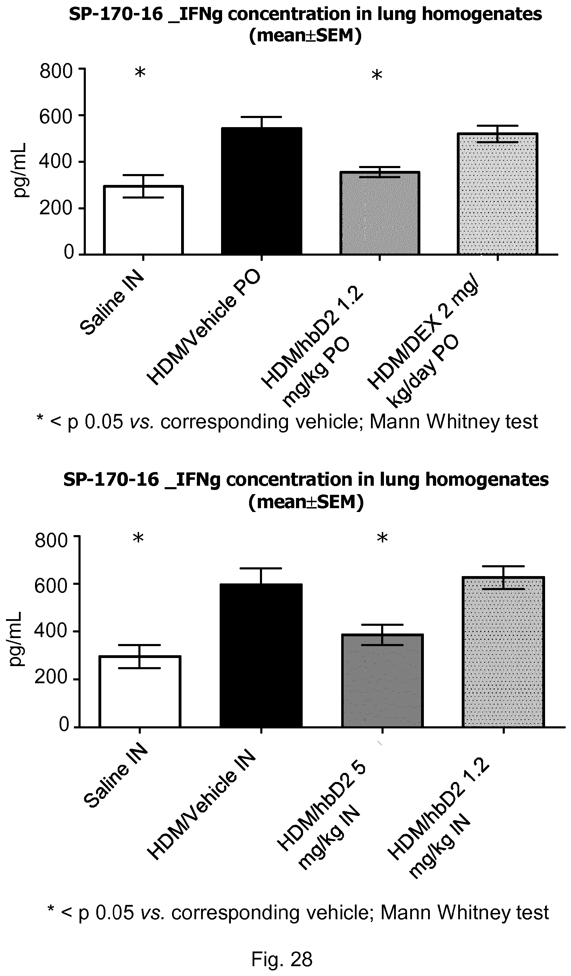

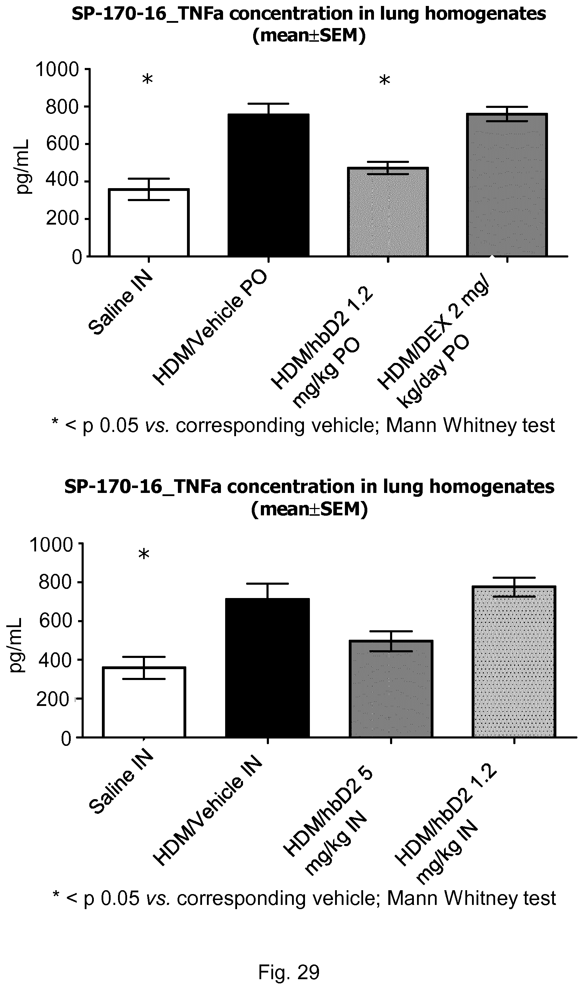

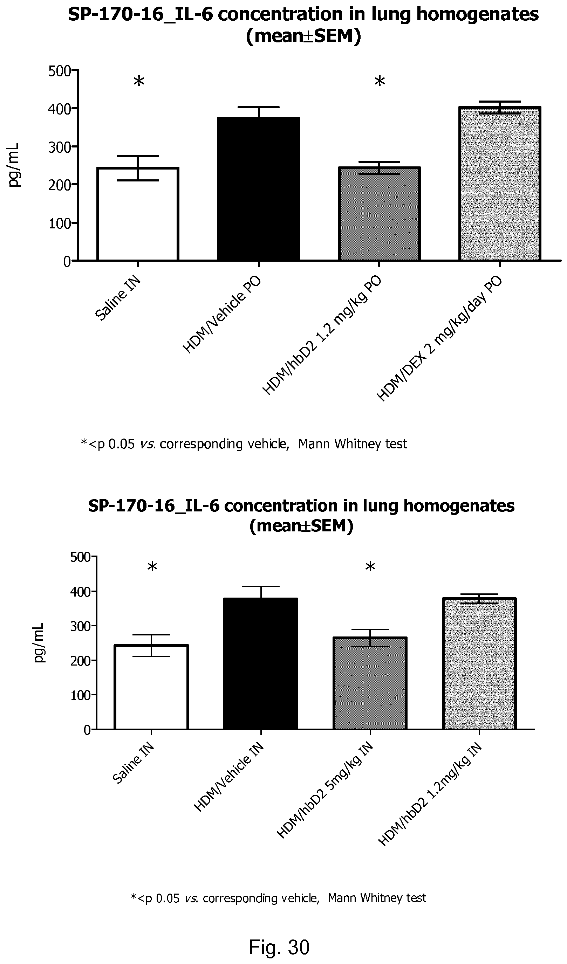

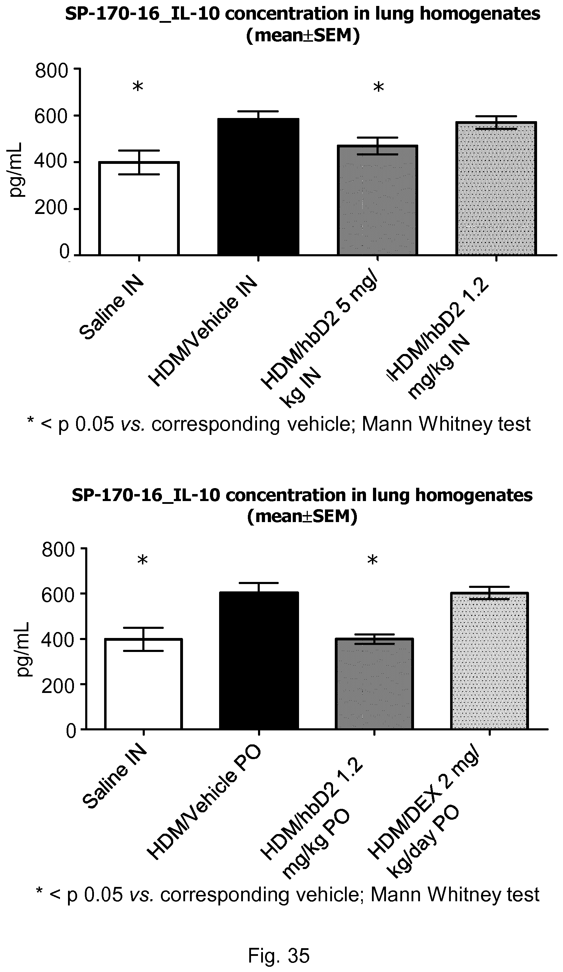

Dexamethasone treatment significantly inhibited total cell and eosinophil counts but not neutrophil, macrophage and lymphocyte counts in BALF (data not shown). In accordance with the cellular data, dexamethasone did not influence levels of TNF-α, IL-6, IL-10 and IFN-γ in lung tissue homogenates as compared to HDM/vehicle control (Figures 28 to 29). However, it influenced AHR measurements related to eosinophil counts. Obtained results indicate that this model is steroid resistant to a certain degree. hBD-2, both after oral and intranasal application TID, on day 14, effectively inhibited increase of airway resistance (Figure 25a and 25b) and decrease of pulmonary compliance (Figure 26a and 26b) as compared to HDM challenged vehicle treated animals. A more prominent effect was observed on some measured parameters after intranasal application, such as cellular influx in BALF, where both doses (0.4 mg/kg/day TID and 1.7 mg/kg/day TID) significantly inhibited total, neutrophil and macrophage cell counts and a trend towards lowering of eosinophils (Figure 27), while the steroid standard dexamethasone failed to inhibit them (data not shown). Similar significant effects were observed on IL-6, IL-10 and IFN-γ cytokine levels in lung tissue homogenates with both dosing routes (Figure 28, 32, 35). Perorally administered hBD-2 significantly reduced TNF-α (Figure 29), while the intranasally administered hBD-2 was not significantly different from the controlAll obtained results indicate clear anti-inflammatory effects of hBD-2 in the house dust mite/Freund's complete adjuvant driven mouse model of allergic asthma.

### Conclusions:

The high mortality in especially ELBW infants is primarily caused by sepsis either due to bacterial translocation from necrotizing enterocolitis in the immature intestines or bacterial translocation from pneumonia and inflammation of the immature lung. The lung in especially ELBW infants is not fully developed and production of hBD-2 at best limited. This example serves to demonstrate that oral treatment with hBD-2 has a profound influence on inflammation and cytokine production in the lung and perhaps even more importantly on lung function.

### Example 7. To determine and assess the efficacy of Intranasal versus Oral therapeutic intervention with administration of mammalian β-defensins in a murine house dust mite/Freund's complete adjuvant driven model of allergic asthma (Reference example).

### Materials and Methods:

Study design is shown in Figure 3.

Treatment regime: Female 7-10 weeks old BALB/c mice randomly allocated into 4 study groups one day prior to study start were subcutaneously (SC) sensitized to house dust mite (100 µg HDM in 200 µL saline plus Freund's complete adjuvant in 0.9% saline). Mice were intranasally (IN) challenged with HDM on day 14 (HDM 25 µg in 50µL of saline). hBD-2 was administered IN or orally (0.4 mg/kg TID IN; 0.4 mg/kg TID orally, 50 µL phosphate buffered saline) on day 14. The initial dose was administered 60 minutes prior to challenge, and the subsequent doses approximately 6 hours apart (Figure 3, groups 1-4)).

### Tests:

Blood sampling: All terminal blood samples were collected via jugular vein bleeds.

Blood was sampled to Li-heparin tubes, put on ice and immediately centrifuged at 4⁰C.

Plasma was separated and stored at -80⁰C until the potential SCFA analysis.

Lung tissue sampling: The lungs were exposed and excised by gently opening the thorax and by cutting down either side of the sternum and ribs and trimming back. Lungs from the first 6 animals per group were removed from thorax, snap frozen in liquid nitrogen and stored frozen at -80°C until analysis of cytokine concentration of IL-4, IL-5, IL-8 (KC), IL-9 and IL-13 in lung homogenate by ELISA.

Lungs from the other 8 animals per group were inflated *in situ* with 10% buffered formalin, removed from thorax, placed individually in 10% buffered formalin, paraffin embedded *in toto,* sectioned and H&E/PAS stained. The paraffin blocks were retained for the IHC analysis.

### Read-outs

- Histopathology (H&E; PAS) (N=8/group; total N=32)
- Cytokines in lung tissue homogenates (IL-4, IL-5, IL-8 (KC), IL-9 and IL-13) (N=6/group; total N=24)

### Histopathology

Cellular influx (mononuclears, eosinophils, neutrophils) was assessed semi-quantitatively on H&E stained slides separately for peri-bronchial/bronchiolar and perivascular space as follows:
0 absent
1 few scattered inflammatory cells
2 larger aggregates
3 marked accumulation of cells

Overall score for inflammation was calculated as the sum of all individual scores. Goblet cell metaplasia, separately at a level of large airways and distal airways, was assessed at PAS-stained slides as follows:
0 no mucus containing cells along basement membrane
1 few positive cells along basement membrane with less than 75% of the cytoplasm stained
2 few positive cells along basement membrane with more than 75% of the cytoplasm stained
3 numerous positive cells along basement membrane with less than 75% of the cytoplasm stained
4 numerous positive cells along basement membrane with more than 75% of the cytoplasm stained

### Statistical evaluation

Data was processed using MS Excel. Statistical analysis was performed using GraphPad Prism software (version 5.04). Differences between groups are considered statistically significant when p<0.05.

Statistical analysis of selected histological score-values data was performed using median and non-parametric Mann-Whitney test.

### Results:

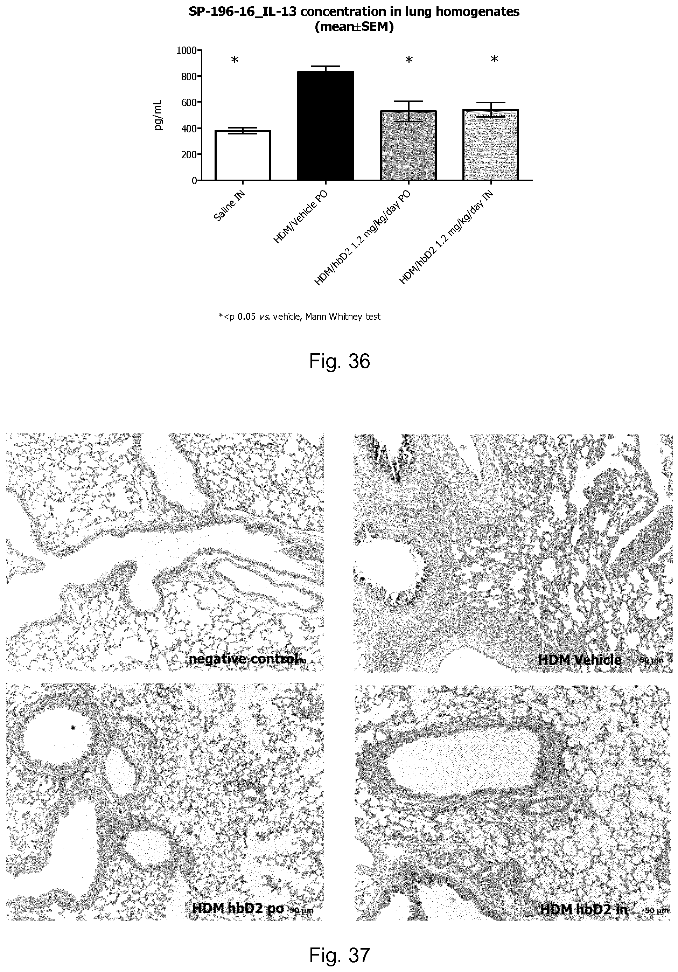

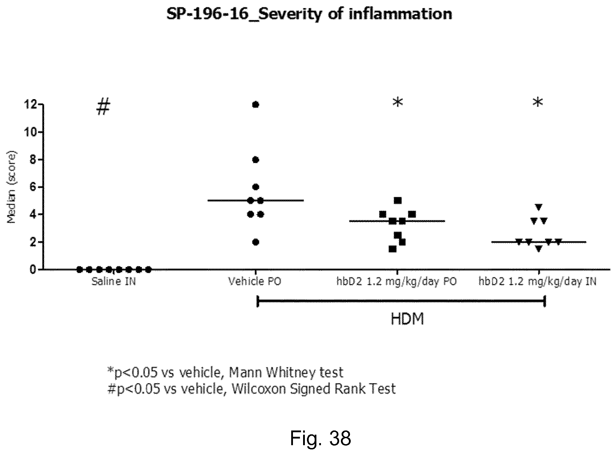

An inflammatory response in both vehicle-treated groups of mice (oral and intranasal) was induced by a single HDM challenge 14 days post sensitization with HDM and adjuvant. It was characterized by a statistically significant increase in concentration of five cytokines IL-4, IL-5, IL-8, IL-9 and IL-13 in lung tissue homogenates and by severe histological inflammatory changes of lung tissue in HDM-challenged animals compared to saline-challenged controls (Figure 37 and 38).

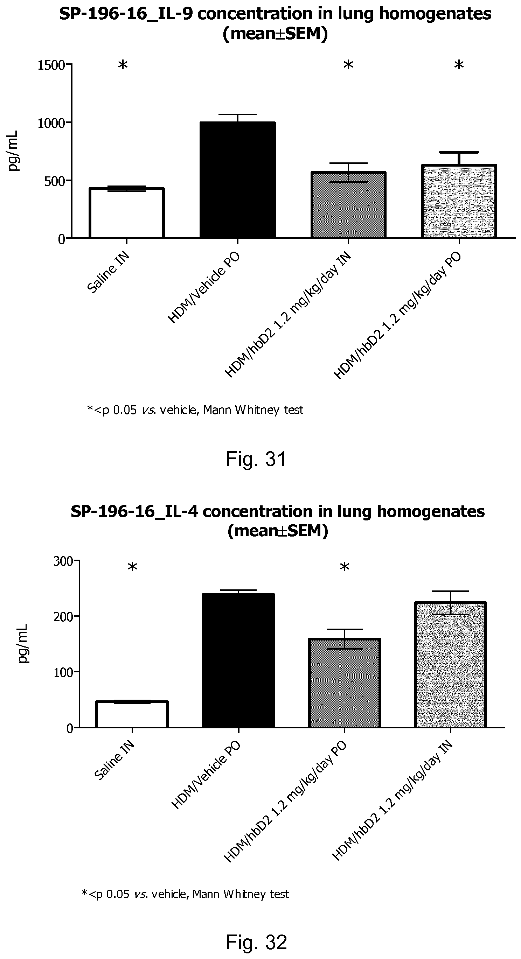

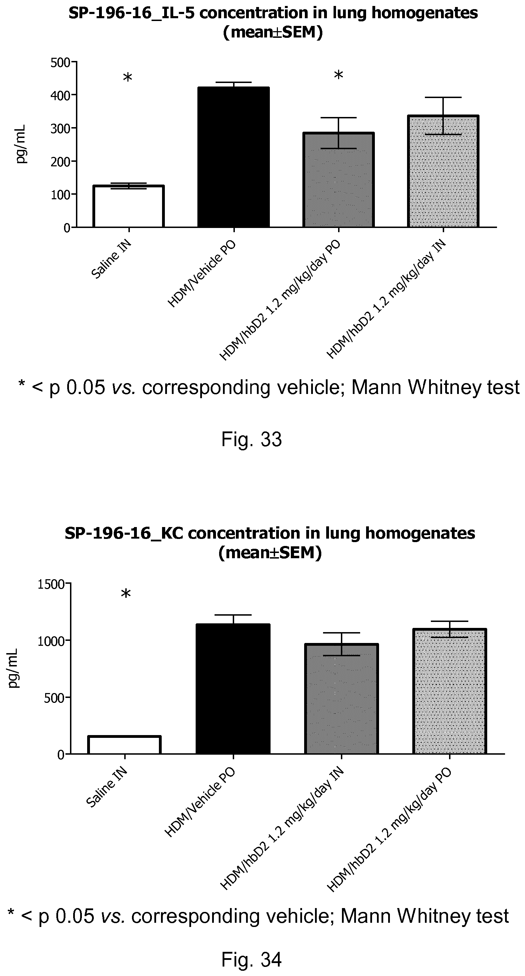

hBD-2, both after oral and intranasal application TID, on day 14, effectively inhibited the increase in histological inflammation of lung tissue as compared to HDM challenged vehicle treated animals (Figure 37 and 38). Significant effects were observed on IL-4, IL-5, IL-9 and IL-13 cytokine levels in lung tissue homogenates following oral administration (Figure 32, 33, 31, 36) and on IL-9 and IL-13 (Figure 31, 36) following IN administration. All obtained results indicate clear anti-inflammatory effects of hBD-2 in the house dust mite/Freund's complete adjuvant driven mouse model of allergic asthma.

### Conclusions:

The high mortality in especially ELBW infants is primarily caused by sepsis either due to bacterial translocation from necrotizing enterocolitis in the immature intestines or bacterial translocation from pneumonia and inflammation of the immature lung. The lung in especially ELBW infants is not fully developed and production of hBD-2 at best limited. This example serves to demonstrate that oral treatment with hBD-2 has a profound influence on inflammation and cytokine production in the lung and perhaps even more importantly on lung function.

### Example 8.

PBMC cell viability following exposure for 24 hours to Alamar Blue and HD5,

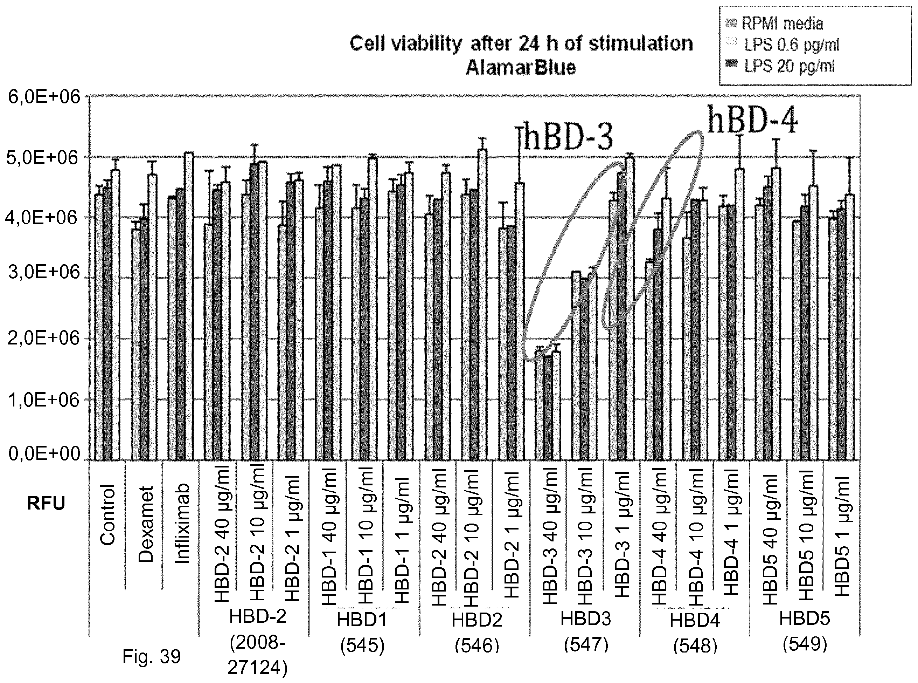

hBD-1, hBD-2, hBD-3 and hBD-4 at concentrations of 1, 10 and 40 µg/ml at 0.6 and 20.0 pg/ml LPS respectively (Figure 39).

The experiment shows that HD5, hBD-1 and hBD-2 are not toxic irrespectively of concentration whereas hBD-4 shows moderately increasing toxicity and hBD-3 shows profoundly increasing toxicity at increasing concentrations.

### Example 9.

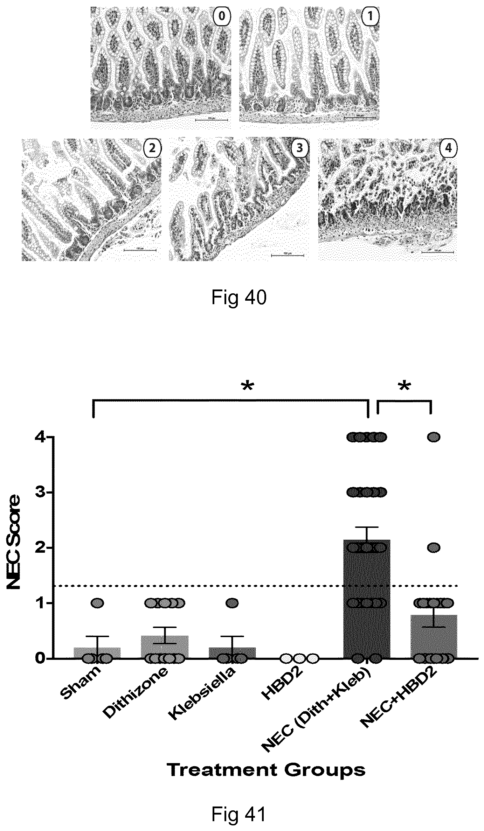

Incidence of necrotizing enterocolitis and IL-22 concentration in a murine model of necrotizing enterocolitis. Intestinal mucosal injury consistent with human necrotizing enterocolitis was induced by a combination of Dithizone followed by Klebsiella pneumonia challenge by oral gavage in postnatal day 14 C57BL/6J mice. The severity of necrotizing enterocolitis was assessed by a histological scoring system with a score of > 2 indicating significant damage (Figure 40). Neither Dithizone nor K. pneumonia challenge induced mucosal injury by themselves, but the combination of Dithizone followed by K. pneumonia challenge induced severe and statistically significant mucosal damage measured 16 hours post challenge (Figure 41). Prophylactic oral treatment with 1.2 mg/kg hBD-2 one hour post K. pneumonia challenge dramatically and statistically significantly reduced the incidence of necrotizing enterocolitis to normal (Figure 41). The concentration of IL-22, a mucosal barrier enhancing cytokine, was statistically significantly increased 9 hours post challenge.

### Example 10.

### Sequences

| **SEQ ID NO:** | **Name** | **Sequence** |
|---|---|---|
| 1 | hBD-1 | DHYNCVSSGGQCLYSACPIFTKIQGTCYRGKAKCCK |
| 2 | hBD-2 | GIGDPVTCLKSGAICHPVFCPRRYKQIGTCGLPGTKCCKKP |
| 3 | hBD-3 | GII NTLQKYYCRVRGGRCAVLSCLPKEEQIGKCSTRGRKCCRRK |
| 4 | hBD-4 | ELDRICGYGTARCRKKCRSQEYRIGRCPNTYACCLRK |
| 5 | HD5 | ATCYCRTGRCATRESLSGVCEISGRLYRLCCR |
| 6 | HD6 | AFTCHCRRSCYSTEYSYGTCTVMGINHRFCCL |
| 7 | Mouse beta defensin 3 | KINNPVSCLRKGGRCWNRCIGNTRQIGSCGVPFLKCCKRK |
| 8 | HNP-1 | ACYCRIPACIAGERRYGTCIYQGRLWAFCC |
| 9 | HNP-2 | CYCRIPACIAGERRYGTCIYQGRLWAFCC |
| 10 | HNP-3 | DCYCRIPACIAGERRYGTCIYQGRLWAFCC |
| 11 | HNP-4 | VCSCRLVFCRRTELRVGNCLIGGVSFTYCCTRV |
| 12 | Human Cathelicidin (LL-37) | LLGDFFRKSKEKIGKEFKRIVQRIKDFLRNLVPRTES |
| 13 | Human Lactoferrin | GRRRSVQWCAVSQPEATKCFQWQRNMRKVRGPPVSCIKRDSP IQCIQAIAENRADAVTLDGGFIYEAGLAPYKLRPVAAEVYGTERQ PRTHYYAVAVVKKGGSFQLNELQGLKSCHTGLRRTAGWNVPIG TLRPFLNWTGPPEPIEAAVARFFSASCVPGADKGQFPNLCRLCA GTGENCAFSSQEPYFSYSGAFKCLRDGAGDVAFIRESTVFEDLS DEAERDEYELLCPDNTRKPVDKFKDCHLARVPSHAVVARSVNG KEDAIWNLLRQAQEKFGKDKSPKFQLFGSPSGQKDLLFKDSAIG FSRVPPRIDSGLYLGSGYFTAIQNLRKSEEEVAARRARVVWCAV GEQELRKCNQWSGLSEGSVTCSSASTTEDCIALVLKGEADAMS LDGGYVYTAGKCGLVPVLAENYKSQQSSDPDPNCVDRPVEGYL AVAVVRRSDTSLTWNSVKGKKSCHTAVDRTAGWNIPMGLLFNQ TGSCKFDEYFSQSCAPGSDPRSNLCALCIGDEQGENKCVPNSN ERYYGYTGAFRCLAENAGDVAFVKDVTVLQNTDGNNNEAWAK DLKLADFALLCLDGKRKPVTEARSCHLAMAPNHAVVSRMDKVE RLKQVLLHQQAKFGRNGSDCPDKFCLFQSETKNLLFNDNTECLA RLHGKTTYEKYLGPQYVAGITNLKKCSTSPLLEACEFLRK |
| 14 | Lactoferricin-H | GRRRSVQWCAVSQPEATKCFQWQRNMRKVRGPPVSCIKRDSP IQCIQA |
| 15 | Human Lysozyme | KVFERCELARTLKRLGMDGYRGISLANWMCLAKWESGYNTRAT NYNAGDRSTDYGIFQINSRYWCNDGKTPGAVNACHLSCSALLQ DNIADAVACAKRVVRDPQGIRAWVAWRNRCQNRDVRQYVQGC GV |
| 16 | truncated hBD2 | PVTCLKSGAICHPVFCPRRYKQIGTCGLPGTKCCKKP |

### Example 11 (Reference example).

To determine and assess the efficacy of prophylactic treatment with oral hBD-2 in a murine model of acute graft versus host disease following stem cell transplantation.

### Materials and methods

Treatment regime: 12 female BALB/c mice were irradiated with 4.5 Gy (2 x 498 sec) at intervals of at least 4 hours on day 0. Bone marrow was harvested: hind limbs harvested from 2 female WT C57BL/6 mice aseptically. Skeletal muscle was removed and epiphysis cut away. Lumen of bone flushed with PBS and cells pelleted and taken up. Harvest of T-cells: spleens from 2 female WT C57BL/6 mice were meshed through a 100 µM cell strainer into a PBS filled dish. Cells were taken up in PBS and transferred into a 50 mL falcon tube and spun down. Cells were taken up in 1 mL PBS and 20 µL of CD4 microbead + 20 µL CD8 microbeads per spleen added for incubation for 20 min at 4 degrees C. Cells were washed with PBS and applied to MACS separation in order to positively select CD4+ and CD8+ cells. Positively selected T-cells were counted and taken up. Transplantation: directly after the second irradiation, all WT BALB/c recipient mice were injected with 5 x 1.000.000 BM cells (50 µL) i.v. into the retroorbital venous plexus under isoflurane anesthesia. 15 BALB/c mice were treated with 1.2 mg of hBD2/kg BW/day in 100 µL PBS per day vial oral gavage from day 0-10.

15 BALB/c mice received vehicle 100 µL PBS per day via oral gavage from day 0-10.

### Tests:

The mice were weighed daily for the initial 7 days and survival was monitored for 100 days.

### Results

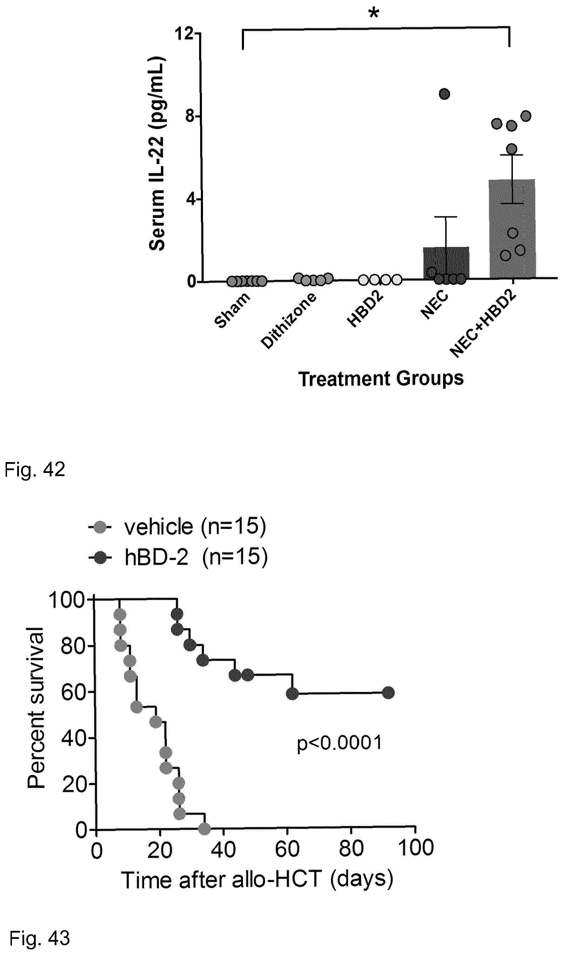

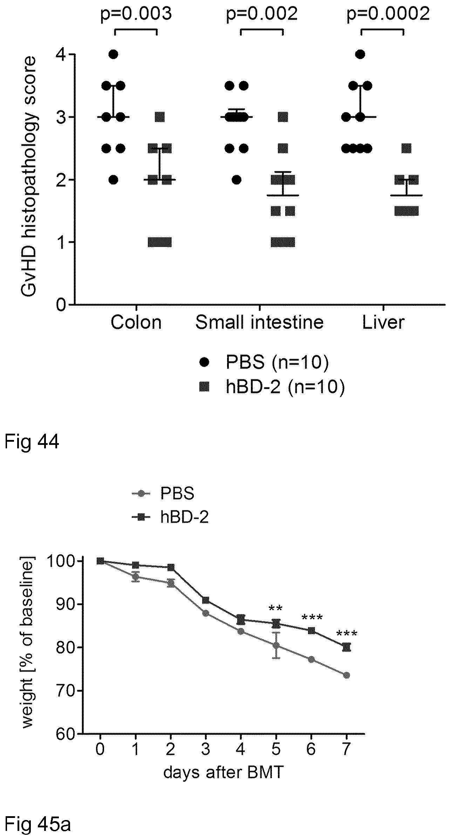

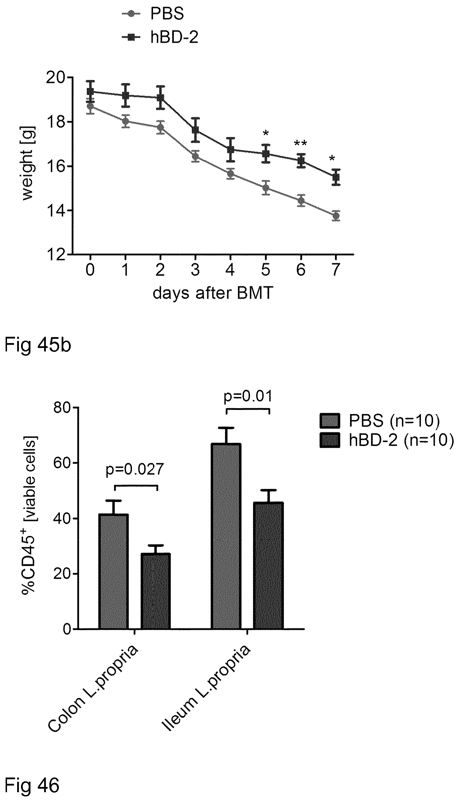

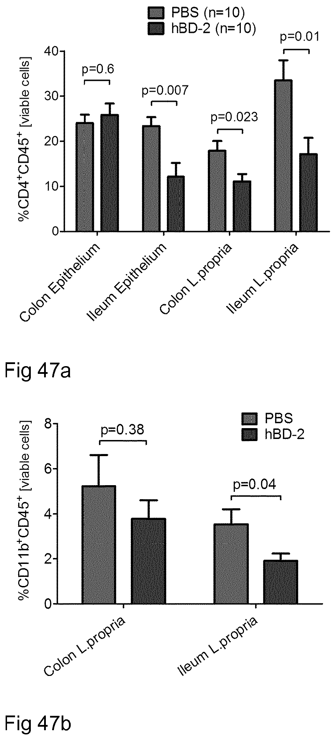

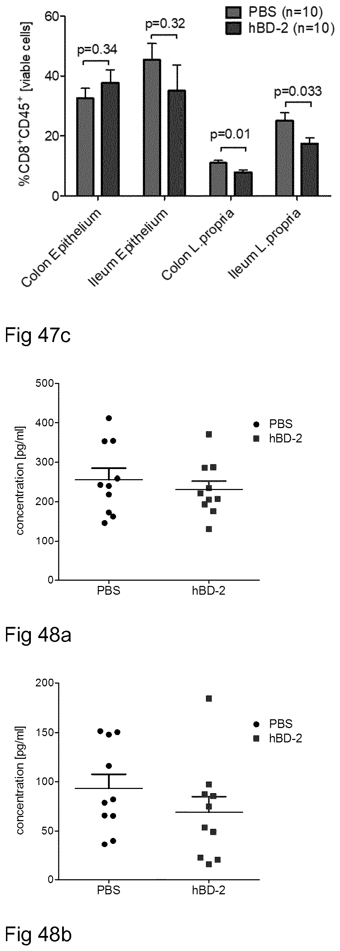

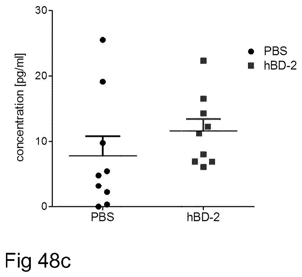

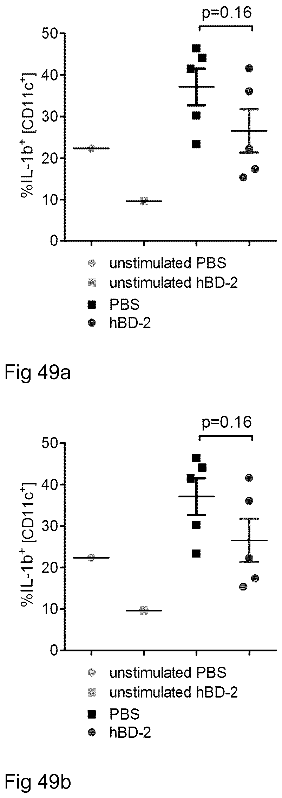

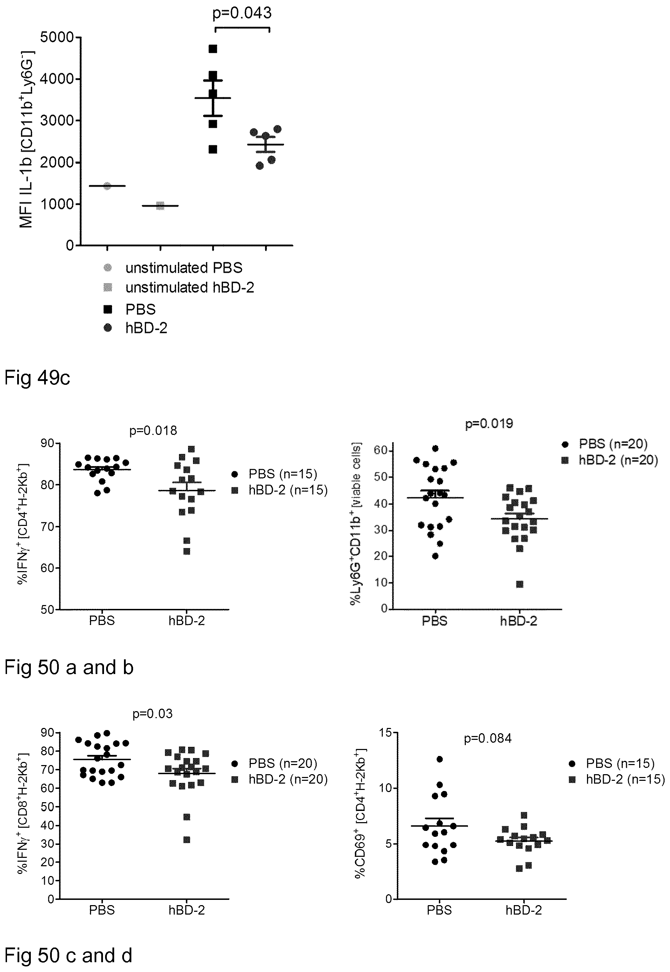

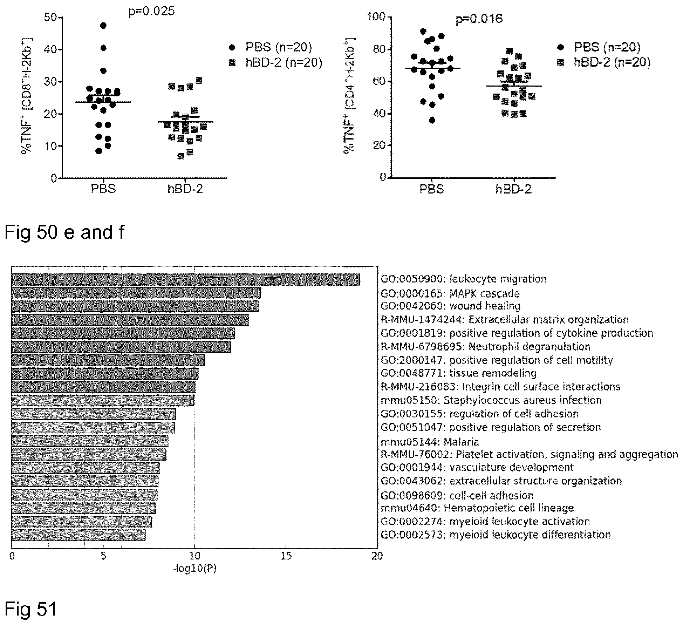

All 15 mice in the vehicle group had died by day 35 whereas only 4 or less than 30% of the hBD2 treated mice had died by day 35 and 8 mice were still alive by day 100 p<0.0001 (Figure 43). The histology score of the small intestine, colon and liver were all highly statistically lower for the hBD-2 treated group compared with PBS (Figure 44). The hBD2 treated mice lost statistically significantly less weight the first 7 days following bone marrow transplantation suggesting improved gut health and gut integrity (Figure 45). Treatment with hBD2 reduced the infiltration with CD45+ leucocytes (FACS analysis in Figure 46); intestinal T cell and myeloid cell infiltration (Figure 47 a-c) in lamina propria of the colon. Prophylactic treatment with hBD2 also showed reduction of TNF-α and IL-6 concentrations and increased concentration of IL-10 (Figure 48 a-c). The hBD-2 treatment additionally showed a reduction of IL-1β from myeloid cells (FACS analysis of the spleen in Figure 49 a-c). FACS analysis of the spleen also showed reduced number of neutrophils (Figure 50a); reduced Th1 cytokine production of especially TNF-α and IFN-γ (Figure 50 b-f). Microarray analysis of the colon samples showed decreased inflammation, leucocyte and myeloid cell migration and tissue remodelling in the hBD-2 treated group versus PBS (Figure 51).

Prior to stem cell transplantation the intestines of the patient are sterilized to the extent possible by treatment with two or three broad spectrum antibiotics. Following this treatment the intestine resembles the sterile intestine of the preterm infant. If a patient develops Graft versus host disease one of the first and most prominent symptom is usually severe colitis sharing characteristics with necrotizing enterocolitis in preterm infants. This example thus serves to demonstrate that oral treatment with hBD-2 from the time of stem cell transplantation prevents development of colitis and reduces mortality dramatically.

### Example 12 (Reference example).

To determine and assess the efficacy of prophylactic treatment with oral hBD-2 versus cyclosporine in a murine model of acute graft versus host disease following stem cell transplantation.

### Materials and methods

Treatment regime: 20 female BALB/c mice were irradiated with 4.5 Gy (2 x 498 sec) at intervals of at least 4 hours on day 0. Bone marrow was harvested: hind limbs harvested from 2 female WT C57BL/6 mice aseptically. Skeletal muscle was removed and epiphysis cut away. Lumen of bone flushed with PBS and cells pelleted and taken up. Harvest of T-cells: spleens from 2 female WT C57BL/6 mice were meshed through a 100 µM cell strainer into a PBS filled dish. Cells were taken up in PBS and transferred into a 50 mL falcon tube and spun down. Cells were taken up in 1 mL PBS and 20 µL of CD4 microbead + 20 µL CD8 microbeads per spleen added for incubation for 20 min at 4 degrees C. Cells were washed with PBS and applied to MACS separation in order to positively select CD4+ and CD8+ cells. Positively selected T-cells were counted and taken up. Transplantation: directly after the second irradiation, all WT BALB/c recipient mice were injected with 5 x 1.000.000 BM cells (50 µL) i.v. into the retroorbital venous plexus under isoflurane anesthesia. 20 BALB/c mice were treated with 1.2 mg of hBD2/kg BW/day (n=7); 50 mg cyclosporine/kg BW on days 0, 3, 6 and 9 (n=7) or 100 µL PBS per day vial oral gavage (n=6) from day 0-10.

### Tests:

The mice were weighed at regular intervals during the study and survival was monitored for 90 days.

### Results

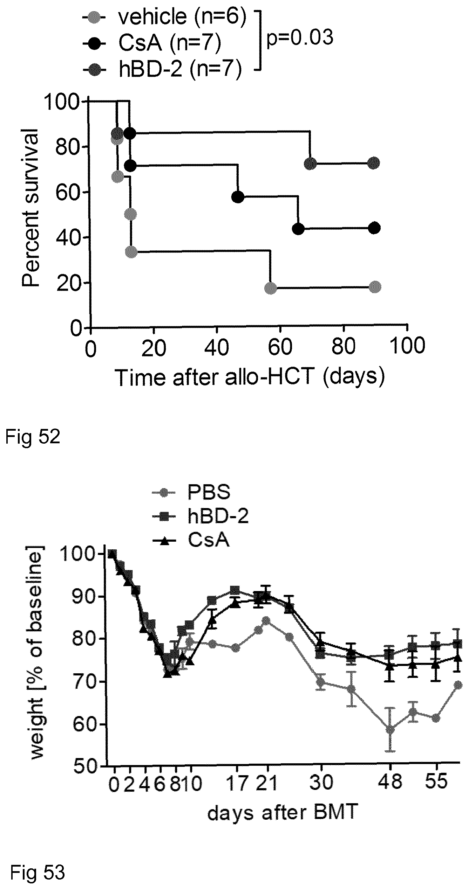

All but one of the PBS treated mice had died by day 90, whereas only 1 of the hBD-2 treated animals had died by day 90 (p = 0.03) and 3 animals of the cyclosporine treated animals had died (Figure 52). The hBD2 and cyclosporine treated mice lost statistically significantly less weight following bone marrow transplantation compared with the PBS treated mice suggesting improved gut health and gut integrity (Figure 53). Prior to stem cell transplantation the intestines of the patient are sterilized to the extent possible by treatment with two or three broad spectrum antibiotics. Following this treatment the intestine resembles the sterile intestine of the preterm infant. If a patient develops Graft versus host disease one of the first and most prominent symptom is usually severe colitis sharing characteristics with necrotizing enterocolitis in preterm infants. This example thus serves to demonstrate that oral treatment with hBD-2 from the time of stem cell transplantation prevents development of colitis and reduces mortality dramatically.

### Example 13 (Reference example).

To determine and assess the efficacy of prophylactic treatment with oral HD5 in a murine model of acute graft versus host disease following stem cell transplantation.

### Materials and methods

Treatment regime: 22 female BALB/c mice were irradiated with 4.5 Gy (2 x 498 sec) at intervals of at least 4 hours on day 0. Bone marrow was harvested: hind limbs harvested from 2 female WT C57BL/6 mice aseptically. Skeletal muscle was removed and epiphysis cut away. Lumen of bone flushed with PBS and cells pelleted and taken up. Harvest of T-cells: spleens from 2 female WT C57BL/6 mice were meshed through a 100 µM cell strainer into a PBS filled dish. Cells were taken up in PBS and transferred into a 50 mL falcon tube and spun down. Cells were taken up in 1 mL PBS and 20 µL of CD4 microbead + 20 µL CD8 microbeads per spleen added for incubation for 20 min at 4 degrees C. Cells were washed with PBS and applied to MACS separation in order to positively select CD4+ and CD8+ cells. Positively selected T-cells were counted and taken up. Transplantation: directly after the second irradiation, all WT BALB/c recipient mice were injected with 5 x 1.000.000 BM cells (50 µL) i.v. into the retroorbital venous plexus under isoflurane anesthesia. 22 BALB/c mice were treated with 1.2 mg of HD5/kg BW/day; 1.2 mg of hBD-2/kg BW/day or 100 µL of PBS per day vial oral gavage from day 0-10.

### Tests:

Survival was monitored for 60 days.

### Results

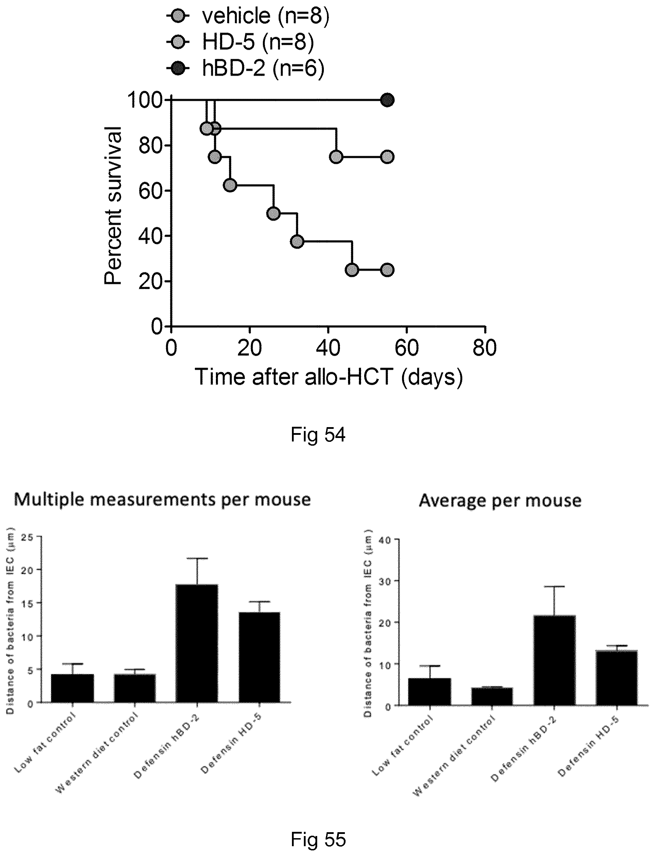

Six out of eight of the PBS treated mice had died by day 60, whereas 2 out of 8 of the HD5 treated animals had died and none of the hBD-2 treated animals had died by day 60 (Figure 54).

Prior to stem cell transplantation the intestines of the patient are sterilized to the extent possible by treatment with two or three broad spectrum antibiotics. Following this treatment the intestine resembles the sterile intestine of the preterm infant. If a patient develops Graft versus host disease one of the first and most prominent symptom is usually severe colitis sharing characteristics with necrotizing enterocolitis in preterm infants. This example thus serves to demonstrate that oral treatment with hBD-2 from the time of stem cell transplantation prevents development of colitis and reduces mortality dramatically.

### Example 14 (Reference example).

Anti microbial effect of orally administered defensins (HD5 and hBD-2).

### Method:

### Immunostaining of mucins and localization of bacteria by FISH

Mucus immunostaining was paired with fluorescent in situ hybridization (FISH), as previously described in order to analyze bacteria localization at the surface of the intestinal mucosa. Briefly, colonic tissues (proximal colon, 2nd cm from the cecum) without fecal material were placed in methanol-Carnoy's fixative solution (60% methanol, 30% chloroform, 10% glacial acetic acid) for a minimum of 3 hours at room temperature. Tissues were then washed in methanol 2 x 30 min, ethanol 2 x 15 min, ethanol/xylene (1:1), 15 min and xylene 2 x 15 min, followed by embedding in Paraffin with a vertical orientation. Five µm sections were performed and dewax by preheating at 60°C for 10 min, followed by xylene 60°C for 10 min, xylene for 10 min and 99.5% ethanol for 10 minutes. Hybridization step was performed at 50°C overnight with EUB338 probe (5'-GCTGCCTCCCGTAGGAGT-3', with a 5' labeling using Alexa 647) diluted to a final concentration of 10 µg/mL in hybridization buffer (20 mM Tris-HCl, pH 7.4, 0.9 M NaCl, 0.1% SDS, 20% formamide). After washing 10 min in wash buffer (20 mM Tris-HCl, pH 7.4, 0.9 M NaCl) and 3 x 10 min in PBS, PAP pen (Sigma-Aldrich) was used to mark around the section and block solution (5% fetal bovine serum in PBS) was added for 30 min at 4°C. Mucin- 2 primary antibody (rabbit H-300, Santa Cruz Biotechnology, Dallas, TX, USA) was diluted 1:1500 in block solution and apply overnight at 4°C. After washing 3 x 10 min in PBS, block solution containing anti-rabbit Alexa 488 secondary antibody diluted 1:1500, Phalloidin- Tetramethylrhodamine B isothiocyanate (Sigma-Aldrich) at 1µg/mL and Hoechst 33258 (Sigma- Aldrich) at 10µg/mL was applied to the section for 2h. After washing 3 x 10 min in PBS slides were mounted using Prolong anti-fade mounting media (Life Technologies, Carlsbad, CA, USA). Observations were performed with a Zeiss LSM 700 confocal microscope with software Zen 2011 version 7.1. This software was used to determine the distance between bacteria and epithelial cell monolayer, as well as the mucus thickness.

### Results:

A highly statistically significant difference in the lysis zone between the intestinal wall and the bacterial population was observed. The distance was small for mice fed both a low fat as well as a western diet, whereas HD5 and in particular hBD-2 had a profound effect on the distance (Figure 55).

### Conclusion:

This experiment demonstrates that orally administered HD5 and hBD-2 in a mouse exerts their microbiota modulating effect at the epithelial surface as if they were produced by the epithelial cells of the mouse themselves and not primarily in the lumen of the intestine as one would expect following oral administration.

### References

AlFaleh and Anabrees. Probiotics for prevention of necrotizing enterocolitis in preterm infants. Cochrane Database Syst Rev 2014;4:CD005496.
Butel et al. Conditions of bifidobacterial colonization in preterm infants: a prospective analysis. J pediatr Gastroenterol Nutr 2007; 44: 577-582.
Campeotto, F. et al. Fecal expression of human β-defensin-2 following birth. Neonatology 2010; 98: 365-369.
Donia, M.S. and Fischbach, M.A. 2015. Small molecules from the human microbiota. Science 349, 1254766
Dorrestein, P.C. et al., 2014. Finding the missing links among metabolites, microbes, and the host. Immunity 40: 824-832.
Gritz and Bandari. The human neonatal gut microbiome: a brief review. Frontiers in pediatrics 2015; 3.
Guaraldi and Salvatori. Effect of breast and formula feeding on gut microbiota shaping in newborns. Front Cell Infect Microbiol 2012; 2: 94
Hanson. Breastfeeding provides passive and likely long-lasting active immunity. Ann Allergy 1998; 81: 523-534.
Jakobsson, H.E. et al. 2014. Decreased gut microbiota diversity, delayed Bacteroidetes colonization and reduced Th1 responses in infants delivered by Caesarian section. Gut 63: 559-566.
Kai-Larsen et al. A review of the innate immune defense of the human foetus and newborn, with emphasis on antimicrobial peptides. Acta Paediatrica 2014; 103: 1000-1008.
Langston et al. Human lung growth in late gestation and in the neonate. Am Rev Respir 1984; 129: 607-613.
Mallow et al. Human enteric defensins. Gene structure and developmental expression. J Biol Chem 1996; 271: 4038-4045.
Manzoni et al. Bovine lactoferrin supplementation for prevention of necrotizing enterocolitis in very-low-birth-weight neonates: a randomized clinical trial. Early Hum Dev 2014; 90: 60-65.
Patel and Denning. Therapeutic use of prebiotics, probiotics and postbiotics to prevent necrotizing enterocolitis: What is the current evidence. Clin Perinatol 2013; 40: 11-25 Puiman, P.J. Paneth cell hyperplasia and metaplasia in necrotizing enterocolitis. Pediatr Res 2011, 69: 217-223.
Richter et al. Influence of gestational age, cesarean section, and type of feeding on fecal human beta-defensin 2 and tumor necrosis factor-alpha. J Pediatr Gastroenterol Nutr 2010; 51:103-5.
Saigal and Doyle. An overview of mortality and sequelae of preterm birth from infancy to adulthood. Lancet 2008; 371:261-269.
Sakurai et al. Extrauterine growth restriction in preterm infants of gestational age < 32 weeks. Pediatrics International 2008; 50: 70-75.
Salzman et al. Enteric defensin expression in necrotizing enterocolitis. Pediatr Res 1998; 44: 20-26.
Salzman NH, Underwood MA and Bevins CL, 2007. Paneth cells, defensins, and the commensal microbiota: a hypothesis on intimate interplay at the intestinal mucosa. Semin Immunol 19(2):70-83.
Sangild et al. Invited review: the preterm pig as a model in pediatric gastroenterology. J Anim Sci 2013;91:4713-29.
Schirmer, M. et al., 2016. Linking the human gut microbiome to inflammatory cytokine production capacity. Cell 167: 1125-1136
Sharma et al. Neonatal gut barrier and multiple organ failure: role of endotoxin and proinflammatory cytokines in sepsis and necrotizing enterocolitis. J Pediatr Surg 2007; 42: 454-461.
Sheng et al. Human β-defensin-3 promotes intestinal epithelial cell migration and reduces the development of necrotizing enterocolitis in a neonatal rat model. Pediatric Researh 2014; 76: 269279.
Siggers et al. Nutritional modulation of the gut microbiota and immune system in preterm neonates susceptible to necrotizing enterocolitis. J Nutr Biochem 2010;
Starner et al. Expression and activity of β-defensins and LL-37 in the developing human lung. J Immunol 2005;174: 1608-1615.
Stoll and Hansen. Infections in VLBW infants: studies from the NICHD neonatal research network. Semin Perinatol 2003; 27: 293-310.
Strunk et al. Infection-induced inflammation and cerebral injury in preterm infants. Lancet Infect Dis 2014; 14:751-62.
Sweet et al. Two-year outcome of infants weighing 600 grams or less at birth and born 1994 through 1998 Obstet Gynecol 2003; 101: 18-23.
Tirone, C. et al. Correlation of levels of alpha-defensins determined by HPLC-ESI-MS in bronchoalveolar lavage fluid with the diagnosis of pneumonia in premature neonates. Pediatr Res 2010; 68: 140-144.
Trend et al. Levels of innate immune factors in preterm and term mother's breast milk during the 1st month postpartum. British Journal of Nutrition 2016;
Trompette, A. et al., 2013. Gut microbiota metabolism of dietary fiber influences allergic airway disease and hematopoiesis. Nature Medicine 20: 159-168.
Wehkamp J, et al., 2002. Innate immunity and colonic inflammation: enhanced expression of epithelial alpha-defensins. Dig Dis Sci. 47(6):1349-55.
Støy, A.C.F (2012). "The effect of bovine colostrum products on intestinal dysfunction and inflammation in a preterm pig model of necrotizing entercolitis." Kgs. Lynby: Technical University of Denmark (DTU) WO 2010/007166
WO 92/06204
WO 95/17413
WO 95/22625
US 5,223,409
WO 2010/007166

## Claims

1. An antimicrobial peptide selected from the group consisting of: hBD-2 (SEQ ID NO: 2), truncated hBD-2 (SEQ ID NO: 16), and functionally equivalent variants having 1-2 amino acid modification(s), for use in the prevention or treatment of necrotizing enterocolitis (NEC) in a preterm infant.

2. The antimicrobial peptide for use according to any of the preceding claims, wherein said antimicrobial peptide is hBD-2 (SEQ ID NO:2).

3. The antimicrobial peptide for use according to any of the preceding claims, wherein two antimicrobial peptides, such as for example hBD-2 and HD5, are administered to the preterm infant.

4. The antimicrobial peptide for use according to any one of the preceding claims, wherein said antimicrobial peptide further comprises at least one additional moiety selected from the group consisting of a cell penetrating peptide (CPP), an Albumin Binding Moiety (ABM), a detectable moiety (Z), and a half-life extending peptide, preferably the additional moiety is a half-life extending peptide.

5. The antimicrobial peptide for use according to claim any one of claims 1-3, wherein the antimicrobial peptide is linked to a pharmaceutically acceptable molecule selected from the group consisting of transferrin, albumin (HSA), XTEN or PEG, a homo-amino acid polymer (HAP), a proline-alanine-serine polymer (PAS), or an elastin-like peptide (ELP), hyaluronic acid, a negatively charged sialylated peptide such as the carboxy-terminal peptide (CTP) of chorionic gonadotropin (CG) β-chain, human IgG, CH₃(CH₂)ₙCO- wherein n is 8 to 22, and a molecule having binding to a neonatal Fc receptor (FcRn).

6. The antimicrobial peptide for use according to any one of the preceding claims, wherein the antimicrobial peptide is administered in combination with surfactants and/or prebiotics and/or probiotics and/or tryptophane, and/or glucocorticoids and/or antibiotics and/or immunosuppressants and/or GLP-2 and/or GLP-2 analogs or any combination thereof.

7. The antimicrobial peptide for use according to any one of the preceding claims, wherein said antimicrobial peptide is administered to a preterm infant at least every other day, one time a day, such as at least twice a day, such as at least three times a day, such as at least four times a day, such as five times a day or continuously.

8. The antimicrobial peptide for use according to any of the preceding claims, wherein said antimicrobial peptide is administered to the preterm infant starting on the date of birth.

9. The antimicrobial peptide for use according to any one of the preceding claims, wherein said antimicrobial peptide is administered by oral, buccal, sublingual, rectal, vaginal, intratracheal, intrapulmonary, intranasal, intracranial, subcutaneous, intravenous, dermal, or transdermal administration.

10. The antimicrobial peptide for use according to any one of the preceding claims, wherein
said antimicrobial peptide is administered by oral administration; or
said antimicrobial peptide is administered by intrapulmonary, intratracheal, or intranasal administration, such as intrapulmonary, intratracheal, or intranasal administration by an inhaler, nebulizer, or vaporizer.

11. The antimicrobial peptide for use according to any one of the preceding claims, wherein said antimicrobial peptide is administered at a daily dosage of 0.1 mg/kg body weight to 10 mg/kg body weight.

12. The antimicrobial peptide for use according to any of the preceding claims, wherein the preterm infant has a birth weight of 1,500 grams or less, such as 1,000 grams or less.

## Patentansprüche

1. Antimikrobielles Peptid, ausgewählt aus der Gruppe bestehend aus: hBD-2 (SEQ ID NO: 2), verkürztem hBD-2 (SEQ ID NO: 16) und funktionell äquivalenten Varianten mit 1-2 Aminosäuremodifikation(en), zur Verwendung bei der Prävention oder Behandlung der nekrotisierenden Enterokolitis (NEC) bei einem Frühgeborenen.

2. Antimikrobielles Peptid zur Verwendung nach einem der vorhergehenden Ansprüche, wobei das antimikrobielle Peptid hBD-2 (SEQ ID NO:2) ist.

3. Antimikrobielles Peptid zur Verwendung nach einem der vorhergehenden Ansprüche, wobei zwei antimikrobielle Peptide, wie beispielsweise hBD-2 und HD5, dem Frühgeborenen verabreicht werden.

4. Antimikrobielles Peptid zur Verwendung nach einem der vorhergehenden Ansprüche, wobei das antimikrobielle Peptid ferner mindestens eine zusätzliche Einheit umfasst, ausgewählt aus der Gruppe bestehend aus einem zellpenetrierenden Peptid (CPP), einer Albumin-Bindungseinheit (ABM), einer nachweisbaren Einheit (Z) und einem die Halbwertszeit verlängernden Peptid, wobei die zusätzliche Einheit vorzugsweise ein die Halbwertszeit verlängerndes Peptid ist.

5. Antimikrobielles Peptid zur Verwendung nach einem der Ansprüche 1-3, wobei das antimikrobielle Peptid an ein pharmazeutisch akzeptables Molekül gebunden ist, ausgewählt aus der Gruppe bestehend aus Transferrin, Albumin (HSA), XTEN oder PEG, einem Homoaminosäurepolymer (HAP), einem Prolin-Alanin-Serin-Polymer (PAS) oder einem elastinähnlichen Peptid (ELP), Hyaluronsäure, einem negativ geladenen sialylierten Peptid wie dem Carboxy-terminalen Peptid (CTP) der Choriongonadotropin (CG)-β-Kette, humanem IgG, CH₃(CH₂)ₙCO-, wobei n 8 bis 22 ist, und einem Molekül mit Bindung an einen neonatalen Fc-Rezeptor (FcRn).

6. Antimikrobielles Peptid zur Verwendung nach einem der vorhergehenden Ansprüche, wobei das antimikrobielle Peptid in Kombination mit Tensiden und/oder Präbiotika und/oder Probiotika und/oder Tryptophan und/oder Glucocorticoiden und/oder Antibiotika und/oder Immunsuppressiva und/oder GLP-2 und/oder GLP-2-Analoga oder einer beliebigen Kombination davon verabreicht wird.

7. Antimikrobielles Peptid zur Verwendung nach einem der vorhergehenden Ansprüche, wobei das antimikrobielle Peptid einem Frühgeborenen mindestens jeden zweiten Tag, einmal täglich, wie mindestens zweimal täglich, wie mindestens dreimal täglich, wie mindestens viermal täglich, wie fünfmal täglich oder kontinuierlich verabreicht wird.

8. Antimikrobielles Peptid zur Verwendung nach einem der vorhergehenden Ansprüche, wobei das antimikrobielle Peptid dem Frühgeborenen beginnend am Tag der Geburt verabreicht wird.

9. Antimikrobielles Peptid zur Verwendung nach einem der vorhergehenden Ansprüche, wobei das antimikrobielle Peptid durch orale, bukkale, sublinguale, rektale, vaginale, intratracheale, intrapulmonale, intranasale, intrakranielle, subkutane, intravenöse, dermale oder transdermale Verabreichung verabreicht wird.

10. Antimikrobielles Peptid zur Verwendung nach einem der vorhergehenden Ansprüche, wobei
das antimikrobielle Peptid durch orale Verabreichung verabreicht wird; oder
das antimikrobielle Peptid durch intrapulmonale, intratracheale oder intranasale Verabreichung verabreicht wird, wie intrapulmonale, intratracheale oder intranasale Verabreichung mittels eines Inhalators, Verneblers oder Verdampfers.

11. Antimikrobielles Peptid zur Verwendung nach einem der vorhergehenden Ansprüche, wobei das antimikrobielle Peptid in einer Tagesdosis von 0,1 mg/kg Körpergewicht bis 10 mg/kg Körpergewicht verabreicht wird.

12. Antimikrobielles Peptid zur Verwendung nach einem der vorhergehenden Ansprüche, wobei das Frühgeborene ein Geburtsgewicht von 1.500 Gramm oder weniger aufweist, wie 1.000 Gramm oder weniger.

## Revendications

1. Peptide antimicrobien sélectionné dans le groupe constitué de : hBD-2 (SEQ ID NO: 2), hBD-2 tronquée (SEQ ID NO: 16), et variantes fonctionnellement équivalentes présentant 1-2 modification(s) d'acides aminés, pour utilisation dans la prévention ou le traitement de l'entérocolite nécrosante (NEC) chez un nourrisson prématuré.

2. Peptide antimicrobien pour utilisation selon l'une quelconque des revendications précédentes, dans lequel ledit peptide antimicrobien est hBD-2 (SEQ ID NO:2).

3. Peptide antimicrobien pour utilisation selon l'une quelconque des revendications précédentes, dans lequel deux peptides antimicrobiens, tels que par exemple hBD-2 et HD5, sont administrés au nourrisson prématuré.

4. Peptide antimicrobien pour utilisation selon l'une quelconque des revendications précédentes, dans lequel ledit peptide antimicrobien comprend en outre au moins un groupement supplémentaire sélectionné dans le groupe constitué d'un peptide pénétrant dans les cellules (CPP), d'un groupement de liaison à l'albumine (ABM), d'un groupement détectable (Z), et d'un peptide prolongeant la demi-vie, de préférence le groupement supplémentaire est un peptide prolongeant la demi-vie.

5. Peptide antimicrobien pour utilisation selon l'une quelconque des revendications 1-3, dans lequel le peptide antimicrobien est lié à une molécule pharma-ceutiquement acceptable sélectionnée dans le groupe constitué de transferrine, albumine (HSA), XTEN ou PEG, polymère d'homo-acides aminés (HAP), polymère proline-alanine-sérine (PAS), ou peptide de type élastine (ELP), acide hyaluronique, peptide sialylé chargé négativement tel que peptide carboxy-terminal (CTP) de la chaîne β de gonadotrophine chorionique (CG), IgG humaine, CH₃(CH₂)ₙCO- où n est de 8 à 22, et une molécule ayant une liaison à un récepteur Fc néonatal (FcRn).

6. Peptide antimicrobien pour utilisation selon l'une quelconque des revendications précédentes, dans lequel le peptide antimicrobien est administré en combinaison avec des tensioactifs et/ou des prébiotiques et/ou des probiotiques et/ou du tryptophane, et/ou des glucocorticoïdes et/ou des antibiotiques et/ou des immunosuppresseurs et/ou du GLP-2 et/ou des analogues du GLP-2 ou toute combinaison de ceux-ci.

7. Peptide antimicrobien pour utilisation selon l'une quelconque des revendications précédentes, dans lequel ledit peptide antimicrobien est administré à un nourrisson prématuré au moins un jour sur deux, une fois par jour, tel qu'au moins deux fois par jour, tel qu'au moins trois fois par jour, tel qu'au moins quatre fois par jour, tel que cinq fois par jour ou de manière continue.

8. Peptide antimicrobien pour utilisation selon l'une quelconque des revendications précédentes, dans lequel ledit peptide antimicrobien est administré au nourrisson prématuré à partir de la date de naissance.

9. Peptide antimicrobien pour utilisation selon l'une quelconque des revendications précédentes, dans lequel ledit peptide antimicrobien est administré par administration orale, buccale, sublinguale, rectale, vaginale, intratrachéale, intrapulmonaire, intranasale, intracrânienne, sous-cutanée, intraveineuse, dermique, ou transdermique.

10. Peptide antimicrobien pour utilisation selon l'une quelconque des revendications précédentes, dans lequel
ledit peptide antimicrobien est administré par administration orale ; ou
ledit peptide antimicrobien est administré par administration intrapulmonaire, intratrachéale, ou intranasale, telle qu'une administration intrapulmonaire, intratrachéale, ou intranasale au moyen d'un inhalateur, d'un nébuliseur, ou d'un vaporisateur.

11. Peptide antimicrobien pour utilisation selon l'une quelconque des revendications précédentes, dans lequel ledit peptide antimicrobien est administré à une dose quotidienne de 0,1 mg/kg de poids corporel à 10 mg/kg de poids corporel.

12. Peptide antimicrobien pour utilisation selon l'une quelconque des revendications précédentes, dans lequel le nourrisson prématuré a un poids de naissance de 1 500 grammes ou moins, tel que 1 000 grammes ou moins.
